(19) **Europäisches Patentamt**
**European Patent Office**
**Office européen des brevets**

(11) **EP 2 145 180 B1**

(12) **EUROPEAN PATENT SPECIFICATION**

(45) Date of publication and mention
of the grant of the patent:
**04.12.2013 Bulletin 2013/49**

(51) Int Cl.:
*G01N 33/48* (2006.01) *C12Q 1/68* (2006.01)

(21) Application number: **08745685.1**

(22) Date of filing: **11.04.2008**

(86) International application number:
**PCT/US2008/060125**

(87) International publication number:
**WO 2008/128111 (23.10.2008 Gazette 2008/43)**

(54) **COMPARATIVE SEQUENCE ANALYSIS PROCESSES AND SYSTEMS**

VERGLEICHENDE SEQUENZANALYSEVERFAHREN UND -SYSTEME

PROCÉDÉS ET SYSTÈMES D'ANALYSE DE SÉQUENCE COMPARATIVE

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **13.04.2007 US 911845 P**

(43) Date of publication of application:
**20.01.2010 Bulletin 2010/03**

(73) Proprietor: **SEQUENOM, INC.**
**San Diego, California 92121 (US)**

(72) Inventors:
• **CHEN, Yong Qing**
**San Diego, CA 92130 (US)**
• **HONISCH, Christiane**
**La Jolla, CA 92037 (US)**
• **VAN DEN BOOM, Dirk J.**
**La Jolla, CA 92037 (US)**

(74) Representative: **Vossius & Partner**
**Siebertstrasse 4**
**81675 München (DE)**

(56) References cited:
**WO-A1-2005/100607    WO-A1-2006/121295**
**WO-A2-03/066882      US-A1- 2005 186 588**
**US-A1- 2005 272 070**

• **HONISCH C ET AL: "High-troughput mutation detection underlying adaptive evolution of Escherichia coli-K12", GENOME RESEARCH, vol. 14, no. 12, 1 December 2004 (2004-12-01), pages 2495-2502, XP002991060, COLD SPRING HARBOR LABORATORY PRESS, WOODBURY, NY, US ISSN: 1088-9051, DOI: 10.1101/GR. 2977704**
• **EHRICH MATHIAS ET AL: "Multiplexed discovery of sequence polymorphisms using base-specific cleavage and MALDI-TOF MS.", NUCLEIC ACIDS RESEARCH, vol. 33, no. 4, E38, 2005, pages 1-6, XP002659972, ISSN: 1362-4962**
• **BÖCKER S: "SNP and mutation discovery using base-specific cleavage and MALDI-TOF mass spectrometry", BIOINFORMATICS, vol. 19, no. SUPPL 1, 1 January 2003 (2003-01-01), pages I44-I53, XP002338050, OXFORD UNIVERSITY PRESS, SURREY, GB ISSN: 1367-4803, DOI: 10.1093/BIOINFORMATICS/BTG1004**
• **HONISCH C ET AL: "Improving the odds in comparative sequence analysis: Mass spectrometry for sensitive detection of sequence variations", AMERICAN JOURNAL OF HUMAN GENETICS; 53RD ANNUAL MEETING OF THE AMERICAN SOCIETY OF HUMAN GENETICS , vol. 73, no. 5 8 November 2003 (2003-11-08), page 441, XP008143340, AMERICAN SOCIETY OF HUMAN GENETICS, CHICAGO, IL, US ISSN: 0002-9297 Retrieved from the Internet: URL:http://www.ashg.org/genetics/abstracts/abs03/f1590.htm**

• **SPRATT B G: "Multilocus sequence typing: Molecular typing of bacterial pathogens in an Era of rapid DNA sequencing and the internet", CURRENT OPINION IN MICROBIOLOGY, vol. 2, 1 January 1999 (1999-01-01), pages 312-316, XP002967926, CURRENT BIOLOGY LTD, GB ISSN: 1369-5274, DOI: 10.1016/S1369-5274(99) 80054-X**

**Description**

Field of the Invention

The invention pertains to methods for analyzing sequence information and pattern information of biomolecule sequences as defined by the claims. The invention thus pertains to detecting and identifying biomolecules sequence information in a sample.

Background

Genetic information of all living organisms (e.g., animals, plants and microorganisms) and other forms of replicating genetic information like viruses is encoded in deoxyribonucleic acid (DNA) or ribonucleic acid (RNA). Genetic information is the succession of nucleotides or modifications thereof representing the primary structure of real or hypothetical DNA/RNA molecule or strands with the capacity to carry information. In humans, the complete genome contains of about 30.000 genes located on 24 chromosomes (The Human Genome, T. Strachan, BIOS Scientific Publishers, 1992). Each gene codes for a specific protein, which after its expression via transcription and translation, fulfills a specific biochemical function within a living cell.

A change or variation in the genetic code can result in a change in the sequence or level of expression of mRNA and potentially in the protein encoded by the mRNA. These changes, which sometimes are polymorphisms or mutations, can give rise to modifications to the encoded RNA or protein and thereby lead to significant adverse effects, sometimes resulting in disease.

Many diseases caused by genetic variations are known and include hemophilia, thalassemia, Duchenne Muscular Dystrophy (DMD), Huntington's Disease (HD), Alzheimer's Disease and Cystic Fibrosis (CF) (Human Genome Mutations, D. N. Cooper and M. Krawczak, BIOS Publishers, 1993). Genetic diseases such as these can result from a single addition, substitution, or deletion of a single nucleotide in the deoxynucleic acid (DNA) forming the particular gene. Certain birth defects are the result of chromosomal abnormalities such as Trisomy 21 (Down's Syndrome), Trisomy 13 (Patau Syndrome), Trisomy 18 (Edward's Syndrome), Monosomy X (Tumers Syndrome) and other sex chromosome aneuploidies such as Klinefelter's Syndrome (XXY). Further, there is growing evidence that some DNA sequences can predispose an individual to any of a number of diseases such as diabetes, arteriosclerosis, obesity, various autoimmune diseases and cancer (e.g., colorectal, breast, ovarian, lung).

A change in a single nucleotide between genomes of more than one individual of the same species (e.g. human beings), that accounts for heritable variation among the individuals, is referred to as a "single nucleotide polymorphism" (SNP). Not all SNPs result in disease. The effect of a SNP is dependent on its position and frequency of occurrence, and can range from harmless to fatal. Certain polymorphisms are thought to predispose some individuals to disease or are related to morbidity levels of certain diseases. Atherosclerosis, obesity, diabetes, autoimmune disorders, and cancer are a few of such diseases thought to have a correlation with polymorphisms. In addition to a correlation with disease, SNPs are also thought to play a role in a patient's response to therapeutic agents given to treat disease. For example, SNPs are believed to play a role in a patient's ability to respond to drugs, radiation therapy, and other forms of treatment.

Identifying genetic variance can lead to better understanding of particular diseases and potentially more effective therapies for such diseases. An example of a publication concerning the determination of genetic variance is the abstract Honisch et al., "Improving the odds in comparative sequence analysis Mass spectrometry for sensitive detection of sequence variations" program Nr: 1590 from 2003 ASHG Annual Meeting (http://www.ashg.org/genetics/abstracts/abs03/f1590.htm). In Honisch et al. mass spectrometry is combined with base-specific cleavage thereby offering significant advantages in speed and accuracy of data acquisition. Personalized therapy regiments based on a patient's identified genetic variance can result in life saving medical interventions. Novel drugs or compounds can be discovered that interact with products of a specific variance, once the variance is identified. Identification of infectious organisms, including viruses, bacteria, prions, and fungi, can also be achieved based on identification of genetic signatures and variance, and can result in an appropriate targeted therapeutic and monitoring of the infection and treatment. Identification and/or grouping of sequence signatures of infectious organisms also can lead to epidemiological characterizations of a disease outbreak or organism profile.

Summary

The present invention is defined by the claims. Described herein are processes and systems for rapid and accurate sequence or composition sequence detection as well as identification and grouping. Such processes and systems can be applied to a variety of comparative sequence analyses, and can be utilized to rapidly detect and/or identify the presence or absence of one or more target biomolecules in a sample or mixture, identify frequencies of biomolecules in a sample or mixture, determine common sequence patterns in a sample or mixture, and prepare reference sequence patterns for application to prospective analyses, for example. Processes and systems provided herein can be utilized in basic research, clinical research, diagnostics and medical procedures, can be applied to biomolecule sequence analysis in a variety of organisms (e.g., mammals, and particularly humans), and can be used in variety of analytical processes, including, but not limited to, disease marker identification (e.g., cancer marker identification), HLA typing, mutation detection, forensics, vaccine control, vector identity, population studies, microbial identification, and the like.

Thus, provided herein are processes for determining the presence or absence of a target biomolecule sequence of a sample, which comprise: (a) identifying and scoring matching peak patterns between (i) a sample set of signals derived from cleavage products resulting from contacting a biomolecule in the sample with a specific cleavage agent and (ii) a reference set of signals derived from cleavage products resulting from a reference biomolecule contacted with, or virtually contacted with, the specific cleavage agent; (b) selecting a top-ranked subset of matching peak patterns between the sample set of signals and the reference set of signals based on the scoring; (c) iteratively re-scoring matching peak patterns in the subset and identifying one or more top-ranked matching peak patterns; and (d) determining the presence or absence of the target biomolecule sequence or a combination of sequences or mixtures of compositions in the sample by the match between the one or more top-ranked matching peak patterns. The processes can comprise identifying one or more potential sequence variations (e.g., mutation(s)) in the biomolecule sequence of the one or more top-ranked matching peak patterns of the reference set and/or the sample set. The processes also can comprise assigning a confidence value to the match between the one or more top-ranked matching peak patterns in some embodiments.

Also provided are processes for determining the presence or absence of a target biomolecule sequence of a sample, which comprise: identifying matching peak patterns between (i) a sample set of signals derived from cleavage products resulting from contacting a biomolecule in the sample with a specific cleavage agent and (ii) a reference set of signals derived from cleavage products resulting from a reference biomolecule contacted with, or virtually contacted with, the specific cleavage agent; where the reference peak pattern is determined by aligning by mass all the reference peaks within a set, representing each reference peak with a peak intensity, calculating the distance between each peak intensity within the reference set, and clustering reference peaks to generate a minimum set of cleavage reactions. The peak intensity is determined by acquiring and filtering a subset of mass spectra, grouping one or more sets of peaks together, calculating the group intensity using the heights and masses for each peak in the group, and normalizing the group intensities. The clustering is determined by identifying peaks present in one set of references but absent in other sets, sub-clustering until each cluster has only one sequence or a set of indistinguishable sequences, summing up the intensities of the peaks in the sub-clusters, and evaluating the differences between sub-clusters. The subset of mass spectra is selected by selecting 10-20 anchor peak sets from the reference peak pattern, representing all reference peaks by one or more peaks in each anchor peak set, filtering the peaks by applying a moving width filter with Gaussian kernel, grouping together one or a set of peaks together and determining a common baseline in the original spectrum for the group, and adjusting baseline data points from the original spectrum for the group of peaks to fit to a Gaussian curve to determine peak intensities and signal to noise ratios. The peak intensities are calculated from the heights and widths of the mass spectra. The signal to noise ratios are calculated from the heights and widths of the mass spectra. The peaks with low signal to noise ratios are evaluated to establish a threshold and the peaks are removed from a final peak list. The peak intensities are then normalized to be in the range of 2000-4000 Da.

Also provided are processes for determining the presence or absence of a target biomolecule sequence of a sample, which comprise: identifying matching peak patterns between (i) a sample set of signals derived from cleavage products resulting from contacting a biomolecule in the sample with a specific cleavage agent and (ii) a reference set of signals derived from cleavage products resulting from a reference biomolecule contacted with, or virtually contacted with, the specific cleavage agent, where the sample matching peak patterns is calibrated by matching the sample peaks to reference peaks within a certain mass window, removing sample peak outliners by evaluating an overall deviation pattern, selecting high intensity peaks which are evenly distributed across the whole mass range as anchor peaks, and comparing the number of peaks matching a preselected set of peaks or anchor peak sets from the reference peak patterns. The peak intensities are adjusted by fitting peak intensities to a standard profile of different mass ranges, fitting the center mass regions of the profile to a Gaussian curve, and revising the intensities for all detected peaks with the adjustment. The anchor peaks are calibrated by their mass and spectrum quality.

Also provided are processes for determining the presence or absence of a target biomolecule sequence of a sample, which comprise: (a) identifying and scoring matching peak patterns between (i) a sample set of signals derived from cleavage products resulting from contacting a biomolecule in the sample with a specific cleavage agent and (ii) a reference set of signals derived from cleavage products resulting from a reference biomolecule contacted with, or virtually contacted with, the specific cleavage agent; wherein the scoring is based upon one or more criteria selected from the group consisting of a bitmap score, a discriminating feature matching score, a distance score, a peak pattern identity score, and an adjChange score; (b) identifying one or more top-ranked matching peak patterns; and (c) determining the presence or absence of the target biomolecule sequence in the sample by the match between the one or more top-ranked matching peak patterns. An average of the bitmap score and the peak pattern identity score, or "final score" can be determined, which can be utilized for the comparison of sequences in different samples and between samples, for example. The one or more top-ranked matching peak patterns are identified by iteratively re-scoring matching peak patterns in a subset of top-ranked matching peak patterns between the sample set of signals and the reference set of signals. The processes may comprise identifying potential sequence variations (e.g., mutations) in the biomolecule sequence of the one or more top-ranked matching peak patterns of the reference set and/or the sample set and the probability of their occurance. The processes can comprise assigning a confidence value to the match between the one or more top-ranked matching peak patterns. The assignment of a likelihood of the occurance of sequence variations can be based on a certain probability model.

Provided also are processes for determining the presence or absence of a target biomolecule sequence or a mixture of regions in the genome or a mixture of targets in a population (e.g. consesnsus sequence) which or sequence composition in a sample, which comprise: (a) identifying and scoring matching peak patterns between (i) a sample set of signals derived from cleavage products resulting from contacting a biomolecule in the sample with a specific cleavage agent and (ii) a reference set of signals derived from cleavage products resulting from a reference biomolecule contacted with, or virtually contacted with, the specific cleavage agent; wherein the scoring is based upon one or more criteria selected from the group consisting of a bitmap score, a discriminating feature matching score, a distance score, a peak pattern identity score and an adjChange score; (b) identifying one or more top-ranked matching peak patterns; wherein the one or more top-ranked matching peak patterns are identified by iteratively re-scoring matching peak patterns in a subset of top-ranked matching peak patterns between the sample set of signals and the reference set of signals; (c) identifying potential sequence variations in the biomolecule sequence of the one or more top-ranked matching peak patterns of the reference set and/or the sample set; (d) determining the presence or absence of the target biomolecule sequence in the sample by the match between the one or more top-ranked matching peak patterns; and (e) assigning a confidence value to the match between the one or more top-ranked matching peak patterns (f) applying a probability model to determine the likelihood of any sequence variation to occur.

Also provided are processes where the bitmap score can be calculated by comparing intensities of detected and individual reference peak patterns weighted by reference peak intensity. The discriminating feature matching score can be calculated by evaluating a subset of features that discriminate one feature pattern from another or one set of patterns from another set. The  distance score can be calculated based on distance of the identified feature vectors to all reference feature vectors. And the distance may be a Euclidian distance. The peak pattern identity score may be calculated from the sum of the matched peak intensities, missing and additional peak intensities, silent missing peak intensities and silent additional peak intensities. The top-ranked matching peak patterns are identified by iteratively re-scoring matching peak patterns in about five or more, in about ten or more, in about 50 or more or in about 100 or more cycles. The sample set of mass signals is subject to one or more signal processing methods selected from the group consisting of peak detection, calibration, normalization, spectra quality, intensity scaling and compomer adjustment filters. The reference set of mass signals may be derived from cleavage products resulting from a reference nucleic acid virtually contacted with the specific cleavage agent. The reference set of mass signals may be subject to clustering. The clustering may be based upon peak masses and peak intensities. Any of the process above may have two or more reference sets of mass signals each derived from cleavage products resulting from a reference nucleic acid contacted with, or virtually contacted with, the specific cleavage agent. The process above may contain a step where each of the reference sets is compared to the sample set, or a step where the reference sets are mixed and compared as a single set to the sample set, or a step where the reference sets are mixed and compared as a single set to a mixed sample set, or a step where the reference samples are mixed and compared as a single set to a mixed sample set, or a step where the reference samples are compared as a single set to a mixed sample set.

Also provided are processes where the reference sets of mass signals derived from cleavage products resulting from a microbial or viral or vector or eukaryotic or prokaryotic reference nucleic acid contacted with, or virtually contacted with, the specific cleavage agent. The microbe may be a bacterium, fungus or virus. Any processes above may have each sample set and each reference set derived from one or more of (i) a first primer product contacted or virtually contacted with a first specific cleavage agent; (ii) a second primer product contacted or virtually contacted with a first cleavage agent; (iii) the first primer product contacted or virtually contacted with a second specific cleavage agent; (iv) the second primer product contacted or virtually contacted with a second cleavage agent. The first primer product may be a forward primer product. The second primer product may be a reverse primer product. The first primer product may be a reverse primer product. The second primer product may be a forward primer product. The first primer product may be a T7 primer product. The second primer product may be a SP6 primer product. For any of the above processes, the sample may be obtained from an organism; the sample may be obtained from a human.

In any of the above processes, a set of mass signals may be prepared by a method having the steps of contacting a sample DNA with a primer, extending the primer to form a primer product, transcribing the primer product to form a primer product RNA, contacting the primer product RNA with a specific cleavage agent to form cleavage products, and preparing a set of mass signals from the cleavage products. The primer may be extended by an amplification process and amplified primer products are prepared. The amplification process may be a polymerase chain reaction process (PCR). The set of mass signals may be prepared by mass spectrometric analysis. The mass spectrometric analysis may be MALDI-TOF MS.

In any of the above processes, a set of mass signals may be prepared by a method having the steps of contacting a sample DNA with a first primer and a second primer, extending the first primer and the second primer by an amplification process to form an amplified first primer product and an amplified second primer product, transcribing the first primer product and the second primer product to form a first primer product RNA and a second primer product RNA, contacting the first primer product RNA and the second primer product RNA with a first specific cleavage agent to form a first fragment set and a second fragment set, contacting the first primer product RNA and the second primer product RNA with a second specific cleavage agent to form a third fragment set and a fourth fragment set, and preparing a set of mass signals for each fragment set.

Also provided are inputs for clustering sequence analysis processes. Clustering processes often include grouping of samples based on their identified features. Grouping can be in comparison to one or more simulated references, it can be independent of references and/or it can entail a reference set alone, for example. It can be within one acquired experiment or between multiple experiments by database query on one or multiple databases. Grouping also can be performed with mixtures or with concatenated features (such as regions or cleavage reactions), for example. Clustering can be enhanced by learning algorithms and other processes known to the person of ordinary skill in the art. Distance measures/clustering processes can be utilized to group sequence signals in a sample, reference, sample sets and/or reference sets and mixtures thereof, for example. Cluster analysis allows the organization of samples or references without any knowledge of sequences of the samples or the references according to signal patterns of cleaved products. Clustering analysis is useful for a variety of applications, including without limitation, phylogenic analyses, epidemiology analyses (e.g., changes in microbe populations over time; comparison of microbe strains in one sample to another), drug effect monitoring (e.g., changes in microbe populations over time after administration of a drug), surveillance treatment monitoring, host-pathogen interactions, any sort of marker screening and monitoring (e.g. cancer marker, antibiotic resistance marker), forensics mutation screening, mitochondrial resequencing and HLA typing.

Thus, provided herein are clustering processes for grouping one or more sequences or sequence signals, which comprise: (a) comparing peak patterns between (i) a sample set of signals derived from cleavage products resulting from contacting a biomolecule in the sample with a specific cleavage agent or a mixture of cleavage agents and (ii) a reference set of signals derived from cleavage products resulting from a reference biomolecule contacted with, or virtually contacted with, the specific cleavage agent; (b) identifying cluster patterns of the signals; and (c) grouping the signals according to the cluster patterns in (b).

Some clustering processes include grouping or classifying samples (e.g., sets of samples) or references (e.g., sets of references) or a combination of samples and references (e.g., sets of samples and sets of references) based on their specific features (e.g. masses and intensities). The sequences or sequences signals can be derived from a biomolecule from a sample. Any applicable clustering methodologies known to the person of ordinary skill in the art may be utilized, including, but not limited to, unweighted pair group method analyses, neighbor joining analyses, maximum likelihood analyses, supervised/unsupervised analyses, hierachical/non-hierachical analyses, and the like. The cluster patterns can be determined from an array of peak positions in combination with intensities of the signals converted to integers. (a)(ii) can be two or more reference sets of signals each derived from cleavage products resulting from a reference biomolecule contacted with, or virtually contacted with, the specific cleavage agent. Clustering processes described herein can be enhanced by learning algorithms and other processes known to the person of ordinary skill in the art. Cluster patterns can be determined by an unweighted pair group method analysis. Cluster patterns may be determined from an array of peak positions in combination with intensities of the signals converted to integers. In certain examples, multiple sample sets or reference sequence sets are mixed (e.g., multiplexed) and grouped as a single set to an individual sample set. A sample set can be derived from an individual sample or may be derived from multiple samples by mixing. Peak patterns from different regions or organisms (e.g. multiple types in a population), whether mixed or not, whether from one or multiple cleavage reactions and whether simulated or detected, can be concatenated before clustering.

Methods provided herein can be carried out using mixtures of samples and/or mixtures of references or mixtures between the two. For example, reference sets can be grouped and compared to a sample set. The latter described methods are useful for determining whether a particular sample shares one or more signal patterns present in the mixture of reference sets or a previously acquired pattern of a sample mixture, for example.

A biomolecule can be any polymeric biological molecule. Examples of biomolecule sequences include nucleic acid sequences, such as DNA and RNA and derivatives thereof, and amino acid sequences, such as peptide, polypeptide and protein sequences, for example. A sequence variation can be any type of variation in a biomolecule sequence, including, but not limited to, a substitution of one or more nucleotides, a single-nucleotide polymorphism, an insertion of one or more nucleotides or a deletion of one or more nucleotides. Biomolecules also can be non-protein and non-nucleic acid molecules, such as lipids and carbohydrates, for example. For non-amino acid and non-nucleotide molecules, determining the presence or absence of a sequence generally involves analyzing signals arising from the molecules or cleavage products or fragments thereof (e.g., mass signals and/or intensities corresponding to lipid molecules or portions thereof).

A signal can be any type of signal representative of a biomolecule fragment sequence that can be measured by a person of ordinary skill in the art. Signals include, but are not limited to, gel electrophoresis signals, capillary electrophoresis signals, fluorescence signals, and mass spectrometry signals (e.g., signals generated by MALDI-TOF or other mass spectrometry processes). A mass spectrometry signal can be a mass signal and can be expressed as a mass to charge ratio. The intensity of a mass spectrometry signal or other signal can depend on the copy number or amount of a particular cleavage product represented by the signal. A target biomolecule sequence in certain embodiments can be, but is not limited to, a single sequence, a mixture of sequences, a mixture of different sequence regions or a mixture of different cleavage reactions. A target biomolecule sequence can be one or more sequence signatures of a sample biomolecule sequence or reference biomolecule sequence. A sequence can be a string of nucleic acids in a sequence or any composition of stretches of DNA or RNA.

A bitmap score can be calculated by comparing intensities of detected and individual reference peak patterns weighted by reference peak intensity. The discriminating feature matching score can be calculated by evaluating a subset of features that discriminate one feature pattern from another or one set of patterns from another set. A distance score can be based on any appropriate type of distance selected by the person of ordinary skill in the art, such as an Euclidian distance, for example. The distance score may be calculated based on distance of the identified feature vectors to all reference feature vectors. The peak pattern identity score can be calculated from the sum of the matched peak intensities, missing and additional peak intensities, silent missing peak intensities and silent additional peak intensities.

Top-ranked matching peak patterns can be identified by iteratively re-scoring matching peak patterns in (b) of process above in about five or more, in about ten or more, in about 50 or more or in about 100 or more cycles or in about 1000 or more cycles.

A sample set of mass signals may be subject to one or more signal processing methods selected from the group consisting of peak detection, calibration, normalization, spectra quality, intensity scaling and compomer adjustment filters. A compomer is a cleavage product with a specific nucleotide composition, as described in greater detail hereafter. Signals based on adducts (e.g. salt matrix doubly charged molecules, degenerate primer signals, abortive cycling products) as a result of the biochemistry in combination with the applied data acquisition tool, which are not referring to the features of the reference, can be identified and explained. These products can also be referred to as e.g. byproducts, chemical noise or impurities. The reference set of mass signals can be derived from cleavage products resulting from a reference biomolecule virtually contacted with the specific cleavage agent. The reference set of mass signals may be subject to clustering. Clustering can be based upon peak masses and peak intensities, or can be based on one or more components of signals described herein.

An adjChange score can be the sum of the adjMissing, adjMismatch and adjExtra score. The adjMissing score can be the sum of missing peak intensities weighted by reactions. The adjMismatch score can be the sum of mismatch peak intensities weighted by reactions. Mismatches are signals expected for the reference set, but not for the particular sample reference. The adjExtra score is the sum of additional peak intensities weighted by the reaction performed. Extra signals are signals not expected for the reference set.

(a)(ii) can be two or more reference sets of mass signals each derived from cleavage products resulting from a reference biomolecule contacted with, or virtually contacted with, the specific cleavage agent. Each of the reference sets can be compared to the sample set. The reference sets may be mixed and compared as a single set to the sample set. Accordingly, reference set of mass signals can be derived by single references, mixtures of references from different origin (e.g. samples) or different regions or different cleavage reactions, for example. Reference sets of signals in certain embodiments can be derived from cleavage products resulting from a variety of types of sequence sources, including but not limited to an a genomic signature region of an organism (mammal, animal, plant or single celled life forms), such as a eukaryotic or prokaryotic organism (e.g., microbial (bacterial), fungal organism, healthy (non-pathogenic) or unhealthy (pathogenic) organism, dead or alive organism) and viruses. Mixtures can be prepared from other sources as well, such as from cancer and forensics samples, for example. Mixed sample sets can be resolved by comparison to a reference set. The reference sets can be individual sequences or mixtures and derivates thereof (e.g. concatenated sequences, sequences with different modified nucleotides, consensus sequences).

A sample set and/or reference set is derived from one or more of (i) a first primer product contacted or virtually contacted with a first specific cleavage agent; (ii) a second primer product contacted or virtually contacted with a first specific cleavage agent; (iii) the first primer product contacted or virtually contacted with a second specific cleavage agent; (iv) the second primer product contacted or virtually contacted with a second specific cleavage agent. Any useful number of specific cleavage reagents may be utilized, and signals generated from the use of one, two, three, four, five, six, seven, eight, nine, ten or more specific cleavage agents may be analyzed. The first primer product can be a forward primer product, the second primer product can be a reverse primer product, the first primer product can be a T7 primer product, and the second primer product can be a SP6 primer product, in some embodiments. Or vice versa. Alternatively two PCR primer products can be amplified with a T7 forward product and a corresponding non transcribable tag and a T7 reverse product and a corresponding non transcribable tag. The same applies for the SP6. Other RNA or RNA/DNA polymerase promoters also may be utilized as known and selected by the person of ordinary skill in the art. Prometers for mutant polymerases can be utilized, such as for polymerases that can extend with modified (unnatural) nucleotides.

A set of mass signals can be prepared by a method comprising: (a) contacting a sample DNA with a primer, (b) extending the primer to form a primer product; (c) transcribing the primer product to form a primer product RNA; (d) contacting the primer product RNA with a specific cleavage agent to form cleavage products; and (e) preparing a set of mass signals from the cleavage products. A primer may be extended by an amplification process and amplified primer products can be prepared (e.g., using linear or exponential amplification). In certain embodiments, the amplification process is a polymerase chain reaction process (PCR) or any other applicable exponential amplification method known by the person of ordinary skill in the art. The set of mass signals may be prepared by mass spectrometric analysis, and sometimes the mass spectrometric analysis is MALDI-TOF, ESI or O-TOF.

A set of mass signals can be prepared by a method comprising: (a) contacting a sample DNA with a first primer and a second primer; (b) extending the first primer and the second primer by an amplification process to form an amplified first primer product and an amplified second primer product; (c) transcribing the first primer product and the second primer product to form a first primer product RNA and a second primer product RNA; (d) contacting the first primer product RNA and the second primer product RNA with a first specific cleavage agent to form a first cleavage product set and a second cleavage product set; (e) contacting the first primer product RNA and the second primer product RNA with a second specific cleavage agent to form a third cleavage product set and a fourth cleavage product set; and (f) preparing a set of mass signals for each cleavage product set. As noted above, processes described herein can be carried out with any useful number of cleavage agents (e.g., one to ten specific cleavage agents), and cleavage product sets from each specific cleavage reaction product set can be analyzed. Further, any type of useful cleavage agent can be utilized, as described herein (e.g., RNAse T1, RNaseA or other cleavage agent).

The sample may be obtained from any applicable source, such as an organism (e.g., pathogen, microbe, virus, animal (e.g., mammalian, human sample), an agricultural sample (e.g., plant sample) or an environmental sample (e.g., soil sample, building sample). In certain embodiments, the sample may be from a subject diagnosed with a disease (e.g., cancer) or microbial Infection, can be from a subject as part of a forensic analysis, and can be from a pregnant female at any stage of gestation (e.g., within the first trimester, within the second trimester, within the third trimester) as part of prenatal testing, for example.

Process described herein can be carried out on nucleic acid fragments generated from amplification processes that generate fragments of a target sequence. Amplification processes are all processes, which create multiple copies of DNA or RNA single or double stranded or fragments thereof using living organisms, enzymes, enzyme systems or any biochemical or chemical agent. Thus, peak patterns can be determined from fragments generated by such amplification processes in lieu of cleaved products resulting from specific cleavage of a target sequence. An example of such amplification processes include without limitation linear and exponential amplification methods (e.g. primer extension methods, PCR, ligase chain reaction, in vitro transcription, cloning, RNA amplification processes).

Provided also are program products for use in a computer that executes program instructions recorded in a computer-readable media to determine the presence of a target biomolecule sequence of a sample, the program product comprising: a recordable media; and a plurality of computer-readable program instructions on the recordable media that are executable by the computer to perform a process as described herein.

Also provided are computer-based processes for determining the presence of a target biomolecule sequence of a sample, which may comprise elements of any processes described herein. For example, a computer-based process may comprise, for example: (a) identifying and scoring matching peak patterns between (i) a sample set of signals entered into the computer that are derived from cleavage products resulting from contacting a biomolecule in the sample with a specific cleavage agent and (ii) a reference set of signals entered into the computer that are derived from cleavage products resulting from a reference biomolecule contacted with, or virtually contacted with, the specific cleavage agent; wherein the scoring is based upon one or more criteria selected from the group consisting of a bitmap score, a discriminating feature matching score, a distance score, a peak pattern identity score and an adjChange score; (b) identifying one or more top-ranked matching peak patterns; wherein the one or more top-ranked matching peak patterns are identified by iteratively re-scoring matching peak patterns in a subset of top-ranked matching peak patterns between the sample set of signals and the reference set of signals; (c) identifying potential sequence variations (e.g., mutations) in the biomolecule sequence of the one or more top-ranked matching peak patterns of the reference set; (d) determining the presence or absence or identity of the target biomolecule sequence in the sample by the match between the one or more top-ranked matching peak patterns; and (e) assigning a confidence value to the match between the one or more top-ranked matching peak patterns (f) assigning a probability value for the likelihood of any further sequence variations. Step (a)(i) can include identifying and scoring matching peak patterns using a reference set of samples.

Provided also are systems for high throughput automated analysis for determining the presence or identification of a target biomolecule sequence of a sample, which comprise: a processing station that cleaves a biomolecule (e.g., with one or more specific cleavage reagents); a robotic system that transports or transfers the resulting cleavage products from the processing station (e.g., fragments or cleavage products) to a measuring station, wherein one or more analyte-specific measurements are determined (e.g., mass and/or length determined by mass spectrometry); and a data analysis system that processes the data from the measuring station by performing the computer-based process of any one of the embodiments set forth herein to identify the presence of the target biomolecule sequence in the sample. Included in this can be a barcoding system for sample tracking.

Analyses described herein can be qualitative and quantitative analyses. For example, the amount of a particular target sequence, or the relative amount of a particular signal in a sample can be determined or the relative or absolute amount of different target sequences, for example. An internal control can be utilized in the processes described herein, which can be useful In  quantitative analyses. An internal control can be a known quantity of a known sequence, and an internal control may be part of a reference set. An internal control can be generated, for example, from mass modified nucleotides, chemically or enzymatically modified nucleotides. An internal control also may be a methylated or de-methylated nucleic acid. It can be a modified or non-modified amino acid, or fatty acid or saccharide or a sequence of them. It can be any modification, which creates a mass difference between the detectable cleaved product and any internal control, whether cleavable or non-cleavable.

One of ordinary skill in the art can identify different parameter sets e.g. the normal parameter set is used when samples are expected to match one of the sequences in the reference set except a few point mutations. Anchor peaks for peak matching quality are selected from simulated peak patterns of the reference sequence set so that at least one peak in each anchor peak group will be found for any reference sequence. Spectrum quality is calculated by combining contributions derived from pek intensities and peak SNRs with that derived from anchor peak matching in a 33% and 67% ratio.

The relaxed parameter set is used when samples are expected to be far away from the known reference sequences in the reference set, e.g., reference set with only one known sequence. Anchor peaks for peak matching quality are selected from simulated peak patterns of the reference sequence set so that at least two peaks in each anchor peak group will be found in any reference sequence. Spectrum quality is calculated by combining contribution derived from peak intensities and peak SNRs with that derived from anchor peak matching in a 90% and 10% ratio.

Also provided are kits for conducting the processes described herein. Embodiments and features of the invention are described in greater detail in the claims.

Brief Description of the Drawings

Figure 1: Flow diagram of the procedural steps involved in comparative sequence analysis by PCR, *in vitro* transcription, base-specific cleavage and MALDI-TOF MS. Step 1: Import of references (e.g., sequences or patterns) into the system database; Step 2: PCR and Post-PCR biochemistry including a suitable clean-up step; Step 3: MALDI-TOF MS sample specific fingerprint and peak pattern comparison; Step 4: Tabulated identification (e.g., typing) results including sequence variations with probability and confidence assignments.

Figure 2: Comparative sequence analysis result screen. Best matching reference signals (e.g., sequences), confidence, deviations and variation probability for each of the samples are  displayed. Details windows show mass spectrometry data and matching scores as well as *in silico* banding patterns.

Figure 3: Flow Chart of probability calculation using a probability model.

Figure 4: Analysis options

Figure 5: MALDI-TOF MS multi-locus sequencing typing (MLST) statistics of 96 typeable *N. meningitidis* samples. For 97.6% of the sample alleles the software automatically assigned the correct top matching reference sequence, for 1.8% the correct matching reference was listed among a group of top matching references with equal score and for 0.6% a wrong reference sequence was presented.

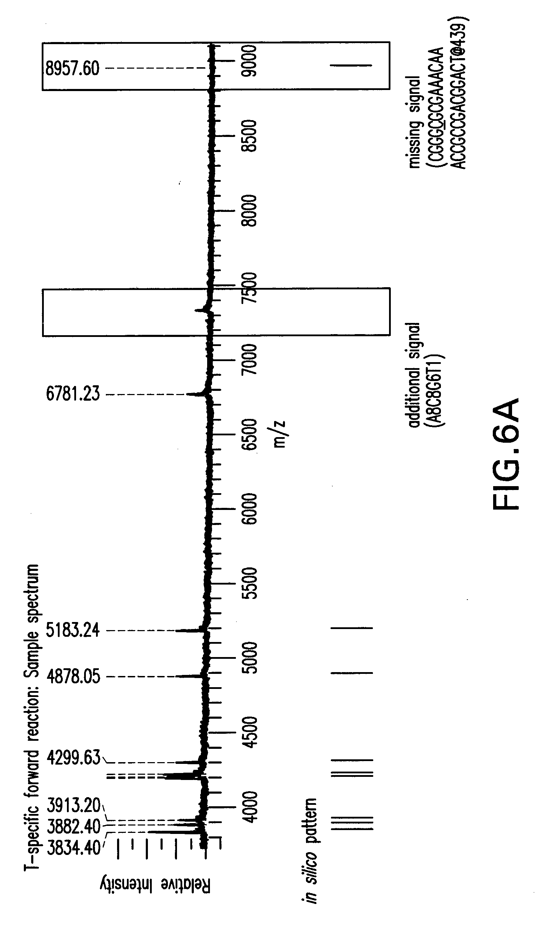

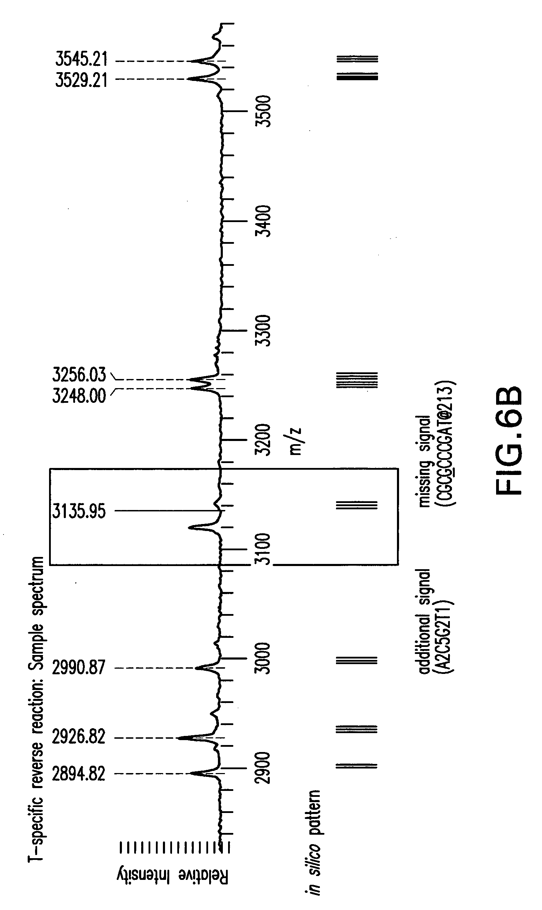

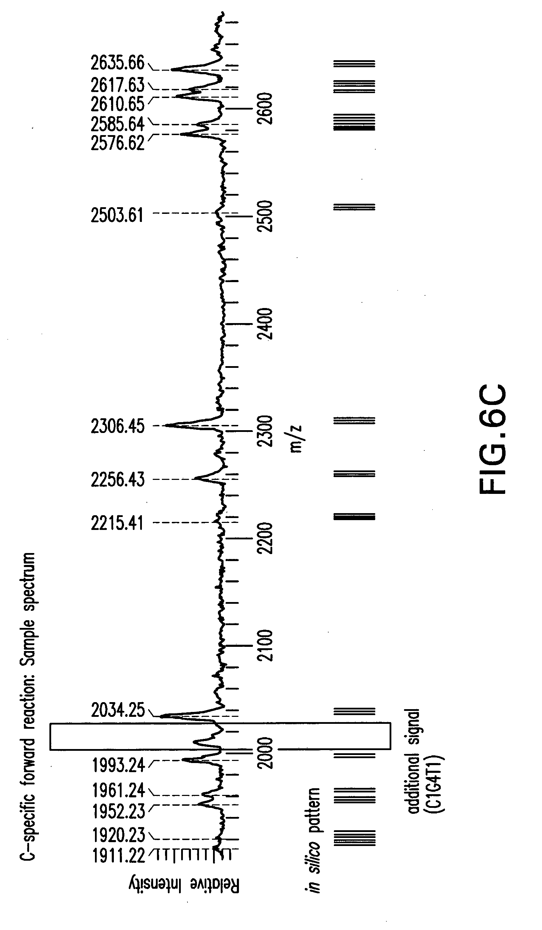

Figure 6: Base-specific cleavage and MALDI-TOF MS based discovery of a mutation C to T in allele *aroE9* at position 443. Mutation specific changes in comparison to the simulated banding pattern of the best matching reference sequence *aroE9* are highlighted. (A) Overlay of the mass spectrum of the T-specific cleavage reaction of the forward RNA transcript and the banding pattern of the *in silico* cleavage with mutation specific signal changes at 7343.5 and 8957.9 Da. (B) Overlay of the mass spectrum of the T-specific cleavage reaction of the reverse RNA transcript and the banding pattern of the *in silico* cleavage with mutation specific signal changes at 3120.0 and 3136.0 Da. (C) Overlay of the mass spectrum of the C-specific cleavage reaction of the forward RNA transcript and the banding pattern of the *in silico* cleavage with mutation specific signal change at 2010.0 Da.

Figure 7: (A) Unweighted pair group method (UPGMA) tree of base-specific cleavage and MALDI-TOF MS patterns in comparison to (B) a UPGMA tree derived from the primary sequences of the same sample set. Samples are labeled by allele and sample number (x_y). ED 2.8 is the cutoff for the degree of spectra similarity between identical samples. Clades that are defined by one tree but not by the other are highlighted by asterisks (*).

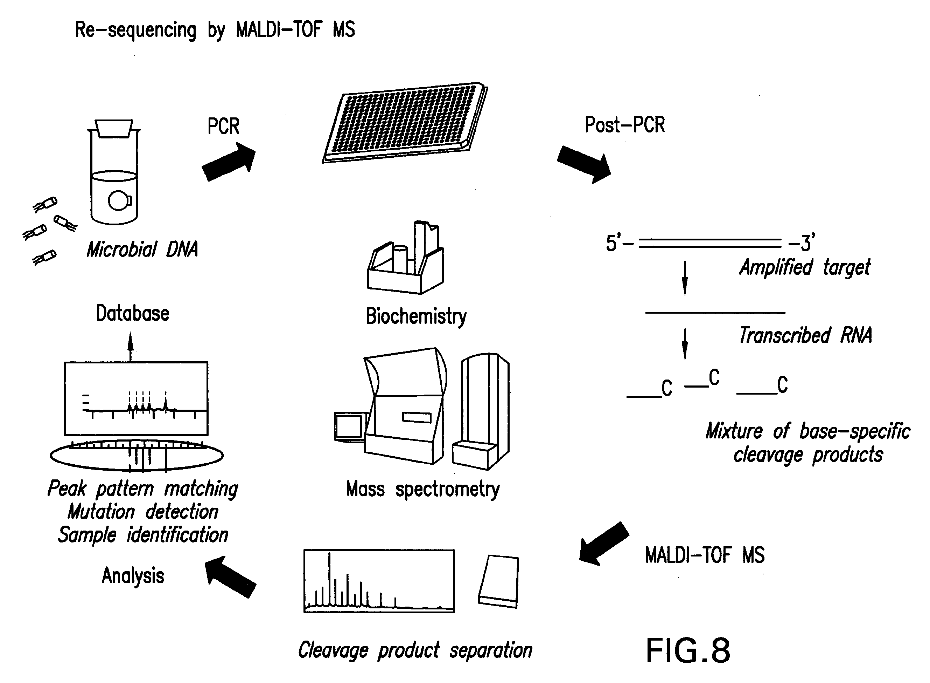

Figure 8 shows a general schematic for a mass spectrometry comparative sequence analysis embodiment involving re-sequencing.

Figure 9 shows a general representation of cleavage processes involving compomer analysis of mass spectrometric signals.

Figure 10 is a general depiction of an embodiment for synthesizing mass signal sets.

Figure 11 is a general depiction of a peak processing embodiment.

Figure 12 depicts a peak pattern matching analysis embodiment.

Figure 13 is a general depiction of an iterative pattern matching and scoring embodiment.

Figure 14 shows a flow diagram for certain comparative sequence analysis embodiments involving the comparison of sample signal sets to one or more reference signal sets using signature sequence identification analyses.

Figure 15 depicts a flow diagram for certain comparative sequence analysis embodiments involving the comparison of sample mass signal sets using clustering analyses.

Figure 16 shows a flow diagram of a process embodiment for calculating a confidence value.

Figure 17 shows a comparative sequence analysis system embodiment.

Figure 18 shows a computer-based method embodiment.

Detailed Description

The beginning of this millennium has seen dramatic advances in genomic research. Milestones like the complete sequencing of the human genome and of many other species were achieved and complemented by the systematic discovery of variations. Public and private databases provide comprehensive reference sets for comparative sequence and variation analysis. Efficient comparison of the information contained therein is one of today's focuses in biology, evolution and medicine. The majority of sequencing applications are thus currently focused on comparative sequencing - that is, sequencing a multitude of individuals in parallel on a specific set of genomic regions or the entire genome if possible to ascertain variation within a population and thus to define new informative DNA marker sets.

The continuing progress of genome projects provide the basis for the identification of large sets of DNA markers, stretches of polymorphic nucleotide sequence. They have been provided useful in assessing inter-and intra-species specific variations and help to understand the genetic contributions to phenotypic expression of an organism. DNA markers are widely used in diverse applications including criminal suspect identification, linkage analysis, pharmacogenomics or routine clinical diagnostics and will be of increasing importance in the future improving treatment monitoring and providing personalized medicine.

Comparison of genome sequences from evolutionarily diverse species (intra- and inter-species comparisons) has emerged as a powerful tool for identifying functionally important genomic elements and understanding biological pathways.

Development, evaluation and application of genome-based diagnostic methods are of value for the detection of an infectious agent, the prediction of susceptibility to disease, prediction of drug response, accurate molecular classification of disease. In addition, identification of gene variants that contribute to good health and resistance to disease or in microbes to antibiotic resistance are   needed as well as genome based approaches to prediction of disease susceptibility and drug response, early detection of disease, and molecular taxonomy of disease states.

Comparative sequence analysis in microbial genomes for characterization is the specific identification and differentiation of a microorganism to the genus, species or strain-specific level as well as the classification of its source. These are important aspects for the recognition and monitoring of microbial outbreaks in clinical settings and pharmaceutical production environments.

For global surveillance of infectious diseases new technologies for whole genome comparative sequencing currently are prohibitively expensive and lack ease of use to allow for the comparison of large numbers of isolates in an automated high-throughput scenario. The same obstacles apply for whole genome DNA microarrays and their routine application in epidemiology. Future use still requires the reduction of costs per reaction, robust and simplified formats focused on established regions of genetic variance and an adequate evaluation in comparison with other molecular methods. Ambiguities in the interpretation of the ratios of hybridization and cross-hybridization to paralogous genes are important limitation of the technique. In addition, PCR product microarrays generally do not have the resolution to detect minor deletions and point mutations (Garaizar et al. 2006).

Accordingly, typing methods based on PCR amplified DNA marker regions and nucleotide sequence analysis like dideoxy sequencing or comparative sequence analysis by MALDI-TOF MS are important alternatives. Probing large collections of microbial isolates utilizing a partial genetic signature provides the framework for these sequence-based typing approaches (van Belkum 2003). PCR techniques make the analysis of molecular marker regions easily achievable even for trace amounts of material, uncultured species or clinical samples. The resulting DNA sequences allow for the construction of electronically accessible genetic databases, which are most applicable to prospective epidemiologic surveillance efforts and allow for the data transfer between centers (Pfaller 1999).

Over the past decade microbial marker regions like 16S or 23S rDNA, see e.g. Woese (1997) Nucleic Acid Research, 25(1), 109-11, as well as informative typing approaches like multi-locus sequence typing (MLST) have been established for microbial characterization by comparative sequence analysis. Multi-locus sequence typing was introduced in 1998 as a comparative sequencing method to assess the population structure of bacterial isolates. MLST elucidates the genomic relatedness at the inter- and intra-species level using dideoxy sequencing of a restricted number of house-keeping genes. The use of multiple loci is essential to achieve the resolution required to provide meaningful relationships among strains. It can be important to follow diversification of clones with age as a consequence of mutational or recombinational events (Maiden 2006; Maiden et al. 1998; Urwin and Maiden 2003).

MLSTs can be obtained from clinical material (e.g., cerebrospinal fluid or blood) by PCR amplification and isolates can be precisely characterized even if they can not be cultured (Enright and Spratt 1999). Data are unambiguous and can easily be compared to those in a large central database via the Internet. As of today, the continuously expanding MLST database covers 18 species. Additional schemes are under constant development and can include antigene regions like known for e.g. MAST typing or *N. gonorrhoeae* as well as antibiotic resistance regions.

The standardized application of existing signature sequences like, e.g., MLST or 16S and 23S rDNA loci, in the clinical research environment and the identification of new informative marker sets require liquid handling robotics, standardized protocols and an automated analysis platform.

Base-specific endonuclease digests of RNA followed by MALDI-TOF MS provide a solution for nucleic acid mass fingerprinting and comparative sequence analysis. PCR amplified genetic signature sequences are subject to *in vitro* transcription and base-specific RNA cleavage. Subsequently, specific mass signal patterns of the resulting cleavage products, a mixture of RNA compomers are acquired and provide a fingerprint of the sample. Since the exact masses of each of the bases in the RNA compomers are known, the high precision obtained by MALDI-TOF MS is used to derive a base composition of each signal. The list of possible base compositions is constrained by the single representation of the known cleavage base at the 3'-end of the compomer.

After annotation and calibration of the data, the detected list of experimental compomer masses is compared to a calculated list of molecular weights derived from an *in silico* digest of a set of reference sequences in the system database. These simulated patterns of the reference set are the comparative measure to identify the sample by its best matching reference sequence and deliver the homology with the best fit.

Microheterogeneities between the best matching reference and the sample sequence, such as single base deviations, affect one or more cleavage products of the compomer mixture and show up as a deviation between the *in silico* and the detected sample spectrum. Time-efficient algorithms utilize these detected deviations to identify and localize sequence differences down to single base pair change (Bocker 2003; Stanssens et al. 2004) and identify novel sequences.

Processes and systems described herein find multiple uses to the person of ordinary skill in the art. Such processes and systems can be utilized, for example, to: (a) rapidly determine whether a particular target sequence is present in a sample; (b) perform mixture analysis, e.g., identify a mixture and/or its composition or determine the frequency of a target sequence in a mixture (e.g., mixed communities, quasispecies); (c) prepare parameter sets; (d) detect sequence variations (e.g., mutations, single nucleotide polymorphisms) in a sample; (e) perform haplotyping determinations; (f) perform pathogen typing; (g) detect the presence or absence of a viral or bacterial target sequence in a sample; (h) profile antibiotics, profile antibiotic resistance; (i) identify disease markers; (j) detect microsatellites; (k) identify short tandem repeats; (l) identify an organism or organisms; (m) detect allelic variations; (n) determine allelic frequency; (o) determine methylation patterns; (p) perform epigenetic determinations; (q) re-sequence a region of a biomolecule; (r) perform multiplex analysis; (s) human clinical research and medicine (e.g. cancer marker detection, sequence variation detection; detection of sequence signatures favorable or unfavorable for a particular drug administration), (t) HLA typing; (u) forensics; (v) vaccine quality control; (w) treatment monitoring; (x) vector identity; (y) perform vaccine or production strain QC; (z) detect mutants e.g. disease mutant; (aa) test strain identity and (ab) detect the identity of a nucleic acid sequence stretch in general in any context of direct or indirect measurement as an identification tag.

<u>Definitions</u>

Unless defined otherwise, all technical and scientific terms used herein have the same meaning as is commonly understood by one of skill in the art to which the invention(s) belong. In the event that there are a plurality of definitions for terms herein, those in this section prevail. Where reference is made to a URL or other such identifier or address, it is understood that such identifiers can change and particular information on the internet can come and go, but equivalent information can be found by searching the internet. Reference thereto evidences the availability and public dissemination of such information.

As used herein, a molecule refers to any molecular entity and includes, but is not limited to, biopolymers, biomolecules, macromolecules or components or precursors thereof, such as peptides, proteins, organic compounds, oligonucleotides or monomeric units of the peptides, organics, nucleic acids, modified nucleic acids and other macromolecules. A monomeric unit refers to one of the constituents from which the resulting compound is built. Thus, monomeric units include, nucleotides, amino acids, and pharmacophores from which small organic molecules are synthesized.

As used herein, a biomolecule is any molecule that occurs in nature, or derivatives thereof. Biomolecules include biopolymers and macromolecules and all molecules that can be isolated from living organisms and viruses, including, but are not limited to, cells, tissues, prions, mammals, animals, plants, viruses, bacteria, prions and other organsims. Biomolecules also include, but are not limited to oligonucleotides, oligonucleosides, ribonucleotides, ribonucleosides, proteins, peptides, amino acids, lipids, steroids, peptide nucleic acids (PNAs), oligosaccharides and monosaccharides, organic molecules, such as enzyme cofactors, metal complexes, such as heme, iron sulfur clusters, porphyrins and metal complexes thereof, metals, such as copper, molybedenum, zinc and others. Biomolecules can as well be tags used as identifiers.

As used herein, macromolecule refers to any molecule having a molecular weight from the hundreds up to the millions. Macromolecules include, but are not limited to, peptides, proteins, nucleotides, nucleic acids, carbohydrates, and other such molecules that are generally synthesized by biological organisms, but can be prepared synthetically or using recombinant molecular biology methods.

As used herein, biopolymer refers to biomolecules, including macromolecules, composed of two or more monomeric subunits, or derivatives thereof, which are linked by a bond or a macromolecule. A biopolymer can be, for example, a polynucleotide, a polypeptide, a carbohydrate, or a lipid, or derivatives or combinations thereof, for example, a nucleic acid molecule containing a peptide nucleic acid portion or a glycoprotein.

As used herein "nucleic acid" refers to polynucleotides such as deoxyribonucleic acid (DNA) and ribonucleic acid (RNA) or a combination of the two and any chemical or enzymatic modification thereof (e.g. methylated DNA, DNA of modified nucleotides). The term should also be understood to include, as equivalents, derivatives, variants and analogs of either RNA or DNA made from nucleotide analogs, single (sense or antisense) and double-stranded polynucleotides. Deoxyribonucleotides include deoxyadenosine, deoxycytidine, deoxyguanosine and deoxythymidine. For RNA, the uracil base is uridine.

Reference to a nucleic acid as a "polynucleotide" is used in its broadest sense to mean two or more nucleotides or nucleotide analogs linked by a covalent bond, including single stranded or double stranded molecules. The term "oligonucleotide" also is used herein to mean two or more nucleotides or nucleotide analogs linked by a covalent bond, although those in the art will recognize that oligonucleotides such as PCR primers generally are less than about fifty to one hundred nucleotides in length. The term "amplifying," when used in reference to a nucleic acid, means the repeated copying of a DNA sequence or an RNA sequence, through the use of specific or non-specific means, resulting in an increase in the amount of the specific DNA or RNA sequences intended to be copied.

As used herein, "nucleotides" include, but are not limited to, the naturally occurring nucleoside mono-, di-, and triphosphates: deoxyadenosine mono-, di- and triphosphate; deoxyguanosine mono-, di- and triphosphate; deoxythymidine mono-, di- and triphosphate; and deoxycytidine mono-, di- and triphosphate (referred to herein as dA, dG, dT and dC or A, G, T and C, respectively). Nucleotides also include, but are not limited to, modified nucleotides and nucleotide analogs such as deazapurine nucleotides, e.g., 7-deaza-deoxyguanosine (7-deaza-dG) and 7-deaza-deoxyadenosine (7-deaza-dA) mono-, di- and triphosphates, deutero-deoxythymidine (deutero-dT) mon-, di- and triphosphates, methylated nucleotides e.g., 5-methyldeoxycytidine triphosphate, $^{13}C/^{15}N$ labelled nucleotides and deoxyinosine mono-, di- and triphosphate. For those skilled in the art, it will be clear that modified nucleotides, isotopically enriched, depleted or tagged nucleotides and nucleotide analogs can be obtained using a variety of combinations of functionality and attachment positions.

As used herein, the phrase "chain-elongating nucleotides" is used in accordance with its art recognized meaning. For example, for DNA, chain-elongating nucleotides include 2'deoxyribonucleotides (e.g., dATP, dCTP, dGTP and dTTP) and chain-terminating nucleotides include 2',3'-dideoxyribonucleotides (e.g., ddATP, ddCTP, ddGTP, ddTTP). For RNA, chain-elongating nucleotides include ribonucleotides (e.g., ATP, CTP, GTP and UTP) and chain-terminating nucleotides include 3'-deoxyribonucleotides (e.g., 3'dA, 3'dC, 3'dG and 3'dU) and 2',3'-dideoxyribonucleotides (e.g., ddATP, ddCTP, ddGTP, ddTTP). A complete set of chain elongating nucleotides refers to dATP, dCTP, dGTP and dTTP for DNA, or ATP, CTP, GTP and UTP for RNA. The term "nucleotide" is also well known in the art.

As used herein, the term "nucleotide terminator" or "chain terminating nucleotide" refers to a nucleotide analog that terminates nucleic acid polymer (chain) extension during procedures wherein a DNA or RNA template is being sequenced or replicated. The standard chain terminating nucleotides, i.e., nucleotide terminators include 2',3'-dideoxynucleotides (ddATP, ddGTP, ddCTP and ddTTP, also referred to herein as dideoxynucleotide terminators). As used herein, dideoxynucleotide terminators also include analogs of the standard dideoxynucleotide terminators, e.g., 5-bromo-dideoxyuridine, 5-methyl-dideoxycytidine and dideoxyinosine are analogs of ddTTP, ddCTP and ddGTP, acyclic nucleotides, respectively.

The term "polypeptide," as used herein, means at least two amino acids, or amino acid derivatives, including mass modified amino acids, that are linked by a peptide bond, which can be a modified peptide bond. A polypeptide can be translated from a nucleotide sequence that is at least a portion of a coding sequence, or from a nucleotide sequence that is not naturally translated due, for example, to its being in a reading frame other than the coding frame or to its being an intron sequence, a 3' or 5' untranslated sequence, or a regulatory sequence such as a promoter. A polypeptide also can be chemically synthesized and can be modified by chemical or enzymatic methods following translation or chemical synthesis. The terms "protein," "polypeptide" and "peptide" are used interchangeably herein when referring to a translated nucleic acid, for example, a gene product.

As used herein, a biomolecule fragment, such as a biopolymer fragment, is a smaller portion than the whole. Fragments can contain from one constituent up to less than all. Typically when cleaving, the fragments will be of a plurality of different sizes such that most will contain more than two constituents, such as a constituent monomer.

As used herein, the term "cleavage products" refers to products produced by specific cleavage of a biomolecule. Any known specific cleavage reagent or process known to the person of ordinary skill in the art can be selected and utilized, and examples of such include without limitation specific physical, chemical or enzymatic cleavage of a biomolecule. Cleavage products sometimes are referred to herein as "cleavage fragments" or "fragments." As used herein "cleavage products of a target nucleic acid" refers to cleavage products produced by specific physical, chemical or enzymatic cleavage of the target nucleic acid. As used herein, specific cleavage products or fragments obtained by specific cleavage refers to cleavage products or fragments that are cleaved at a specific position in a target nucleic acid sequence based on the base/sequence specificity of the cleaving reagent (e.g., A, G, C, T or U, or the recognition of modified bases or nucleotides); or the recognition of certain features/motifs e.g. sequence specific motivs (e.g. restriction enzymes) or the structure of the target nucleic acid; or physical processes, such as ionization by collision-induced dissociation during mass spectrometry; or a combination thereof. Fragments can contain from one up to less than all of the constituent nucleotides of the target nucleic acid molecule. The collection of fragments from such cleavage contains a variety of different size oligonucleotides and nucleotides. Fragments can vary in size, and suitable nucleic acid fragments are typically less that about 2000 nucleotides. Suitable nucleic acid fragments can fall within several ranges of sizes including but not limited to: less than about 1000 bases, between about 100 to about 500 bases, from about 25 to about 200 bases, from about 3 to about 50 bases, from about 2 to about 30 bases or from about 4 to about 30 bases. In some aspects, fragments of about one nucleotide may be present in the set of products obtained by specific cleavage.

As used herein, a target nucleic acid refers to any nucleic acid of interest in a sample. It can contain one or more nucleotides. A target nucleotide sequence refers to a particular sequence of nucleotides in a target nucleic acid molecule. Detection or identification of such sequence results in detection of the target and can indicate the presence or absence of a particular mutation, sequence variation (mutation or polymorphism). Similarly, a target polypeptide as used herein refers to any polypeptide of interest whose mass is analyzed, for example, by using mass spectrometry to determine the amino acid sequence of at least a portion of the polypeptide, or to  determine the pattern of peptide fragments of the target polypeptide produced, for example, by treatment of the polypeptide with one or more endopeptidases. The term "target polypeptide" refers to any polypeptide of interest that is subjected to mass spectrometry for the purposes disclosed herein, for example, for identifying the presence of a polymorphism or a mutation. A target polypeptide contains at least 2 amino acids, generally at least 3 or 4 amino acids, and particularly at least 5 amino acids, but can be longer. A target polypeptide can be encoded by a nucleotide sequence encoding a protein, which can be associated with a specific disease or condition, or a portion of a protein. A target polypeptide also can be encoded by a nucleotide sequence that normally does not encode a translated polypeptide. A target polypeptide can be encoded, for example, from a sequence of dinucleotide repeats or trinucleotide repeats or the like, which can be present in chromosomal nucleic acid, for example, a coding or a non-coding region of a gene, for example, in the telomeric region of a chromosome. The phrase "target sequence" as used herein refers to either a target nucleic acid sequence or a target polypeptide or protein sequence or small RNAs (microRNAs).

A process as disclosed herein also provides a means to identify a target polypeptide by mass spectrometric analysis of peptide fragments of the target polypeptide. As used herein, the term "peptide fragments of a target polypeptide" refers to cleavage fragments produced by specific chemical or enzymatic degradation of the polypeptide. The production of such peptide fragments of a target polypeptide is defined by the primary amino acid sequence of the polypeptide, since chemical and enzymatic cleavage occurs in a sequence specific manner. Peptide fragments of a target polypeptide can be produced, for example, by contacting the polypeptide, which can be immobilized to a solid support, with a chemical agent such as cyanogen bromide, which cleaves a polypeptide at methionine residues, or hydroxylamine at high pH, which can cleave an Asp-Gly peptide bond; or with an endopeptidase such as trypsin, which cleaves a polypeptide at Lys or Arg residues.

The identity of a target polypeptide can be determined by comparison of the molecular mass or sequence with that of a reference or known polypeptide. For example, the mass spectra of the target and known polypeptides can be compared.

As used herein, the term "corresponding or known polypeptide or nucleic acid" is a known polypeptide or nucleic acid generally used as a control or reference to determine, for example, whether a target polypeptide or nucleic acid is an allelic variant of the corresponding known polypeptide or nucleic acid or for its identification. It should be recognized that a corresponding known protein or nucleic acid can have substantially the same amino acid or base sequence as the target polypeptide, or can be substantially different. For example, where a target polypeptide is an allelic variant that differs from a corresponding known protein by a single amino acid difference, the amino acid sequences of the polypeptides will be the same except for the single amino acid difference. Where a mutation in a nucleic acid encoding the target polypeptide changes, for example, the reading frame of the encoding nucleic acid or introduces or deletes a STOP codon, the sequence of the target polypeptide can be substantially different from that of the corresponding known polypeptide.

As used herein, a reference biomolecule refers to a biomolecule, which is generally, although not necessarily, to which a target biomolecule is compared. Thus, for example, a reference nucleic acid is a nucleic acid to which the target nucleic acid is compared in order to identify potential or actual sequence variations in the target nucleic acid relative to the reference nucleic acid. Reference nucleic acids typically are of known sequence or of a sequence that can be determined. This can be a sequence or just a pattern.

As used herein, a reference polypeptide is a polypeptide to which the target polypeptide is compared in order to identify the polypeptide in methods that do not involve sequencing the polypeptide. Reference polypeptides typically are known polypeptides. Reference sequence, as used herein, refers to a reference nucleic acid or a reference polypeptide or protein sequence.

As used herein, transcription-based processes include *"in vitro* transcription system", which refers to a cell-free system containing an RNA polymerase and other factors and reagents necessary for transcription of a DNA molecule operably linked to a promoter that specifically binds an RNA polymerase. An *in vitro* transcription system can be a cell extract, for example, a eukaryotic cell extract. The term "transcription," as used herein, generally means the process by which the production of RNA molecules is initiated, elongated and terminated based on a DNA template. In addition, the process of "reverse transcription," which is well known in the art, is considered as encompassed within the meaning of the term "transcription" as used herein. Transcription is a polymerization reaction that is catalyzed by DNA-dependent or RNA-dependent RNA polymerases. Examples of RNA polymerases include the bacterial RNA polymerases, SP6 RNA polymerase, SP6 RNA and DNA polymerase, T3 RNA polymerase, T3 RNA polymerase, T7 RNA polymerase and T7 RNA and DNA polymerase as well as any mutant variant thereof

As used herein, the term "translation" describes the process by which the production of a polypeptide is initiated, elongated and terminated based on an RNA template. For a polypeptide to be produced from DNA, the DNA must be transcribed into RNA, then the RNA is translated due to the interaction of various cellular components into the polypeptide. In prokaryotic cells, transcription and translation are "coupled", meaning that RNA is translated into a polypeptide during the time that it is being transcribed from the DNA. In eukaryotic cells, including plant and animal cells, DNA is transcribed into RNA in the cell nucleus, then the RNA is processed into mRNA, which is transported to the cytoplasm, where it is translated into a polypeptide.

The term "isolated" as used herein with respect to a nucleic acid, including DNA and RNA, refers to nucleic acid molecules that are substantially separated from other macromolecules normally associated with the nucleic acid in its natural state. An isolated nucleic acid molecule is substantially separated from the cellular material normally associated with it in a cell or, as relevant, can be substantially separated from bacterial or viral material; or from culture medium when produced by recombinant DNA techniques; or from chemical precursors or other chemicals when the nucleic acid is chemically synthesized. In general, an isolated nucleic acid molecule is at least about 50% enriched with respect to its natural state, and generally is about 70% to about 80% enriched, particularly about 90% or 95% or more. Preferably, an isolated nucleic acid constitutes at least about 50% of a sample containing the nucleic acid, and can be at least about 70% or 80% of the material in a sample, particularly at least about 90% to 95% or greater of the sample. An isolated nucleic acid can be a nucleic acid molecule that does not occur in nature and, therefore, is not found in a natural state.

The term "isolated" also is used herein to refer to polypeptides that are substantially separated from other macromolecules normally associated with the polypeptide in its natural state. An isolated polypeptide can be identified based on its being enriched with respect to materials it naturally is associated with or its constituting a fraction of a sample containing the polypeptide to the same degree as defined above for an "isolated" nucleic acid, i.e., enriched at least about 50% with respect to its natural state or constituting at least about 50% of a sample containing the polypeptide. An isolated polypeptide, for example, can be purified from a cell that normally expresses the polypeptide or can be produced using recombinant DNA methodology.

As used herein, "structure" of the nucleic acid includes but is not limited to secondary structures due to non-Watson-Crick base pairing (see, e.g., Seela, F. and A. Kehne (1987) Biochemistry, 26, 2232-2238.) and structures, such as hairpins, loops and bubbles, formed by a combination of base-paired and non base-paired or mis-matched bases in a nucleic acid.

As used herein, epigenetic changes refer to variations in a target sequence relative to a reference sequence (e.g., a mutant sequence relative to the wild-type sequence) that are not dependent on changes in the identity of the natural bases (A, G, C, T/U) or the twenty natural amino acids. Such variations include, but are not limited to, e.g.,

differences in the presence of modified bases or methylated bases between a target nucleic acid sequence and a reference nucleic acid sequence. Epigenetic changes refer to mitotically and/or meiotically heritable changes in gene function or changes in higher order nucleic acid structure that cannot be explained by changes in nucleic acid sequence. Examples of systems that are subject to epigenetic variation or change include, but are not limited to, DNA methylation patterns in animals, histone modification and the Polycomb-trithorax group (Pc-G/tx) protein complexes. Epigenetic changes usually, although not necessarily, lead to changes in gene expression that are usually, although not necessarily, inheritable.

As used herein, a "primer" refers to an oligonucleotide that is suitable for hybridizing, chain extension, amplification and sequencing. Similarly, a probe is a primer used for hybridization. The primer refers to a nucleic acid that is of low enough mass, typically about between about 3 and 200 nucleotides, generally about 70 nucleotides or less than 70, and of sufficient size to be conveniently used in the methods of amplification and methods of detection and sequencing provided herein. These primers include, but are not limited to, primers for detection, amplification, transcription initiation and sequencing of nucleic acids, which require a sufficient number nucleotides to form a stable duplex, typically about 6-30 nucleotides, about 10-25 nucleotides and/or about 12-20 nucleotides. Thus, for purposes herein, a primer is a sequence of nucleotides contains of any suitable length, typically containing about 6-70 nucleotides, 12-70 nucleotides or greater than about 14 to an upper limit of about 70 nucleotides, depending upon sequence and application of the primer. A primer may include one or more tags to facilitate a process (e.g., in vitro transcription).

As used herein, reference to mass spectrometry encompasses any suitable mass spectrometric format known to those of skill in the art. Such formats include, but are not limited to, Matrix-Assisted Laser Desorption/Ionization, Time-of-Flight (MALDI-TOF), Electrospray (ES), IR-MALDI (see, e.g., published International PCT application No.99/57318 and U.S. Pat. No. 5,118,937), Ion Cyclotron Resonance (ICR), Fourier Transform and combinations thereof. MALDI formats, particular UV and IR, Ortagonal TOF (OTOF) are useful formats for conducting processes described herein.

As used herein, mass spectrum refers to the presentation of data obtained from analyzing a biopolymer fragment or cleavage product thereof by mass spectrometry either graphically or encoded numerically.

As used herein, pattern or cleavage pattern or fragmentation pattern or fragmentation spectrum with reference to a mass spectrum or mass spectrometric analyses, refers to a characteristic distribution and number of signals (such as peaks or digital representations thereof). In general, a cleavage pattern as used herein refers to a set of cleavage products that are generated by specific cleavage of a biomolecule such as, but not limited to, nucleic acids and proteins.

As used herein, signal, mass signal or output signal in the context of a mass spectrum or any other method that measures mass and analysis thereof refers to the output data, which is the number or relative number of molecules having a particular mass. Signals include "peaks" and digital representations thereof.

As used herein, the term "peaks" refers to prominent upward projections from a baseline signal of a mass spectrometer spectrum ("mass spectrum") which corresponds to the mass and intensity of a cleavage product. Peaks can be extracted from a mass spectrum by a manual or automated "peak finding" procedure.

As used herein, the mass of a peak in a mass spectrum refers to the mass computed by the "peak finding" procedure.

As used herein, the intensity of a peak in a mass spectrum refers to the intensity computed by the "peak finding" procedure that is dependent on parameters including, but not limited to, the height of the peak in the mass spectrum and its signal-to-noise ratio.

As used herein, "analysis" refers to the determination of certain properties of a single oligonucleotide or polypeptide, or of mixtures of oligonucleotides or polypeptides. These properties include, but are not limited to, the nucleotide or amino acid composition and complete sequence, the existence of single nucleotide polymorphisms and other mutations or sequence variations between more than one oligonucleotide or polypeptide, the masses and the lengths of oligonucleotides or polypeptides and the presence of a molecule or sequence within a molecule in a sample or any modifications on the molecule.

As used herein, "multiplexing" refers to the simultaneous determination of more than one oligonucleotide or polypeptide molecule, or the simultaneous analysis of more than one oligonucleotide or oligopeptide, in a single mass spectrometric or other mass measurement, i.e., a single mass spectrum or other method of reading sequence. Multiplexing sometimes is the simultaneous detection of cleavage products from multiple cleavage reactions with (a) the same cleavage agent applied to different products, or (b) different cleavage agents applied to the same product (e.g., genomic region) or combinations thereof. Multiplexing can also mean analyzing multiple genomic or proteomic regions in a combination of one versus multiple reactions. Multiplexing or better pooling can also mean analyzing a pool of samples in the same reaction(s).

As used herein, amplifying refers to means for increasing the amount of a biopolymer, especially nucleic acids. Based on the 5' and 3' primers that are chosen, amplification also serves to restrict and define the region of the genome which is subject to analysis. Amplification can be by any means known to those skilled in the art, including use of the polymerase chain reaction (PCR), etc. Amplification, e.g., PCR, may be performed quantitatively when, for example, the frequency of polymorphism is to be determined.

As used herein, "polymorphism" refers to the coexistence of more than one form of a gene or portion thereof. A portion of a gene of which there are at least two different forms, i.e., two different nucleotide sequences, is referred to as a "polymorphic region of a gene". A polymorphic region can be a single nucleotide, the identity of which differs in different alleles. A polymorphic region can also be several nucleotides in length. Thus, a polymorphism, e.g. genetic variation, refers to a variation in the sequence of a gene in the genome amongst a population, such as allelic variations and other variations that arise or are observed. Thus, a polymorphism refers to the occurrence of two or more genetically determined alternative sequences or alleles in a population. These differences can occur in coding and non-coding portions of the genome, and can be manifested or detected as differences in nucleic acid sequences, gene expression, including, for example transcription, processing, translation, transport, protein processing, trafficking, DNA synthesis, expressed proteins, other gene products or products of biochemical pathways or in post-translational modifications and any other differences manifested amongst members of a population. A single nucleotide polymorphism (SNP) refers to a polymorphism that arises as the result of a single base change, such as an insertion, deletion or change (substitution) in a base.

A polymorphic marker or site is the locus at which divergence occurs. Such site can be as small as one base pair (an SNP). Polymorphic markers include, but are not limited to, restriction fragment length polymorphisms, variable number of tandem repeats (VNTR's), hypervariable regions, minisatellites, dinucleotide repeats, trinucleotide repeats, tetranucleotide repeats and other repeating patterns, simple sequence repeats and insertional elements, such as Alu. Polymorphic forms also are manifested as different Mendelian alleles for a gene. Polymorphisms can be observed by differences in proteins, protein modifications, RNA expression modification, DNA and RNA methylation, regulatory factors that alter gene expression and DNA replication, and any other manifestation of alterations in genomic nucleic acid or organelle nucleic acids.

As used herein, "polymorphic gene" refers to a gene having at least one polymorphic region.

As used herein, "allele", which is used interchangeably herein with "allelic variant," refers to alternative forms of a genomic region, for example a gene or portion(s) thereof. Alleles occupy the same locus or position on homologous chromosomes. When a subject has two identical alleles of a gene or only one allele, the subject is said to be homozygous for the gene or allele. When a subject has at least two different alleles of a gene, the subject is said to be heterozygous for the gene. Alleles of a specific gene can differ from each other in a single nucleotide, or several nucleotides, and can include substitutions, deletions, and insertions of nucleotides. An allele of a gene can also be a form of a gene containing a mutation.

As used herein, "predominant allele" refers to an allele that is represented in the greatest frequency for a given population. The allele or alleles that are present in lesser frequency are referred to as allelic variants.

As used herein, changes in a nucleic acid sequence known as mutations can result in proteins with altered or in some cases even lost biochemical activities; this in turn can cause genetic disease. Mutations include nucleotide deletions, insertions or alterations/substitutions (i.e. point mutations). Point mutations can be either "missense", resulting in a change in the amino acid sequence of a protein or "nonsense" coding for a stop codon and thereby leading to a truncated protein.

As used herein, a sequence variation contains one or more nucleotides or amino acids that are different in a target nucleic acid or protein sequence when compared to a reference nucleic acid or protein sequence. The sequence variation can include, but is not limited to, a mutation, a polymorphism, or sequence differences between a target sequence and a reference sequence that belong to different organisms. A sequence variation will in general, although not always, contain a subset of the complete set of nucleotide, amino acid, or other biopolymer monomeric unit differences between the target sequence and the reference sequence.

As used herein, additional or missing peaks or signals are peaks or signals corresponding to fragments of a target sequence that are either present or absent, respectively, relative to fragments obtained by actual or simulated cleavage of a reference sequence or reference sample, under the same cleavage reaction conditions. Besides missing or additional signals, differences between target fragments and reference fragments can be manifested as other differences including, but not limited to, differences in peak intensities (height, area, signal-to-noise or combinations thereof) of the signals.

As used herein, different cleavage products are cleavage products of a target sequence that are different relative to cleavage products obtained by actual or simulated cleavage of a reference sequence or sample, under the same cleavage reaction conditions. Different cleavage products can be cleavage products that are missing in the target fragment pattern relative to a reference cleavage pattern, or are additionally present in the target fragmentation pattern relative to the reference fragmentation pattern. Besides missing or additional signals, different signals can also be differences between the target cleavage pattern and the reference cleavage pattern that are qualitative and quantitative including, but not limited to, differences that lead to differences in peak intensities (height, area, signal-to-noise or combinations thereof) of the signals corresponding to the different fragments.

As used herein, the term "compomer" refers to the composition of a sequence cleavage product in terms of its monomeric component units. For nucleic acids, compomer refers to the base composition of the cleavage product with the monomeric units being bases; the number of each type of base can be denoted by $B.sub.n$ (i.e.: $A.sub.aC.sub.cG.sub.gT.sub.t$, with $A.sub.0C.sub.0G.sub.0T.sub.0$ representing an "empty" compomer or a compomer containing no bases). A natural compomer is a compomer for which all component monomeric units (e.g., bases for nucleic acids and amino acids for proteins) are greater than or equal to zero. For purposes of comparing sequences to determine sequence variations, however, in the methods provided herein, "unnatural" compomers containing negative numbers of monomeric units may be generated by an algorithm (e.g., WO 2004/050839, D. van den Boom et al.). For polypeptides, a compomer refers to the amino acid composition of a polypeptide fragment, with the number of each type of amino acid similarly denoted. A compomer corresponds to a sequence if the number and type of bases in the sequence can be added to obtain the composition of the compomer. For example, the compomer $A.sub.2G.sub.3$ corresponds to the sequence AGGAG. In general, there is a unique compomer corresponding to a sequence, but more than one sequence can correspond to the same compomer. For example, the sequences AGGAG, AAGGG, GGAGA, etc. all correspond to the same compomer $A.sub.2G.sub.3$, but for each of these sequences, the corresponding compomer is unique, i.e., $A.sub.2G.sub.3$.

As used herein, witness compomers or compomer witnesses refer to all possible compomers whose masses differ by a value that is less than or equal to a sufficiently small mass difference from the actual mass of each different fragment generated in the target cleavage reaction relative to the same reference cleavage reaction. A sufficiently small mass difference can be determined empirically, if needed, and is generally the resolution of the mass measurement. For example, for mass spectrometry measurements, the value of the sufficiently small mass difference is a function of parameters including, but not limited to, the mass of the different fragment (as measured by its signal) corresponding to a witness compomer, peak separation between fragments whose masses differ by a single nucleotide in type or length, and the absolute resolution of the mass spectrometer. Cleavage reactions specific for one or more of the four nucleic acid bases (A, G, C, T or U for RNA, or modifications thereof) or of the twenty amino acids or modifications thereof, can be used to generate data sets containing the possible witness compomers for each different fragment such that the masses of the possible witness compomers near or equal the actual measured mass of each different fragment by a value that is less than or equal to a sufficiently small mass difference.

As used herein, two or more sequence variations of a target sequence relative to a reference sequence are said to interact with each other if the differences between the cleavage pattern of the target sequence and the reference sequence for a specific cleavage reaction are not a simple sum of the differences representing each sequence variation in the target sequence. For sequence variations in the target sequence that do not interact with each other, the separation (distance) between sequence variations along the target sequence is sufficient for each sequence variation to generate a distinct different product (of the target sequence relative to the reference sequence) in a specific cleavage reaction, the differences in the cleavage pattern of the target sequence relative to the reference sequence represents the sum of all sequence variations in the target sequence relative to the reference sequence.

As used herein, a sufficiently small mass difference is the maximum mass difference between the measured mass of an identified different fragment and the mass of a compomer such that the compomer can be considered as a witness compomer for the identified different fragment. A sufficiently small mass difference can be determined empirically, if needed, and is generally the resolution of the mass measurement. For example, for mass spectrometry measurements, the value of the sufficiently small mass difference is a function of parameters including, but not limited to, the mass of the different fragment (as measured by its signal) corresponding to a witness compomer, the peak separation between fragments whose masses differ by a single nucleotide in type or length, and the absolute resolution of the mass spectrometer.

As used herein, a substring or subsequence $s[i,j]$ denotes a cleavage product of the string s, which denotes the full length nucleic acid or protein sequence. As used herein, i and j are integers that denote the start and end positions of the substring. For example, for a nucleic acid substring, i and j can denote the base positions in the nucleic acid sequence where the substring begins and ends, respectively. As used herein, $c[i,j]$ refers to a compomer corresponding to $s[i,j]$.

As used herein, sequence variation order k refers to the sequence variation candidates of the target sequence constructed by the techniques provided herein, where the sequence variation candidates contain at most k mutations, polymorphisms, or other sequence variations, including, but not limited to, sequence variations between organisms, insertions, deletions and substitutions, in the target sequence relative to a reference sequence. The value of k is dependent on a number of parameters including, but not limited to, the expected type and number of sequence variations between a reference sequence and the target sequence, e.g., whether the sequence variation is a single base or multiple bases, whether sequence variations are present at one location or at more than one location on the target sequence relative to the reference sequence, or whether the sequence variations interact or do not interact with each in the target sequence. For example, for the detection of SNPs, the value of k is usually, although not necessarily, 1 or 2. As another example, for the detection of mutations and in resequencing, the value of k is usually, although not necessarily, 3 or higher.

As used herein, given a specific cleavage reaction of a base, amino acid, or other feature X recognized by the cleavage reagent in a string s, then the boundary $b[i,j]$ of the substring $s[i,j]$ or the corresponding compomer $c[i,j]$ refers to a set of markers indicating whether cleavage of string s does not take place immediately outside the substring s[i,j]. Possible markers are L, indicating whether "s is not cleaved directly before i", and R, indicating whether "s is not cleaved directly after j". Thus, b[i,j] is a subset of the set {L,R} that contains L if and only if X is present at position i-1 of the string s, and contains R if and only if X is present at position j+1 of the string s. #b denotes the number of elements in the set b, which can be 0, 1, or 2, depending on whether the substring s[i,j] is specifically cleaved at both immediately flanking positions (i.e., at positions i-1 and j+1), at one immediately flanking position (i.e., at either position i-1 or j+1) or at no immediately flanking position (i.e., at neither position i-1 nor j+1).

As used herein, a compomer boundary or boundary b is a subset of the set {L,R} as defined above for b[i,j]. Possible values for b are the empty set {}, i.e., the number of elements in b (#b) is 0; {L}, {R}, i.e., #b is 1; and {L,R}, i.e., #b is 2.

As used herein, bounded compomers refers to the set of all compomers c that correspond to the set of subsequences of a reference sequence, with a boundary that indicates whether or not cleavage sites are present at the two ends of each subsequence. The set of bounded compomers can be compared against possible compomer witnesses to construct all possible sequence variations of a target sequence relative to a reference sequence. For example, (c,b) refers to a 'bounded compomer' that contains a compomer c and a boundary b.

As used herein, C refers to the set of all bounded compomers within the string s; i.e., for all possible substrings s[i,j], find the bounded compomers (c[i,j],b[i,j]) and these will belong to the set C. C can be represented as C:={(c[i,j],b[i,j]): 1.ltoreq.i.ltoreq.j.ltoreq.length of s}

As used herein, ord[i,j] refers to the number of times substring s[i,j] will be cleaved in a particular cleavage reaction.

As used herein, given compomers c,c' corresponding to fragments f,f, d(c,c') is a function that determines the minimum number of sequence variations, polymorphisms or mutations (insertions, deletions, substitutions) that are needed to convert c to c', taken over all potential cleavage products f,f corresponding to compomers c,c', where c is a compomer of a cleavage product s of the reference biomolecule and c' is the compomer of a cleavage product s' of the target biomolecule resulting from a sequence variation of the s cleavage. As used herein, d(c,c') is equivalent to d(c',c).

For a bounded compomer (c,b) constructed from the set C, The function D(c',c,b) measures the minimum number of sequence variations relative to a reference sequence that is needed to generate the compomer witness c'. D(c',c,b) can be represented as D(c',c,b):=d(c',c)+#b. As used herein, D(c',c,b) is equivalent to D(c,c',b)

As used herein, C.sub.k is a subset of C such that compomers for substrings containing more than k number of sequence variations of the cut string will be excluded from the set C. Thus, if there is a sequence variation containing at most k insertions, deletions, and substitutions, and if c' is a compomer corresponding to a peak witness of this sequence variation, then there exists a bounded compomer (c,b) in C.sub.k such that D(c',c,b).ltoreq.k. C.sub.k can be represented as C.sub.k:={(c[i,j], b[i,j]):1.ltoreq.i.ltoreq.j.ltoreq.length of s, and ord[i,j]+#b[i,j].ltoreq.k}. The algorithm provided herein is based on this reduced set of compomers corresponding to possible sequence variations.

As used herein, L.sub..DELTA. or L_.DELTA. denotes a list of peaks or signals corresponding to cleavage products that are different in a target cleavage reaction relative to the same reference cleavage reaction. The differences include, but are not limited to, signals that are present or absent in the target cleavage signals relative to the reference cleavage signals, and signals that differ in intensity between the target cleavage signals and the reference cleavage signals.

As used herein, sequence variation candidate refers to a potential sequence of the target sequence containing one or more sequence variations. The probability of a sequence variation candidate being the actual sequence of the target biomolecule containing one or more sequence variations is measured by a score.

As used herein, a reduced set of sequence variation candidates refers to a subset of all possible sequence variations in the target sequence that would generate a given set of signals upon specific cleavage of the target sequence. A reduced set of sequence variation candidates can be obtained by creating, from the set of all possible sequence variations of a target sequence that can generate a particular cleavage pattern (as detected by measuring the masses of the cleavage products) in a particular specific cleavage reaction, a subset containing only those sequence variations that generate cleavage products of the target sequence that are different from the cleavage products generated by actual or simulated cleavage of a reference sequence in the same specific cleavage reaction.

As used herein, cleavage products that are consistent with a particular sequence variation in a target molecule refer to those different cleavage products that are obtained by cleavage of a target molecule in more than one reaction using more than one cleavage reagent whose characteristics, including, but not limited to, mass, intensity or signal-to-noise ratio, when analyzed according to the methods provided herein, indicate the presence of the same sequence variation in the target molecule.

As used herein, scoring or a score refers to a calculation of the probability that a particular sequence variation candidate is actually present in the target nucleic acid or protein sequence. The value of a score is used to determine the sequence variation candidate that corresponds to the actual target sequence. Usually, in a set of samples of target sequences, the highest score represents the most likely sequence variation in the target molecule, but other rules for selection can also be used, such as detecting a positive score, when a single target sequence is present.

As used herein, simulation (or simulating) refers to the calculation of a cleavage pattern based on the sequence of a nucleic acid or protein and the predicted cleavage sites in the nucleic acid or protein sequence for a particular specific cleavage reagent. Simulated cleaving also is referred to herein as "virtual" cleaving of a biomolecule sequence. The cleavage pattern can be simulated as a table or array of numbers (for example, as a list of peaks corresponding to the mass signals of cleavage products of a reference biomolecule), as a mass spectrum, as a pattern of bands on a gel, or as a representation of any technique that measures mass distribution. Simulations can be performed in most instances by a computer program.

As used herein, simulating cleavage refers to an *in silico* process in which a target molecule or a reference molecule is virtually cleaved. As used herein, *in silico* refers to research and experiments performed using a computer. *In silico* methods include, but are not limited to, molecular modeling studies, biomolecular docking experiments, and virtual representations of molecular structures and/or processes, such as molecular interactions.

As used herein, a subject includes, but is not limited to, animals (e.g., humans), plants, bacteria, viruses, fungi, parasites and any other organism or entity that has nucleic acid. Among subjects are mammals, preferably, although not necessarily, humans. A patient refers to a subject afflicted with a disease or disorder.

As used herein, a phenotype refers to a set of parameters that includes any distinguishable trait of an organism. A phenotype can be physical traits and can be, in instances in which the subject is an animal, a mental trait, such as emotional traits.

As used herein, "assignment" refers to a determination that the position of a nucleic acid or protein fragment indicates a particular molecular weight and a particular terminal nucleotide or amino acid.

As used herein, "a" refers to one or more.

As used herein, "plurality" refers to two or more polynucleotides or polypeptides, each of which has a different sequence. Such a difference can be due to a naturally occurring variation among the sequences, for example, to an allelic variation in a nucleotide or an encoded amino acid, or can be due to the introduction of particular modifications into various sequences, for example, the differential incorporation of mass modified nucleotides into each nucleic acid or protein in a plurality.

As used herein, an array refers to a pattern produced by three or more items, such as three or more loci on a solid support. An array also may be utilized in vectors and matrices, where a vector is a one dimensional array and a matrix is a two-dimensional array. Processes described herein may manipulate arrays in one or more dimensions.

As used herein, "unambiguous" refers to the unique assignment of peaks or signals corresponding to a particular sequence variation, such as a mutation, in a target molecule and, in the event that a number of molecules or mutations are multiplexed, that the peaks representing a particular sequence variation can be uniquely assigned to each mutation or each molecule. The term "unambiguous" also can refer to the correct matching of a sample pattern to a reference pattern.

As used herein, a data processing routine refers to a process, that can be embodied in software, that determines the biological significance of acquired data (i.e., the ultimate results of the assay). For example, the data processing routine can make a genotype determination based upon the data collected. In the systems and methods herein, the data processing routine also controls the instrument and/or the data collection routine based upon the results determined. The data processing routine and the data collection routines are integrated and provide feedback to operate the data acquisition by the instrument, and hence provide the assay-based judging methods provided herein.

As used herein, a plurality of genes includes at least two, five, 10, 25, 50, 100, 250, 500, 1000, 2,500, 5,000, 10,000, 100,000, 1,000,000 or more genes. A plurality of genes can include complete or partial genomes of an organism or even a plurality thereof. Selecting the organism type determines the genome from among which the gene regulatory regions are selected. Exemplary organisms for gene screening include animals, such as mammals, including human and rodent, such as mouse, insects, yeast, bacteria, viruses, parasites, fungi and plants.

As used herein, "specifically hybridizes" refers to hybridization of a probe or primer only to a target sequence preferentially to a non-target sequence. Those of skill in the art are familiar with parameters that affect hybridization; such as temperature, probe or primer length and composition, buffer composition and salt concentration and can readily adjust these parameters to achieve specific hybridization of a nucleic acid to a target sequence.

As used herein, "sample" refers to a composition containing a material to be detected. A sample may be collected from an organism, mineral or geological site (e.g., soil, rock, mineral deposit, fossil), or forensic site (e.g., crime scene, contraband or suspected contraband), for example. In a preferred embodiment, the sample is a "biological sample." The term "biological sample" refers to any material obtained from a living source or formerly-living source, for example, an animal such as a human or other mammal, a plant, a bacterium, a fungus, a protist or a virus. The biological sample can be in any form, including a solid material such as a tissue, cells, a cell pellet, a cell extract, or a biopsy, or a biological fluid such as urine, blood, saliva, amniotic fluid, exudate from a region of infection or inflammation, or a mouth wash containing buccal cells, urine, cerebral spinal fluid and synovial fluid and organs. Preferably solid materials are mixed with a fluid. In certain embodiments, herein, an analyte from a sample can refer to a mixture of matrix used for mass spectrometric analyses and biological material such as nucleic acids. Derived from means that the sample can be processed, such as by purification or isolation and/or amplification of nucleic acid molecules. As used herein, "of a sample" refers to a biomolecule sequence or sequence pattern determined or identified in a sample or outside a sample. For example, a biomolecule can be isolated from a sample, then fragmented, and the fragments then analyzed to determine the presence or absence of a particular sequence or sequence pattern outside the sample.

As used herein, a composition refers to any mixture. It can be a solution, a suspension, liquid, powder, a paste, aqueous, non-aqueous or any combination thereof.

As used herein, a combination refers to any association between two or among more items.

As used herein, the term "11/4-cutter" refers to a restriction enzyme that recognizes and cleaves a 2 base stretch in the nucleic acid, in which the identity of one base position is fixed and the identity of the other base position is any three of the four naturally occurring bases.

As used herein, the term "11/2-cutter" refers to a restriction enzyme that recognizes and cleaves a 2 base stretch in the nucleic acid, in which the identity of one base position is fixed and the identity of the other base position is any two out of the four naturally occurring bases.

As used herein, the term "2 cutter" refers to a restriction enzyme that recognizes and cleaves a specific nucleic acid site that is 2 bases long.

As used herein, the term "AFLP" refers to amplified fragment length polymorphism, and the term "RFLP" refers to restriction fragment length polymorphism.

As used herein, the term "amplicon" refers to a region of nucleic acids (DNA or RNA) that can be replicated.

As used herein, the term "complete cleavage" or "total cleavage" refers to a cleavage reaction in which all the cleavage sites recognized by a particular cleavage reagent are cut to completion.

As used herein, the term "false positives" refers to mass signals that are from background noise and not generated by specific actual or simulated cleavage of a nucleic acid or protein.

As used herein, the term "false negatives" refers to actual mass signals that are missing from an actual fragmentation/cleavage spectrum but can be detected in the corresponding simulated spectrum.

As used herein, the term "partial cleavage" refers to a reaction in which only a fraction of the cleavage sites of a particular cleavage reagent are actually cut by the cleavage reagent. Cleavage products described herein can result from a partial cleavage.

As used herein, cleave means any manner in which one or multiplenucleic acid or protein molecule(s) are cut into smaller pieces. The cleavage recognition sites can be one, two or more bases long. The cleavage means include physical cleavage, enzymatic cleavage, chemical cleavage and any other way smaller pieces of a nucleic acid are produced.

As used herein, cleavage conditions or cleavage reaction conditions refers to the set of one or more cleavage reagents that are used to perform actual or simulated cleavage reactions, and other parameters of the reactions including, but not limited to, time, temperature, pH, or choice of buffer.

As used herein, uncleaved cleavage sites means cleavage sites that are known recognition sites for a cleavage reagent but that are not cut by the cleavage reagent under the conditions of the reaction, e.g., time, temperature, or modifications of the bases at the cleavage recognition sites to prevent cleavage by the reagent.

As used herein, complementary cleavage reactions refers to cleavage reactions that are carried out or simulated on the same target or reference nucleic acid or protein using different cleavage reagents or by altering the cleavage specificity of the same cleavage reagent such that alternate cleavage patterns of the same target or reference nucleic acid or protein are generated.

As used herein, a combination refers to any association between two or among more items or elements.

As used herein, a composition refers to a any mixture. It can be a solution, a suspension, liquid, powder, a paste, aqueous, non-aqueous or any combination thereof.

As used herein, fluid refers to any composition that can flow. Fluids thus encompass compositions that are in the form of semi-solids, pastes, solutions, aqueous mixtures, gels, lotions, creams and other such compositions.

As used herein, a cellular extract refers to a preparation or fraction which is made from a lysed or disrupted cell.

As used herein, a kit is combination in which components are packaged optionally with instructions for use and/or reagents and apparatus for use with the combination.

As used herein, a system refers to the combination of elements with software and any other elements for controlling and directing methods provided herein.

As used herein, software refers to computer readable program instructions that, when executed by a computer, performs computer operations. Typically, software is provided on a program product containing program instructions recorded on a computer readable medium, such as but not limited to, magnetic media including floppy disks, hard disks, and magnetic tape; and optical media including CD-ROM discs, DVD discs, magneto-optical discs, and other such media on which the program instructions can be recorded.

As used herein, a "mixture" refers to a mixture of samples, a mixture of sample sequences and/or sequence signals from one or more samples, a mixture of reference sequences and/or reference sequence signals from one or more reference sequences, or a mixture of sequences and/or sequence signals from one or more samples and one or more reference sequences, for example.

As used herein a "sequence signal" refers to any detectable signal generated from a sequence (e.g., amino acid sequence or nucleic acid sequence). A sequence signal may be a signal generated from nucleic acid or polypeptide fragment, and can be identified by a mass spectrometry process or electrophoretic process, for example. A sequence signal can be identified by a detectable indicator in certain embodiments, such as an indicator tag linked to a biomolecule or fragment thereof (e.g., fluorescent tag), for example. A sequence signal identified by a mass spectrometric process includes, but is not limited to, a mass signal, a mass to charge signal, and an intensity signal (e.g., peak intensity signal), for example.

Comparative Sequence Analysis Process Embodiments

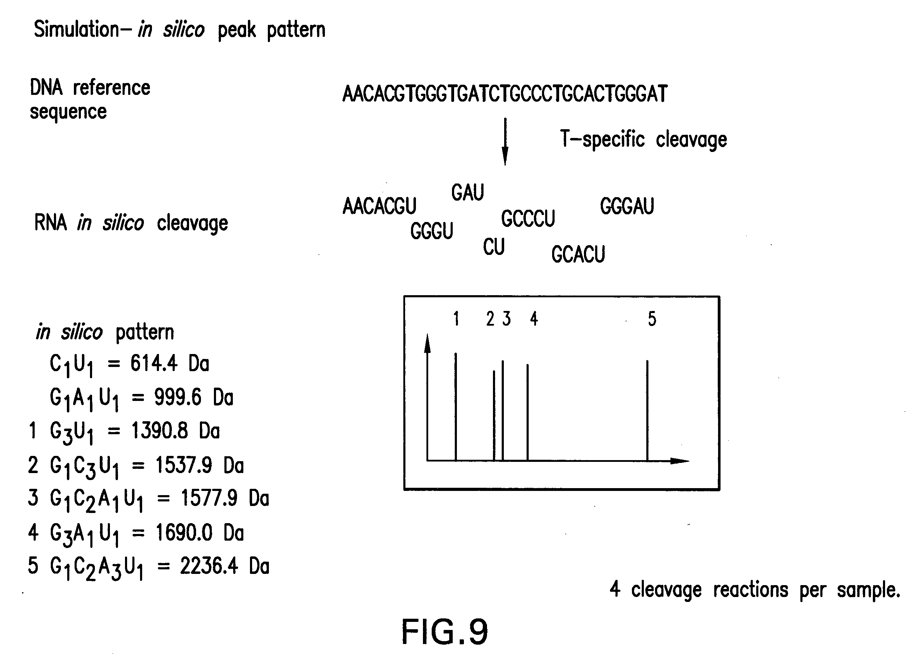

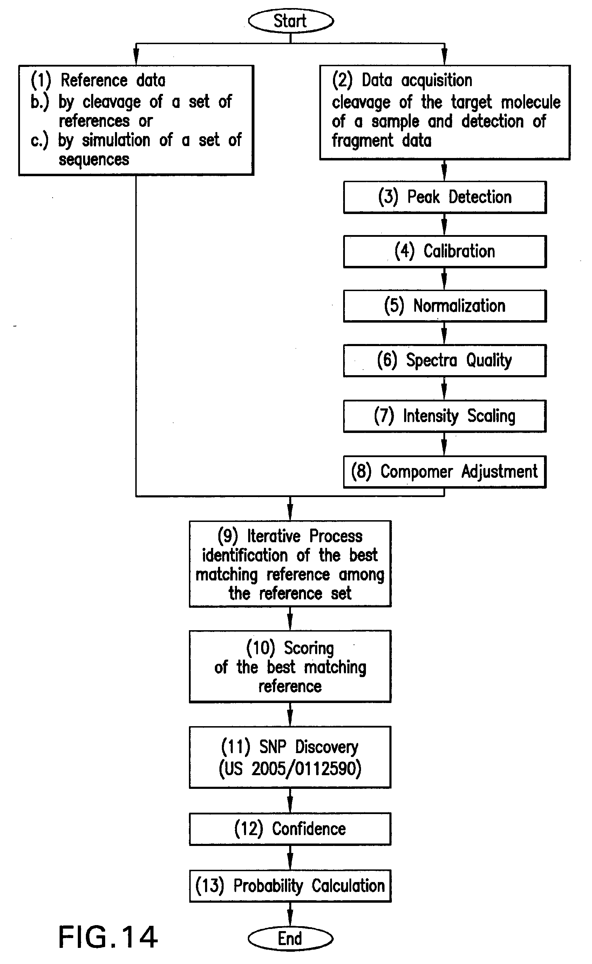

In some comparative sequence analysis embodiments, sequence or pattern information derived from sample signal patterns and reference signal patterns is compared. Reference data can include signal patterns prepared from specifically cleaved fragmented nucleic acid samples or signal patterns prepared by simulated cleavage of nucleic acid sequences *in silico,* as illustrated, for example, in Figure 14, parts (1 b) and (1c). Reference data may be from any suitable source, such as signals derived from simulated cleavage *in silico* (i.e. virtual cleavage) of one or more nucleic acid sequences or mixtures thereof from a sequence database, for example (e.g., Figure 9). Reference data also may comprise signals derived from one or more specifically cleaved and analyzed sample nucleic acids or mixtures thereof. Or a consensus sequence derived from multiple samples.

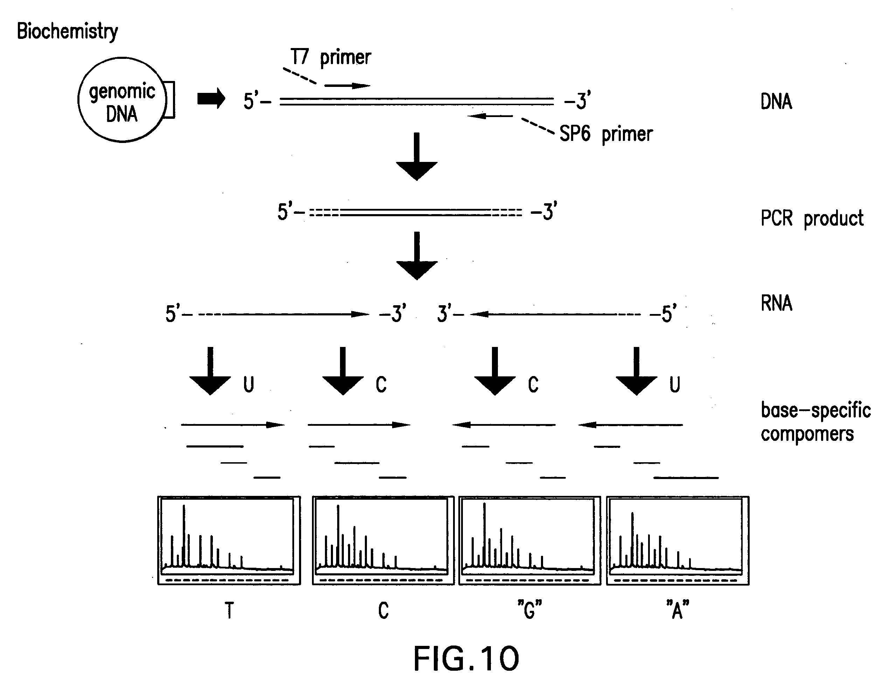

In certain reference sequence comparison embodiments, sequence lengths generally are not restricted in terms of minimal and maximal length; and lengths can range between 200->800 bp for nucleic acid sequences. Target sequences sometimes are flanked by conserved sequence stretches, which determine primer regions for target amplifications. Mismatches (such as degenerate primers) in conserved regions can be allowed. Start and end tags on the 5'- or 3'- end of primers often are tagged with nucleotide sequence stretches, which facilitate *in vitro* transcription. Examples of sequence primers are as follows (e.g., Figure 10):

*T7 primer*
Transcription promoter                 8 bp tag
5'-cagtaatacgactcactataggg agaaggct - gene specific primer part
*SP6 primer*
Transcription promoter                 8 bp tag
5'-cgatttaggtgacactatagaa gagaggct - gene specific primer part.

Base-specific cleavage patterns of nucleic acid sequences (including tag sequences after transcription) can be simulated *in silico.* Each sequence can be represented by four or more possible peak lists. Four peak lists may correspond to a T-specific cleavage of the forward RNA transcript as well as the reverse transcript and the C-specific cleavage of the forward RNA as well as the reverse transcript, but are not restricted to such.

The distance of simulated reference data or acquired data can be obtained in certain embodiments. Clustering processes are known and can be readily selected by the person of ordinary skill in the art. Base-specific cleavage patterns of related nucleic acid sequences sets sometimes are clustered using discriminating features. Discriminating features can be, but are not restricted to, peak masses and intensities or sequence lengths. To distinguish two sequences, discriminating features present in one but not present in the other can be used. For more then two sequences an approach can be to divide simulated peak mass pattern into clusters based on discriminating features, which are unique to each cluster. These clusters can be distinguished from one another, and in an iterative process clusters can be again divided into sub-clusters until individual peak lists are resolved. At each clustering level, there can be multiple solutions. A solution with an optimal amount of discriminating features, while containing the most number of clusters generally is selected. Any clustering method known and selected by the person of ordinary skill in the art can be utilized, including but not limited to clustering methods like neighbor joining, UPGMA, maximum likelihood and any clustering in data mining.

Reference signal sets derived from different sources may be mixed and then compared to sample signal sets. For example, reference data from database sequences or sample sequences of viral strains can be cleaved *in silico* or *in vitro,* respectively, the cleavage products can be detected and the resulting detection signals can be processed. Processing of the signals optionally can include clustering techniques, using techniques known to and selected by the person of ordinary skill in the art.

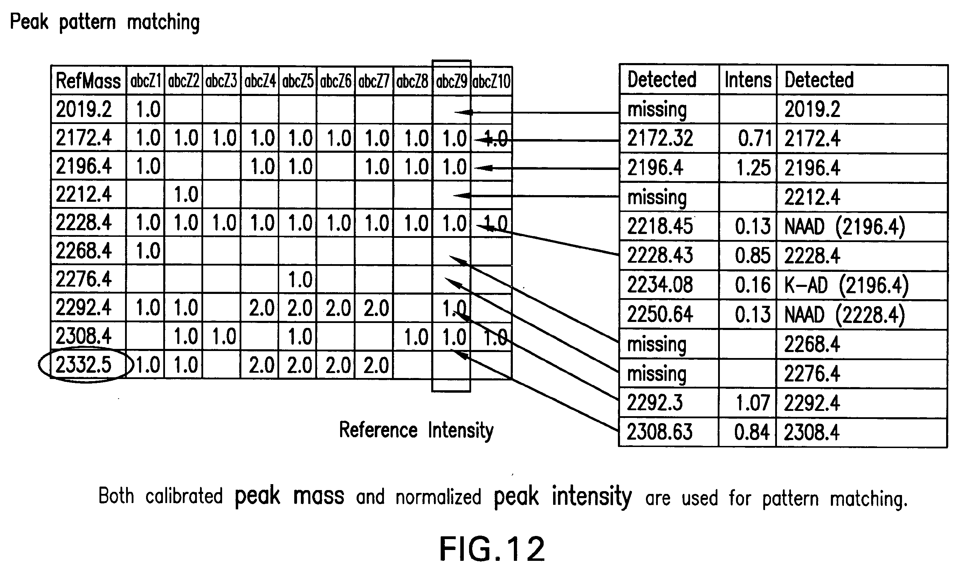

A target molecule may be specifically cleaved and cleavage products can be detected by detection processes. The person of ordinary skill in the art can select appropriate selection process, which include, but are not limited to, gel electrophoresis, capillary electrophoresis and mass spectrometry, for example (e.g., MALDI-TOF mass spectrometry). Signal data from the detection process can be processed using one or more signal processing techniques known to and selected by the person of ordinary skill in the art (e.g., Figure 9). Signal processing techniques, include, but are not limited to, peak detection, calibration, normalization, spectra quality, intensity scaling, compomer adjustment, identification of adduct signals and the like. Figure 12 shows a particular embodiment for analyzing sample sequence signal patterns.

In certain peak detection processes, spectra are filtered by Gaussian filters with moving width (adjusts with mass). Peaks can be identified by local maximum in the filtered spectrum. Peaks meeting a minimum width and signal to noise ratio generally are selected. Noise levels can be approximated from silent windows, where no analysis product related signals are expected.

Intensity scaling processes can be applied as spectra obtained by mass spectrometry provide signal patterns with a technology related intensity distribution. In certain intensity scaling processes, raw peak intensities can be scaled to correct this mass dependent variation. Scaling factors can be obtained by fitting peak intensities to standard profiles in one detection range or multiple detection ranges. The profiles can be connected into one profile covering the whole range of detection. Scaling factors at any particular data point (e.g. mass) can be interpolated (e.g. linearly) from the final profile and revised intensities for all detected signals can be calculated to generate revised intensities. This process sometimes is referred to as "mass dependent peak scaling." In an example involving MALDI-TOF mass spectrometry, peaks in a range of 1100-2500 Da can be fitted to parabolic curve with a positive and second order coefficient and a fixed minimum at 1100 Da. Peaks in the mass range of 2000-4000 Da can be fitted to a parabolic curve with negative second order coefficient. Peaks above 4500 Da can be fitted to an exponential decay.

Compomer adjustment processes can be applied to signals. In addition to the composition of the cleavage product mixture, intensities of signals are a function of the nucleic acid base composition of individual analyte fragments, which Influence their flight behavior in the mass spectrometer and thus their resulting intensity (e.g. T-rich fragments). An empirical relationship between cleavage product composition (%A, %T, %C, %G) and resulting signal intensity can be used to scale peak intensities after mass dependent peak scaling, thereby yielding adjusted peak intensities. Signals based on adducts (e.g. salt, matrix, doubly charged, degenerate primer signals, abortive cycling products etc.) as results of the applied biochemistry in combination with the applied data acquisition tool not referring to the simulated features of the reference set can be identified and explained using such processes.

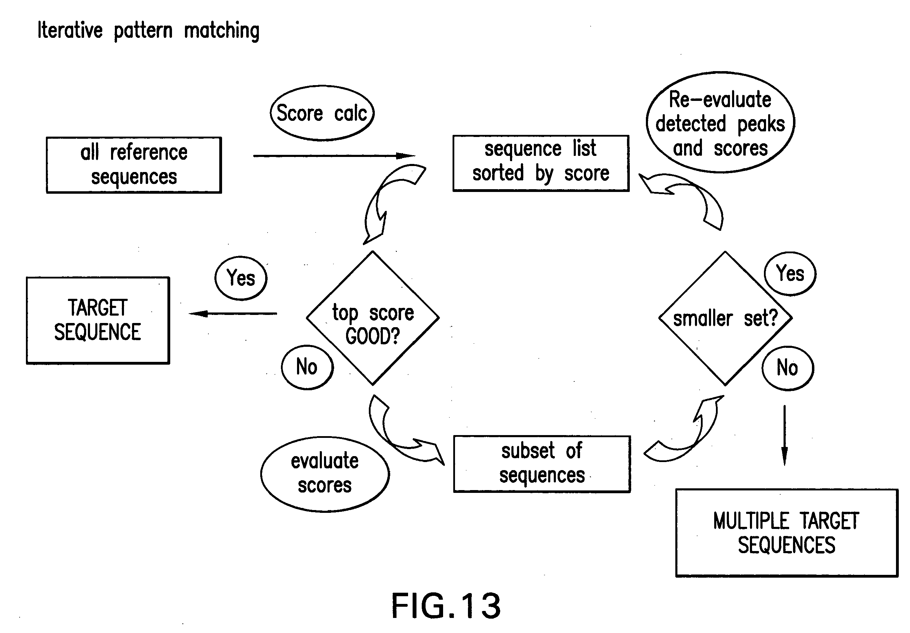

Reference and sample signal patterns can be compared to one another to identify the presence or absence of common sequences (e.g., Figure 14), often after signals are processed. Signal pattern matching may be scored in an iterative process to identify the best-matched signal or signals between sample and reference data sets, as shown, for example, in Figures 13 and 14. The term "iterative" as used herein refers to repeating a process, such as a matching and scoring process, in two or more cycles, such as about 2, 3, 4, 5, 6, 7, 8, 9, 10, 15, 20, 30, 40, 50, 60, 70, 80, 90, 100, 200, 300, 400, 500, 600, 700, 800, 900, or 1000 or more cycles. A set of matched signals may be scored and a subset of top-matching signals is selected in a particular cycle, and in a subsequent cycle, signals in the subset selected in the previous cycle are matched and scored and a smaller subset of best-matched signals is selected.

In certain pattern matching processes that include iterative identification, targets can be identified by comparing peak patterns of base-specific cleavage products obtained by mass spectrometry (e.g. MALDI-TOF MS) to one or more *in silico* base-specific cleavage pattern. Targets identification can be accomplished by iteration and combining overall feature pattern matching and discriminating feature matching.

Some scoring processes include different scores: 1. bitmap score, 2. discriminating feature matching score, 3. distance score, 4. PPIdentity, 5. AdjChange score and 6. overall score. A bitmap score can be calculated by comparing detected and individual reference peak patterns. For each matching peak a score can be calculated by comparing the intensities weighted by the reference intensity, which is obtained in simulation (1). The score can be a measure for minor differences between the peak intensities crucial for sequence identification. A discriminating feature matching score can be calculated by evaluating a subset of features that discriminate one feature pattern from another or one set of pattern from another set. A distance score is calculated based on, e.g., Euclidian distance of the identified feature vectors to all reference feature vectors. A PPIdentity is a peak pattern identity score, which can be calculated from the sum of the matched peak intensities, the missing and additional peak intensities and the silent missing and silent additional peak intensities. Silent peaks can be peaks formed by multiple cleavage products with the same characteristics, e.g., mass. Silent peaks can decrease or increase in intensity, whereas additional signals only increase in intensity starting from zero intensity and whereas missing signals decrease in intensity to zero from a detected intensity. The score generally ignores minor differences between peak intensities as caused by experimental variation. An AdjChange score can be calculated as the sum of the adjMissing, adjMismatch and adjExtra score. The adjMissing score can be the sum of missing peak intensities weighted by reactions. The adjMismatch score can be the sum of mismatch peak intensities weighted by reactions. Mismatches are signals expected for the reference set, but not for the particular sample reference. The adjExtra score is the sum of additional peak intensities weighted by the reaction performed. Extra signals are signals not expected for the reference set. An overall score is the combination of the bitmap score and the PPIdentity score (e.g. the average).

During iteration, detected feature patterns often are scaled based on reference features from the entire reference set. Scores can be assigned to all matching events. A set of best matches generally then are selected. Subsequently, detected features can be re-scaled based on the subset, and scores are calculated again to find a yet smaller set of best matches. This process iterates until one reference or several references with close scores are considerably better than the rest. Targets can be compared against not only one but different reference sets, e.g., extended sets, or sequence-based and feature-based sets.

Sequence variations (e.g., mutatons) may be detected in the best-matched signals (e.g., reference signals and/or sample signals) using techniques known to the person of ordinary skill in the art. The sequence variations may be mutations, single-nucleotide deletions, insertions or substitutions (e.g., single-nucleotide polymorphisms), for example, or deletions, insertions or substitutions of two or more consecutive nucleotides (e.g., microsatellites, insertion repeats). For mass spectrometric signals, mass peak location and intensity can be utilized to determine the presence or absence of sequence modifications, as described, for example, in U.S. Patent Application Publication 2005/0112590, published May 26, 2005 (Boom et al.). Such approaches can allow for target discrimination and identification down to a single base difference.

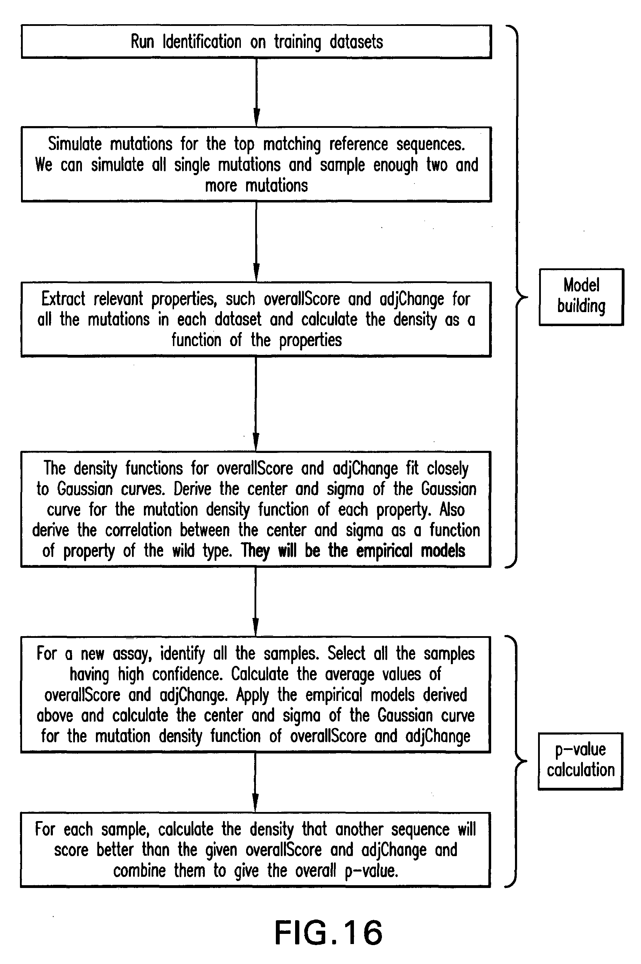

As shown, for example, in Figure 16, a confidence value can be assigned to the match of the top-matched signals. Any applicable confidence assessment processes can be utilized, and can be selected by the person of ordinary skill in the art. A confidence evaluation provides the likelihood that the top scoring sequence is the correct match with no sequence variations occurring, in other words, a probability of having undetected sequence variations. A p-value representative of confidence can be calculated using a Monte Carlo simulation in certain embodiments (J. Samuelsson, "Modular, scriptable and automated analysis tool for high-throughput peptide mass fingerprinting", Bioinformatics, Vol. 20 no. 18, 2004). As an alternative, single nucleotide changes can be simulated in each position of each sequence in a reference set. Matching of the detected peak pattern to all simulated reference sequences and plotting of the resulting scores (adjChange and the overall score) deliver frequency distributions. These distributions can be used to identify the range of scores or corresponding p-values, which result if an alpha-error is defined (e.g., 1% or 5%). Parameters can include, but are not limited to. one or more of the following:

AdjMissing: The sum of missing peak intensity weighted by reactions.

AdjMismatch: The sum of mismatch peak intensity weighted by reactions. Mismatches are signals expected for the reference set, but not for the particular sequence.

AdjExtra: The sum of additional peak intensity weighted by reactions. AdjExtra are signals not expected for the reference set.

AdjChange: The sum of adjMissing, adjMismatch and adjExtra

silMissing: The sum of partial peak intensities, where the detected intensity is substantially lower then the reference intensity, weighted by reaction.

silAddition: The sum of partial peak intensities, where the detected intensity is substantially higher than the reference intensity, weighted by reaction.

totChange: The sum of adjChange, silMissing and silAddition.

Figure 16 shows a process for determining a confidence value. In such processes, the distribution of some scores, such as overallScore and adjChange for a dataset is plotted using simulated mutations. The distributions are close to Gaussian and can be modeled as such. A set of standard parameters can be predetermined and sequence variation (e.g., mutation) probabilities for samples can then be calculated for each score and combined. Standard parameters can include, but are not limited to, one or more of the following:

bitmapScore: a bitmap score can be calculated by comparing detected and reference individual peak patterns (for each matching peak a score is calculated by comparing the intensities and weighted by the reference intensity). This score can measure minor difference between peak intensities which is crucial in sequence identification.

PPIdentity: a peak pattern identity score can be calculated from the sum of the matched peak intensities, the missing and additional peak intensities and the silent missing and silent additional peak intensities. This score ignores minor difference between peak intensities that may be caused by experimental variations.

OverallScore: an overall score is the combination of BitmapScore and PPIdentity score (e.g., average).

adjMissing: this score can be the sum of missing peak intensity weighted by reactions.

adjMismatch: this score can be the sum of mismatch peak intensity weighted by reactions (expected for the reference set, but not for a particular sequence).

adjExtra: this score can be the sum of additional peak intensity weighted by reactions (not expected for the reference set).

adjChange: this score is the sum of adjMissing, adjMismatch and adjExtra.

silMissing: this score is the sum of partial peak intensity where detected intensity is substantially weaker than the reference intensity, weighted by reactions.

silAddition: this score is the sum of partial peak intensity where detected intensity is substantially stronger than the reference intensity, weighted by reactions.

totChange: this score is the sum of adjChange, silMissing and silAddition.

The standard parameters are chosen so that good matches generally have a p-value less than 5% or as defined by the user.

Due to sequence contents and experimental conditions, the standard parameters are not always accurate. One way to compensate the variation is to perform post-identification cluster analysis. Given a reference sequence set, find all the samples having best scores within a certain range (assuming they have low chance of having mutations, otherwise, the SNP discovery algorithm would have detected one). The average scores for these samples will be used to refine the standard parameters for the data set. These refined parameters will be used to calculate confidence for all the samples.

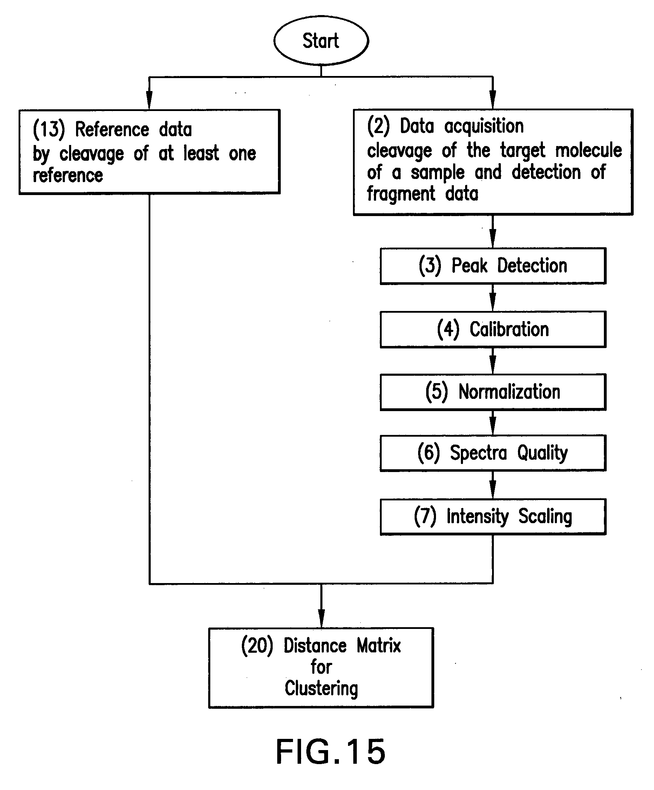

Sample signal data, optionally in combination with reference signal data, can be compared and processed by clustering techniques. Simulated as well as acquired data in array format can be clustered by public clustering algorithms to reflect a relationship of the samples and/or reference sets. In a peak pattern based embodiment, a peak pattern database is built out of data acquired on reference samples. These patterns can be used for target identification as an alternative to *in silico* base-specific cleavage pattern. Peak patterns of one signature region or multiple regions can be concatenated and clustered based on an appropriate distance calculation (e.g. weighted Euclidian distance or any other known distance measure). Detected signals can be manually excluded from identification and prompt reanalysis. Figure 15 shows a representative embodiment of clustering techniques.

Outputs of the comparative sequence analysis processes can be produced by different parameter settings based on the complexity of the reference set or reference sample set. Outputs of comparative sequence analysis processes can include one or more of the following: identification result, sequence variations (e.g., mutations), signal lists, reference sets (extended), failed reactions, sequences identified per sample and overlapping amplicons, distance matrices (cluster) and outputs, which provide input to database queries (e.g. MLST allele profile report) and the like.

Methods for Generating Fragments

*Nucleic Acid Cleavage*

Cleavage of nucleic acids is known in the art and can be achieved in many ways. For example, polynucleotides composed of DNA, RNA, analogs of DNA and RNA or combinations thereof, can be cleaved physically, chemically, or enzymatically, as long as the cleavage is obtained by cleavage at a specific site in the target nucleic acid. Fragmentation generally refers to physical fragmentation of an organic molecule in a mass spectrometer. Molecules can be cleaved at a specific positions in a target nucleic acid sequence based on (i) the base specificity of the cleaving reagent (e.g., A, G, C, T or U, or the recognition of modified bases or nucleotldes); or (ii) the structure of the target nucleic acid; or (iii) a combination of both, are generated from the target nucleic acid. In another embodiment, cleavage occurs at multiple combinations of bases to extract, for example, homopolymer stretches. Cleavage products and fragments can vary in size, and suitable fragments sometimes are less that about 2000 nucleic acids, but can be longer depending upon the selected method. Suitable fragments can fall within several ranges of sizes including but not limited to: less than about 1000 bases, between about 100 to about 500 bases, from about 25 to about 200 bases or about 4 to about 30 bases. In some aspects, cleavage products or fragments of about one nucleic acid (cleavage base) are desirable.

Polynucleotides can be cleaved by chemical reactions including for example, hydrolysis reactions including base and acid hydrolysis. Alkaline conditions can be used to cleave polynucleotides comprising RNA because RNA is unstable under alkaline conditions. See, e.g., Nordhoff et al. (1993) Ion stability of nucleic acids in infrared matrix-assisted laser desorption/ionization mass spectrometry, Nucl. Acids Res., 21(15):3347-57. DNA can be hydrolyzed in the presence of acids, typically strong acids such as 6M HCl. The temperature can be elevated above room temperature to facilitate the hydrolysis. Depending on the conditions and length of reaction time, the polynucleotides can be cleaved into various sizes including single base products. Hydrolysis can, under rigorous conditions, break both of the phosphate ester bonds and also the N-glycosidic bond between the deoxyribose and the purines and pyrimidine bases.

An exemplary acid/base hydrolysis protocol for producing polynucleotide products is described in Sargent et al. (1988) Methods Enzymol., 152:432. Briefly, 1 g of DNA is dissolved in 50 mL 0.1 N NaOH. 1.5 mL concentrated HCl is added, and the solution is mixed quickly. DNA will precipitate immediately, and should not be stirred for more than a few seconds to prevent formation of a large aggregate. The sample is incubated at room temperature for 20 minutes to partially depurinate the DNA. Subsequently, 2 mL 10 N NaOH (OH- concentration to 0.1 N) is added, and the sample is stirred till DNA redissolves completely. The sample is then incubated at 65.degree. C. for 30 minutes to hydrolyze the DNA. Typical sizes range from about 250-1000 nucleotides but can vary lower or higher depending on the conditions of hydrolysis.

Another process whereby nucleic acid molecules are chemically cleaved in a base-specific manner is provided by A. M. Maxam and W. Gilbert, Proc. Natl. Acad. Sci. USA 74:560-64, 1977. Individual reactions were devised to cleave preferentially at guanine, at adenine, at cytosine and thymine, and at cytosine alone.

Polynucleotides can also be cleaved via alkylation, particularly phosphorothioate-modified polynucleotides. K. A. Browne (2002) Metal ion-catalyzed nucleic Acid alkylation and fragmentation. J. Am. Chem. Soc. 124(27):7950-62. Alkylation at the phosphorothioate modification renders the polynucleotide susceptible to cleavage at the modification site. I. G. Gut and S. Beck describe methods of alkylating DNA for detection in mass spectrometry. I. G. Gut and S. Beck (1995) A procedure for selective DNA alkylation and detection by mass spectrometry. Nucleic Acids Res. 23(8):1367-73. Another approach uses the acid lability of P3-N5'-phosphoroamidate-containing DNA (Shchepinov et al., "Matrix-induced fragmentation of P3'-N5'-phosphoroamidate-containing DNA: high-throughput MALDI-TOF analysis of genomic sequence polymorphisms," Nucleic Acids Res. 25: 3864-3872 (2001). Either dCTP or dTTP are replaced by their analog P-N modified nucleoside triphosphates and are introduced into the target sequence by primer extension reaction subsequent to PCR. Subsequent acidic reaction conditions produce base-specific cleavage products. In order to minimize depurination of adenine and guanine residues under the acidic cleavage conditions required, 7-deaza analogs of dA and dG can be used.

Single nucleotide mismatches in DNA heteroduplexes can be cleaved by the use of osmium tetroxide and piperidine, providing an alternative strategy to detect single base substitutions, generically named the "Mismatch Chemical Cleavage" (MCC) (Gogos et al., Nucl. Acids Res., 18: 6807-6817 [1990]).

Polynucleotide fragmentation can also be achieved by irradiating the polynucleotides. Typically, radiation such as gamma or x-ray radiation will be sufficient to fragment the polynucleotides. The size of the fragments can be adjusted by adjusting the intensity and duration of exposure to the radiation. Ultraviolet radiation can also be used. The intensity and duration of exposure can also be adjusted to minimize undesirable effects of radiation on the polynucleotides. Boiling polynucleotides can also produce fragments. Typically a solution of polynucleotides is boiled for a couple hours under constant agitation. Fragments of about 500 bp can be achieved. The size of the fragments can vary with the duration of boiling.

Polynucleotide products can result from enzymatic cleavage of single or multi-stranded polynucleotides. Multistranded polynucleotides include polynucleotide complexes comprising more than one strand of polynucleotides, including for example, double and triple stranded polynucleotides. Depending on the enzyme used, the polynucleotides are cut nonspecifically or at specific nucleotides sequences. Any enzyme capable of cleaving a polynucleotide can be used including but not limited to endonucleases, exonucleases, ribozymes, and DNAzymes. Enzymes useful for cleaving polynucleotides are known in the art and are commercially available. See for example Sambrook, J., Russell, D. W., Molecular Cloning: A Laboratory Manual, the third edition, Cold Spring Harbor Laboratory Press, Cold Spring Harbor, N.Y., 2001. Enzymes can also be used to degrade large polynucleotides into smaller fragments.

Endonucleases are an exemplary class of enzymes useful for cleaving polynucleotides. Endonucleases have the capability to cleave the bonds within a polynucleotide strand. Endonucleases can be specific for either double-stranded or single stranded polynucleotides. Cleavage can occur randomly within the polynucleotide or can cleave at specific sequences. Endonucleases which randomly cleave double strand polynucleotides often make interactions with the backbone of the polynucleotide. Specific cleavage of polynucleotides can be accomplished using one or more enzymes is sequential reactions or contemporaneously. Homogenous or heterogenous polynucleotides can be cleaved. Cleavage can be achieved by treatment with nuclease enzymes provided from a variety of sources including the Cleavase.TM. enzyme, Taq DNA polymerase, E. coli DNA polymerase I and eukaryotic structure-specific endonucleases, murine FEN-1 endonucleases [Harrington and Liener, (1994) Genes and Develop. 8:1344] and calf thymus 5' to 3' exonuclease [Murante, R. S., et al. (1994) J. Biol. Chem. 269:1191]). In addition, enzymes having 3' nuclease activity such as members of the family of DNA repair endonucleases (e.g., the Rrpl enzyme from Drosophila melanogaster, the yeast RAD1/RAD10 complex and E. coli Exo III), can also be used for enzymatic cleavage.

Restriction endonucleases are a subclass of endonucleases which recognize specific sequences within double-strand polynucleotides and typically cleave both strands either within or close to the recognition sequence. One commonly used enzyme in DNA analysis is HaeIII, which cuts DNA at the sequence 5'-GGCC-3'. Other exemplary restriction endonucleases include Acc I, Afl III, Alu I, Alw44 I, Apa I, Asn I, Ava I, Ava II, BamH I, Ban II, Bcl I, Bgl I. Bgl II, Bln I, Bsm I, BssH II, BstE II, Cfo I, Cla I, Dde I, Dpn I, Dra I, EclX I, EcoR I, EcoR I, EcoR II, EcoR V, Hae II, Hae II, Hind II, Hind III, Hpa I, Hpa II, Kpn I, Ksp I, Mlu I, MluN I, Msp I, Nci I, Nco I, Nde I, Nde II, Nhe I, Not I, Nru I, Nsi I, Pst I, Pvu I, Pvu II, Rsa I, Sac I, Sal I, Sau3A I, Sca I, ScrF I, Sfi I, Sma I, Spe I, Sph I, Ssp I, Stu I, Sty I, Swa I, Taq I, Xba I, Xho I etc.. The cleavage sites for these enzymes are known in the art.

Restriction enzymes are divided in types I, II, and III. Type I and type II enzymes carry modification and ATP-dependent cleavage in the same protein. Type III enzymes cut DNA at a recognition site and then dissociate from the DNA. Type I enzymes cleave at random sites within the DNA. Any class of restriction endonucleases can be used to fragment polynucleotides. Depending on the enzyme used, the cut in the polynucleotide can result in one strand overhanging the other also known as "sticky" ends. BamHI generates cohesive 5' overhanging ends. KpnI generates cohesive 3' overhanging ends. Alternatively, the cut can result in "blunt" ends that do not have an overhanging end. DraI cleavage generates blunt ends. Cleavage recognition sites can be masked, for example by methylation, if needed. Many of the known restriction endonucleases have 4 to 6 base-pair recognition sequences (Eckstein and Lilley (eds.), Nucleic Acids and Molecular Biology, vol. 2, Springer-Verlag, Heidelberg [1988]), including cleavage sites at inosine bases, for example.

A small number of rare-cutting restriction enzymes with 8 base-pair specificities have been isolated and these are widely used in genetic mapping, but these enzymes are few in number, are limited to the recognition of G+C-rich sequences, and cleave at sites that tend to be highly clustered (Barlow and Lehrach, Trends Genet., 3:167 [1987]). Recently, endonucleases encoded by group I introns have been discovered that might have greater than 12 base-pair specificity (Perlman and Butow, Science 246:1106 [1989]).

Restriction endonucleases can be used to generate a variety of polynucleotide fragment sizes. For example, CviJ1 is a restriction endonuclease that recognizes between a two and three base DNA sequence. Complete digestion with CviJ1 can result in DNA fragments averaging from 16 to 64 nucleotides in length. Partial digestion with CviJ1 can therefore fragment DNA in a "quasi" random fashion similar to shearing or sonication. CviJ1 normally cleaves RGCY sites between the G and C leaving readily cloneable blunt ends, wherein R is any purine and Y is any pyrimidine. In the presence of 1 mM ATP and 20% dimethyl sulfoxide the specificity of cleavage is relaxed and CviJ1 also cleaves RGCN and YGCY sites. Under these "star" conditions, CviJ1 cleavage generates quasi-random digests. Digested or sheared DNA can be size selected at this point.

Methods for using restriction endonucleases to fragment polynucleotides are widely known in the art. In one exemplary protocol a reaction mixture of 20-50 .mu.l is prepared containing: DNA 1-3 .mu.g; restriction enzyme buffer 1.times.; and a restriction endonuclease 2 units for 1 .mu.g of DNA. Suitable buffers are also known in the art and include suitable ionic strength, cofactors, and optionally, pH buffers to provide optimal conditions for enzymatic activity. Specific enzymes can require specific buffers which are generally available from commercial suppliers of the enzyme. An exemplary buffer is potassium glutamate buffer (KGB). Hannish, J. and M. McClelland. (1988). Activity of DNA modification and restriction enzymes in KGB, a potassium glutamate buffer. Gene Anal. Tech. 5:105; McClelland, M. et al. (1988) A single buffer for all restriction endonucleases. Nucleic Acid Res. 16:364. The reaction mixture is incubated at 37.degree. C. for 1 hour or for any time period needed to produce fragments of a desired size or range of sizes. The reaction can be stopped by heating the mixture at 65.degree. C. or 80.degree. C. as needed. Alternatively, the reaction can be stopped by chelating divalent cations such as Mg.sup.2+ with for example, EDTA.

More than one enzyme can be used to cleave the polynucleotide. Multiple enzymes can be used in sequential reactions or in the same reaction provided the enzymes are active under similar conditions such as ionic strength, temperature, or pH. Typically, multiple enzymes are used with a standard buffer such as KGB. The polynucleotides can be partially or completely digested. Partially digested means only a subset of the restriction sites are cleaved. Complete digestion means all of the restriction sites are cleaved.

Endonucleases can be specific for certain types of polynucleotides. For example, endonuclease can be specific for DNA or RNA. Ribonuclease H is an endoribonuclease that specifically degrades the RNA strand in an RNA-DNA hybrid. Ribonuclease A is an endoribonuclease that specifically attacks single-stranded RNA at C and U residues. Ribonuclease A catalyzes cleavage of the phosphodiester bond between the 5'-ribose of a nucleotide and the phosphate group attached to the 3'-ribose of an adjacent pyrimidine nucleotide. The resulting 2',3'-cyclic phosphate can be hydrolyzed to the corresponding 3'-nucleoside phosphate. RNase T1 digests RNA at only G ribonucleotides and RNase U.sub. 2 digests RNA at only A ribonucleotides. The use of mono-specific RNases such as RNase T.sub.1 (G specific) and RNase U.sub.2 (A specific) has become routine (Donis-Keller et al., Nucleic Acids Res. 4: 2527-2537 (1977); Gupta and Randerath, Nucleic Acids Res. 4: 1957-1978 (1977); Kuchino and Nishimura, Methods Enzymol. 180: 154-163 (1989); and Hahner et al., Nucl. Acids Res. 25(10): 1957-1964 (1997)). Another enzyme, chicken liver ribonuclease (RNase CL3) has been reported to cleave preferentially at cytidine, but the enzyme's proclivity for this base has been reported to be affected by the reaction conditions (Boguski et al., J. Biol. Chem. 255: 2160-2163 (1980)). Recent reports also claim cytidine specificity for another ribonuclease, cusativin, isolated from dry seeds of Cucumis sativus L (Rojo et al., Planta 194: 328-338 (1994)). Alternatively, the identification of pyrimidine residues by use of RNase PhyM (A and U specific) (Donis-Keller, H. Nucleic Acids Res. 8: 3133-3142 (1980)) and RNase A (C and U specific) (Simoncsits et al., Nature 269: 833-836 (1977); Gupta and Randerath, Nucleic Acids Res. 4: 1957-1978 (1977)) has been demonstrated. In order to reduce ambiguities in sequence determination, additional limited alkaline hydrolysis can be performed. Since every phosphodiester bond is potentially cleaved under these conditions, information about omitted and/or unspecific cleavages can be obtained this way ((Donis-Keller et al., Nucleic Acids Res. 4: 2527-2537 (1977)). Benzonase.TM., nuclease P1, and phosphodiesterase I are nonspecific endonucleases that are suitable for generating polynucleotide fragments ranging from 200 base pairs or less. Benzonase.TM. is a genetically engineered endonuclease which degrades both DNA and RNA strands in many forms and is described in U.S. Pat. No. 5,173,418.

DNA glycosylases specifically remove a certain type of nucleobase from a given DNA fragment. These enzymes can thereby produce abasic sites, which can be recognized either by another cleavage enzyme, cleaving the exposed phosphate backbone specifically at the abasic site and producing a set of nucleobase specific fragments indicative of the sequence, or by chemical means, such as alkaline solutions and or heat. The use of one combination of a DNA glycosylase and its targeted nucleotide would be sufficient to generate a base specific pattern of any given target region.

Numerous DNA glycosylases are known. For example, a DNA glycosylase can be uracil-DNA glycolsylase (UDG), 3-methyladenine DNA glycosylase, 3-methyladenine DNA glycosylase II, pyrimidine hydrate-DNA glycosylase, FaPy-DNA glycosylase, thymine mismatch-DNA glycosylase, hypoxanthine-DNA glycosylase, 5-Hydroxymethyluracil DNA glycosylase (HmUDG), 5-Hydroxymethylcytosine DNA glycosylase, or 1,N6-etheno-adenine DNA glycosylase (see, e.g., U.S. Pat. Nos. 5,536,649; 5,888,795; 5,952,176; 6,099,553; and 6,190,865 B1; International PCT application Nos. WO 97/03210, WO 99/54501; see, also, Eftedal et al. (1993) Nucleic Acids Res 21:2095-2101, Bjelland and Seeberg (1987) Nucleic Acids Res. 15:2787-2801, Saparbaev et al. (1995) Nucleic Acids Res. 23:3750-3755, Bessho (1999) Nucleic Acids Res. 27:979-983) corresponding to the enzyme's modified nucleotide or nucleotide analog target.

Uracil, for example, can be incorporated into an amplified DNA molecule by amplifying the DNA in the presence of normal DNA precursor nucleotides (e.g. dCTP, dATP, and dGTP) and dUTP. When the amplified product is treated with UDG, uracil residues are cleaved. Subsequent chemical treatment of the products from the UDG reaction results in the cleavage of the phosphate backbone and the generation of nucleobase specific fragments. Moreover, the separation of the complementary strands of the amplified product prior to glycosylase treatment allows complementary patterns of fragmentation to be generated. Thus, the use of dUTP and Uracil DNA glycosylase allows the generation of T specific fragments for the complementary strands, thus providing information on the T as well as the A positions within a given sequence. A C-specific reaction on both. (complementary) strands (i.e., with a C-specific glycosylase) yields information on C as well as G positions within a given sequence if the fragmentation patterns of both amplification strands are analyzed separately. With the glycosylase method and mass spectrometry, a full series of A, C, G and T specific fragmentation patterns can be analyzed.

Several methods exist where treatment of DNA with specific chemicals modifies existing bases so that they are recognized by specific DNA glycosylases. For example, treatment of DNA with alkylating agents such as methylnitrosourea generates several alkylated bases including N3-methyladenine and N3-methylguanine which are recognized and cleaved by alkyl purine DNA-glycosylase. Treatment of DNA with sodium bisulfite causes deamination of cytosine residues in DNA to form uracil residues in the DNA which can be cleaved by uracil N-glycosylase (also known as uracil DNA-glycosylase). Chemical reagents can also convert guanine to its oxidized form, 8-hydroxyguanine, which can be cleaved by formamidopyrimidine DNA N-glycosylase (FPG protein) (Chung et al., "An endonuclease activity of Escherichia coli that specifically removes 8-hydroxyguanine residues from DNA," Mutation Research 254: 1-12 (1991)). The use of mismatched nucleotide glycosylases have been reported for cleaving polynucleotides at mismatched nucleotide sites for the detection of point mutations (Lu, A-L and Hsu, I-C, Genomics (1992) 14, 249-255 and Hsu, I-C., et al, Carcinogenesis (1994)14, 1657-1662). The glycosylases used include the E. coli Mut Y gene product which releases the mispaired adenines of A/G mismatches efficiently, and releases A/C mismatches albeit less efficiently, and human thymidine DNA glycosylase which cleaves at Gfr mismatches. Cleavage products are produced by glycosylase treatment and subsequent cleavage of the abasic site.

Cleavage of nucleic acids for the methods as provided herein can also be accomplished by dinucleotide ("2 cutter") or relaxed dinucleotide ("1 and 1/2 cutter", e.g.) cleavage specificity. Dinucleotide-specific cleavage reagents are known to those of skill in the art (see, e.g., WO 94/21663; Cannistraro et al., Eur. J. Biochem., 181:363-370, 1989; Stevens et al., J. Bacteriol., 164:57-62, 1985; Marotta et al., Biochemistry, 12:2901-2904, 1973). Stringent or relaxed dinucleotide-specific cleavage can also be engineered through the enzymatic and chemical modification of the target nucleic acid. For example, transcripts of the target nucleic acid of interest can be synthesized with a mixture of regular and .alpha.-thio-substrates and the phosphorothioate intemucleoside linkages can subsequently be modified by alkylation using reagents such as an alkyl halide (e.g., iodoacetamide, iodoethanol) or 2,3-epoxy-1-propanol. The phosphotriester bonds formed by such modification are not expected to be substrates for RNAses. Using this procedure, a mono-specific RNAse, such as RNAse-T1, can be made to cleave any three, two or one out of the four possible GpN bonds depending on which substrates are used in the .alpha.-thio form for target preparation. The repertoire of useful dinucleotide-specific cleavage reagents can be further expanded by using additional RNAses, such as RNAse-U2 and RNAse-A. In the case of RNAse A, for example, the cleavage specificity can be restricted to CpN or UpN dinucleotides through enzymatic incorporation of the 2'-modified form of appropriate nucleotides, depending on the desired cleavage specificity. Thus, to make RNAse A specific for CpG nucleotides, a transcript (target molecule) is prepared by incorporating .alpha.S-dUTP, .alpha.S ATP, .alpha.S-CTP and GTP nucleotides. These selective modification strategies can also be used to prevent cleavage at every base of a homopolymer tract by selectively modifying some of the nucleotides within the homopolymer tract to render the modified nucleotides less resistant or more resistant to cleavage.

DNAses can also be used to generate polynucleotide fragments. Anderson, S. (1981) Shotgun DNA sequencing using cloned DNase I-generated fragments. Nucleic Acids Res. 9:3015-3027. DNase I (Deoxyribonuclease I) is an endonuclease that digests double- and single-stranded DNA into poly- and mono-nucleotides. The enzyme is able to act upon single as well as double-stranded DNA and on chromatin.

Deoxyribonuclease type II is used for many applications in nucleic acid research including DNA sequencing and digestion at an acidic pH. Deoxyribonuclease II from porcine spleen has a molecular weight of 38,000 daltons. The enzyme is a glycoprotein endonuclease with dimeric structure. Optimum pH range is 4.5-5.0 at ionic strength 0.15 M. Deoxyribonuclease II hydrolyzes deoxyribonucleotide linkages in native and denatured DNA yielding products with 3'-phosphates. It also acts on p-nitrophenylphosphodiesters at pH 5.6-5.9. Ehrlich, S. D. et al. (1971) Studies on acid deoxyribonuclease. IX. 5'-Hydroxy-terminal and penultimate nucleotides of oligonucleotides obtained from calf thymus deoxyribonucleic acid. Biochemistry. 10(11):2000-9.

Large single stranded polynucleotides can be fragmented into small polynucleotides using nuclease that remove various lengths of bases from the end of a polynucleotide. Exemplary nucleases for removing the ends of single stranded polynucleotides include but are not limited to S1, Bal 31, and mung bean nucleases. For example, mung bean nuclease degrades single stranded DNA to mono or polynucleotides with phosphate groups at their 5' termini. Double stranded nucleic acids can be digested completely if exposed to very large amounts of this enzyme.

Exonucleases are proteins that also cleave nucleotides from the ends of a polynucleotide, for example a DNA molecule. There are 5' exonucleases (cleave the DNA from the 5'-end of the DNA chain) and 3' exonucleases (cleave the DNA from the 3'-end of the chain). Different exonucleases can hydrolyse single-strand or double strand DNA. For example, Exonuclease III is a 3' to 5' exonuclease, releasing 5'-mononucleotides from the 3'-ends of DNA strands; it is a DNA 3'-phosphatase, hydrolyzing 3'-terminal phosphomonoesters; and it is an AP endonuclease, cleaving phosphodiester bonds at apurinic or apyrimidinic sites to produce 5'-termini that are base-free deoxyribose 5'-phosphate residues. In addition, the enzyme has an RNase H activity; it will preferentially degrade the RNA strand in a DNA-RNA hybrid duplex, presumably exonucleolytically. In mammalian cells, the major DNA 3'-exonuclease is DNase III (also called TREX-1). Thus, fragments can be formed by using exonucleases to degrade the ends of polynucleotides.

Catalytic DNA and RNA are known in the art and can be used to cleave polynucleotides to produce polynucleotide fragments. Santoro, S. W. and Joyce, G. F. (1997) A general purpose RNA-cleaving DNA enzyme. Proc. Natl. Acad. Sci. USA 94: 4262-4266. DNA as a single-stranded molecule can fold into three dimensional structures similar to RNA, and the 2'-hydroxy group is dispensable for catalytic action. As ribozymes, DNAzymes can also be made, by selection, to depend on a cofactor. This has been demonstrated for a histidine-dependent DNAzyme for RNA hydrolysis. U.S. Pat. Nos. 6,326,174 and 6,194,180 disclose deoxyribonucleic acid enzymes-catalytic or enzymatic DNA molecules-capable of cleaving nucleic acid sequences or molecules, particularly RNA. U.S. Pat. Nos. 6,265,167; 6,096,715; 5,646,020 disclose ribozyme compositions and methods.

A DNA nickase, or DNase, can be used to recognize and cleave one strand of a DNA duplex. Numerous nickases are known. Among these, for example, are nickase NY2A nickase and NYS1 nickase (Megabase) with the following cleavage sites:

1 NY2A: 5'...R AG...3' 3'...Y TC...5' where R = A or G and Y = C or T NYS1: 5'...CC[A/G/T]...3' 3'...GG[T/C/A]...5'.

Subsequent chemical treatment of the products from the nickase reaction results in the cleavage of the phosphate backbone and the generation of fragments.

The Fen-1 fragmentation method involves the enzymes Fen-1 enzyme, which is a site-specific nuclease known as a "flap" endonuclease (U.S. Pat. Nos. 5,843,669, 5,874,283, and 6,090,606). This enzyme recognizes and cleaves DNA "flaps" created by the overlap of two oligonucleotides hybridized to a target DNA strand. This cleavage is highly specific and can recognize single base pair mutations, permitting detection of a single homologue from an individual heterozygous at one SNP of interest and then genotyping that homologue at other SNPs occurring within the fragment. Fen-1 enzymes can be Fen-1 like nucleases e.g. human, murine, and Xenopus XPG enzymes and yeast RAD2 nucleases or Fen-1 endonucleases from, for example, M. jannaschii, P. furiosus, and P. woesei.

Another technique, which is under development as a diagnostic tool for detecting the presence of M. tuberculosis, can be used to cleave DNA chimeras. Tripartite DNA-RNA-DNA probes are hybridized to target nucleic acids, such as M. tuberculosis-specific sequences. Upon the addition of RNAse H, the RNA portion of the chimeric probe is degraded, releasing the DNA portions [Yule, Bio/Technology 12:1335 (1994)].

Fragments can also be formed using any combination of cleavage methods as well as any combination of enzymes. Methods for producing specific cleavage products can be combined with methods for producing random cleavage products. Additionally, one or more enzymes that cleave a polynucleotide at a specific site can be used in combination with one or more enzymes that specifically cleave the polynucleotide at a different site. In another example, enzymes that cleave specific kinds of polynucleotides can be used in combination, for example, an RNase in combination with a DNase. In still another example, an enzyme that cleaves polynucleotides randomly can be used in combination with an enzyme that cleaves polynucleotides specifically. Used in combination means performing one or more methods after another or contemporaneously on a polynucleotide.

*Peptide Fragmentation/Cleavage*

As interest in proteomics has increased as a field of study, a number of techniques have been developed for protein fragmentation for use in protein sequencing. Among these are chemical and enzymatic hydrolysis, and fragmentation by ionization energy.

Sequential cleavage of the N-terminus of proteins is well known in the art, and can be accomplished using Edman degradation. In this process, the N-terminal amino acid is reacted with phenylisothiocyanate to a PTC-protein with an intermediate anilinothiazolinone forming when contacted with trifluoroacetic acid. The intermediate is cleaved and converted to the phenylthiohydantoin form and subsequently separated, and identified by comparison to a standard. To facilitate protein cleavage, proteins can be reduced and alkylated with vinylpyridine or iodoacetamide.

Chemical cleavage of proteins using cyanogen bromide is well known in the art (Nikodem and Fresco, Anal. Biochem. 97: 382-386 (1979); Jahnen et al., Biochem. Biophys. Res. Commun. 166: 139-145 (1990)). Cyanogen bromide (CNBr) is one of the best methods for initial cleavage of proteins. CNBr cleaves proteins at the C-terminus of methionyl residues. Because the number of methionyl residues in proteins is usually low, CNBr usually generates a few large fragments. The reaction is usually performed in a 70% formic acid or 50% trifluoroacetic acid with a 50- to 100-fold molar excess of cyanogen bromide to methionine. Cleavage is usually quantitative in 10-12 hours, although the reaction is usually allowed to proceed for 24 hours. Some Met-Thr bonds are not cleaved, and cleavage can be prevented by oxidation of methionines.

Proteins can also be cleaved using partial acid hydrolysis methods to remove single terminal amino acids (Vanfleteren et al., BioTechniques 12: 550-557 (1992). Peptide bonds containing aspartate residues are particularly susceptible to acid cleavage on either side of the aspartate residue, although usually quite harsh conditions are needed. Hydrolysis is usually performed in concentrated or constant boiling hydrochloric acid in sealed tubes at elevated temperatures for various time intervals from 2 to 18 hours. Asp-Pro bonds can be cleaved by 88% formic acid at 37.degree.. Asp-Pro bonds have been found to be susceptible under conditions where other Asp-containing bonds are quite stable. Suitable conditions are the incubation of protein (at about 5 mg/ml) in 10% acetic acid, adjusted to pH 2.5 with pyridine, for 2 to 5 days at 40.degree. C.

Brominating reagents in acidic media have been used to cleave polypeptide chains. Reagents such as N-bromosuccinimide will cleave polypeptides at a variety of sites, including tryptophan, tyrosine, and histidine, but often give side reactions which lead to insoluble products. BNPS-skatole[2-(2-nitrophenylsulfenyl)-3-methylindole] is a mild oxidant and brominating reagent that leads to polypeptide cleavage on the C-terminal side of tryptophan residues.

Although reaction with tyrosine and histidine can occur, these side reactions can be considerably reduced by including tyrosine in the reaction mix. Typically, protein at about 10 mg/ml is dissolved in 75% acetic acid and a mixture of BNPS-skatole and tyrosine (to give 100-fold excess over tryptophan and protein tyrosine, respectively) is added and incubated for 18 hours. The peptide-containing supernatant is obtained by centrifugation.

Apart from the problem of mild acid cleavage of Asp-Pro bonds, which is also encountered under the conditions of BNPS-skatole treatment, the only other potential problem is the fact that any methionine residues are converted to methioninesulfoxide, which cannot then be cleaved by cyanogen bromide. If CNBr cleavage of peptides obtained from BNPS-skatole cleavage is necessary, the methionine residues can be regenerated by incubation with 15% mercaptoethanol at 30.degree. C. for 72 hours.

Treating proteins with o-Iodosobenzoic acid cleaves tryptophan-X bonds under quite mild conditions. Protein, in 80% acetic acid containing 4 M guanidine hydrochloride, is incubated with iodobenzoic acid (approximately 2 mg/ml of protein) that has been preincubated with p-cresol for 24 hours in the dark at room temperature. The reaction can be terminated by the addition of dithioerythritol. Care must be taken to use purified o-iodosobenzoic acid since a contaminant, o-iodoxybenzoic acid, will cause cleavage at tyrosine-X bonds and possibly histidine-X bonds. The function of p-cresol in the reaction mix is to act as a scavenging agent for residual o-iodoxybenzoic acid and to improve the selectivity of cleavage.

Two reagents are available that produce cleavage of peptides containing cysteine residues. These reagents are (2-methyl) N-1-benzenesulfonyl-N-4-(bromoacetyl)quinone diimide (otherwise known as Cyssor, for "cysteine-specific scission by organic reagent") and 2-nitro-5-thiocyanobenzoic acid (NTCB). In both cases cleavage occurs on the amino-terminal side of the cysteine.

Incubation of proteins with hydroxylamine results in the cleavage of the polypeptide backbone (Saris et al., Anal. Biochem. 132: 54-67 (1983). Hydroxylaminolysis leads to cleavage of any asparaginyl-glycine bonds. The reaction occurs by incubating protein, at a concentration of about 4 to 5 mg/ml, in 6 M guanidine hydrochloride, 20 mM sodium acetate+1% mercaptoethanol at pH 5.4, and adding an equal volume of 2 M hydroxylamine in 6 M guanidine hydrochloride at pH 9.0. The pH of the resultant reaction mixture is kept at 9.0 by the addition of 0.1 N NaOH and the reaction allowed to proceed at 45.degree. C. for various time intervals; it can be terminated by the addition of 0.1 volume of acetic acid. In the absence of hydroxylamine, a base-catalyzed rearrangement of the cyclic imide intermediate can take place, giving a mixture of .alpha.-aspartylglycine and .beta.-aspartylglycine without peptide cleavage.

There are many methods known in the art for hydrolysing protein by use of a proteolytic enzymes (Cleveland et al., J. Biol. Chem. 252: 1102-1106 (1977). All peptidases or proteases are hydrolases which act on protein or its partial hydrolysate to decompose the peptide bond. Native proteins are poor substrates for proteases and are usually denatured by treatment with urea prior to enzymatic cleavage. The prior art discloses a large number of enzymes exhibiting peptidase, aminopeptidase and other enzyme activities, and the enzymes can be derived from a number of organisms, including vertebrates, bacteria, fungi, plants, retroviruses and some plant viruses. Proteases have been useful, for example, in the isolation of recombinant proteins. See, for example, U.S. Pat. Nos. 5,387,518, 5,391,490 and 5,427,927, which describe various proteases and their use in the isolation of desired components from fusion proteins.

The proteases can be divided into two categories. Exopeptidases, which include carboxypeptidases and aminopeptidases, remove one or more amino terminal residues from polypeptides. Endopeptidases, which cleave within the polypeptide sequence, cleave between specific residues in the protein sequence. The various enzymes exhibit differing requirements for optimum activity, including ionic strength, temperature, time and pH. There are neutral endoproteases (such as Neutrase.TM.) and alkline endoproteases (such as Alcalase.TM. and Esperase.TM.), as well as acid-resistant carboxypeptidases (such as carboxypeptidase-P).

There has been extensive investigation of proteases to improve their activity and to extend their substrate specificity (for example, see U.S. Pat. Nos. 5,427,927; 5,252,478; and 6,331,427 B1). One method for extending the targets of the proteases has been to insert into the target protein the cleavage sequence that is required by the protease. Recently, a method has been disclosed for making and selecting site-specific proteases ("designer proteases") able to cleave a user-defined recognition sequence in a protein (see U.S. Pat. No. 6,383,775).

The different endopeptidase enzymes cleave proteins at a diverse selection of cleavage sites. For example, the endopeptidase renin cleaves between the leucine residues in the following sequence: Pro-Phe-His-Leu-Leu-Val-Tyr (SEQ ID NO:1) (Haffey, M. L. et al., DNA 6:565 (1987). Factor Xa protease cleaves after the Arg in the following sequences: Ile-Glu-Gly-Arg-X; Ile-Asp-Gly-Arg-X; and Ala-Glu-Gly-Arg-X, where X is any amino acid except proline or arginine, (SEQ ID NOS:2-4, respectively) (Nagai, K. and Thogersen, H. C., Nature 309:810 (1984); Smith, D. B. and Johnson, K. S. Gene 67:31 (1988)). Collagenase cleaves following the X and Y residues in following sequence: -Pro-X-Gly-Pro-Y- (where X and Y are any amino acid) (SEQ ID NO:5) (Germino J. and Bastis, D., Proc. Natl. Acad. Sci. USA 81:4692 (1984)). Glutamic acid endopeptidase from S. aureus V8 is a serine protease specific for the cleavage of peptide bonds at the carboxy side of aspartic acid under acid conditions or glutamic acid alkaline conditions.

Trypsin specifically cleaves on the carboxy side of arginine, lysine, and S-aminoethyl-cysteine residues, but there is little or no cleavage at arginyl-proline or lysyl-proline bonds. Pepsin cleaves preferentially C-terminal to phenylalanine, leucine, and glutamic acid, but it does not cleave at valine, alanine, or glycine. Chymotrypsin cleaves on the C-terminal side of phenylalanine, tyrosine, tryptophan, and leucine. Aminopeptidase P is the enzyme responsible for the release of any N-terminal amino acid adjacent to a proline residue. Proline dipeptidase (prolidase) splits dipeptides with a prolyl residue in the carboxyl terminal position.

*Ionization Fragmentation Cleavage of Peptides or Nucleic Acids*

Ionization fragmentation of proteins or nucleic acids is accomplished during mass spectrometric analysis either by using higher voltages in the ionization zone of the mass spectrometer (MS) to fragment by tandem MS using collision-induced dissociation in the ion trap. (see, e.g., Bieman, Methods in Enzymology, 193:455-479 (1990)). The amino acid or base sequence is deduced from the molecular weight differences observed in the resulting MS fragmentation pattern of the peptide or nucleic acid using the published masses associated with individual amino acid residues or nucleotide residues in the MS.

Complete sequencing of a protein is accomplished by cleavage of the peptide at almost every residue along the peptide backbone. When a basic residue is located at the N-terminus and/or C-terminus, most of the ions produced in the collision induced dissociation (CID) spectrum will contain that residue (see, Zaia, J., in: Protein and Peptide Analysis by Mass Spectrometry, J. R. Chapman, ed., pp. 29-41, Humana Press, Totowa, N.J., 1996; and Johnson, R. S., et al., Mass Spectrom. Ion Processes, 86:137-154 (1988)) since positive charge is generally localized at the basic site. The presence of a basic residue typically simplifies the resulting spectrum, since a basic site directs the fragmentation into a limited series of specific daughter ions. Peptides that lack basic residues tend to fragment into a more complex mixture of fragment ions that makes sequence determination more difficult. This can be overcome by attaching a hard positive charge to the N-terminus. See, Johnson, R. S., et al., Mass Spectrom. Ion Processes, 86:137-154 (1988); Vath, J. E., et al., Fresnius Z Anal. Chem., 331:248-252 (1988); Stults, J. T., et al., Anal. Chem., 65:1703-1708 (1993); Zaia, J., et al., J Am. Soc. Mass Spectrom., 6:423-436 (1995); Wagner, D. S., et al., Biol. Mass Spectrom., 20:419-425 (1991); and Huang, Z.-H., et al., Anal. Biochem., 268:305-317 (1999). The proteins can also be chemically modified to include a label which modifies its molecular weight, thereby allowing differentiation of the mass fragments produced by ionization fragmentation. The labeling of proteins with various agents is known in the art and a wide range of labeling reagents and techniques useful in practicing the methods herein are readily available to those of skill in the art. See, for example, Means et al., Chemical Modification of Proteins, Holden-Day, San Francisco, 1971; Feeney et al., Modification of Proteins: Food, Nutritional and Pharmacological Aspects, Advances in Chemistry Series, Vol. 198, American Chemical Society, Washington, D.C., 1982).

The methods described herein can be used to analyze target nucleic acid or peptide cleavage products obtained by specific cleavage as provided above for various purposes including, but not limited to, identification, polymorphism detection, SNP scanning, bacteria and viral typing, pathogen detection, identification and characterization, antibiotic profiling, organism identification, identification of disease markers, methylation analysis, microsatellite analysis, haplotyping, genotyping, determination of allelic frequency, multiplexing, and nucleotide sequencing and resequencing.

Detection and Identification of Sequence Information from Biomolecule Fragments

Since the sequence of about sixteen (16) nucleotides is specific on a statistical basis for the human genome, relatively short nucleic acid sequences can be used to detect normal and defective genes in higher organisms and to detect infectious microorganisms (e.g., bacteria, fungi, protists and yeast) and viruses. DNA sequences can serve as a fingerprint for detection of different individuals within the same species (see, Thompson, J. S. and M. W. Thompson, eds., Genetics in Medicine, W.B. Saunders Co., Philadelphia, Pa. (1991)).

Several methods for detecting DNA are in use. For example, nucleic acid sequences are identified by comparing the mobility of an amplified nucleic acid molecule with a known standard by gel electrophoresis, or by hybridization with a probe, which is complementary to the sequence to be identified. Identification, however, can only be accomplished if the nucleic acid molecule is labeled with a sensitive reporter function (e.g., radioactive (.sup.32P, .sup.35S), fluorescent or chemiluminescent). Radioactive labels can be hazardous and the signals they produce decay over time. Non-isotopic labels (e.g., fluorescent) suffer from a lack of sensitivity and fading of the signal when high intensity lasers are used. Additionally, performing labeling, electrophoresis and subsequent detection are laborious, time-consuming and error-prone procedures. Electrophoresis is particularly error-prone, since the size or the molecular weight of the nucleic acid cannot be directly correlated to the mobility in the gel matrix. It is known that sequence specific effects, secondary structure and interactions with the gel matrix cause artifacts. Moreover, the molecular weight information obtained by gel electrophoresis is a result of indirect measurement of a related parameter, such as mobility in the gel matrix.

Applications of mass spectrometry in the biosciences have been reported (see Meth. Enzymol., Vol. 193, Mass Spectrometry (McCloskey, ed.; Academic Press, NY 1990); McLaffery et al., Acc. Chem. Res. 27:297-386 (1994); Chait and Kent, Science 257:1885-1894 (1992); Siuzdak, Proc. Natl. Acad. Sci., USA 91:11290-11297 (1994)), including methods for mass spectrometric analysis of biopolymers (see Hillenkamp et al. (1991) Anal. Chem. 63:1193A-1202A) and for producing and analyzing biopolymer ladders (see, International Publ. WO 96/36732; U.S. Pat. No. 5,792,664). Mass spectrometric techniques applied to biomolecules include, but are not limited to Matrix-Assisted Laser Desorption/Ionization, Time-of-Flight (MALDI-TOF), Electrospray (ES), IR-MALDI (see, e.g., published International PCT application No.99/57318 and U.S. Pat. No. 5,118;937), Ion Cyclotron Resonance (ICR), Fourier Transform and combinations thereof.

MALDI-MS generally involves analyzing a biomolecule in a matrix, and has been performed on polypeptides and on nucleic acids mixed in a solid (i.e., crystalline) matrix. In these methods, a laser is used to strike the biopolymer/matrix mixture, which is crystallized on a probe tip, thereby effecting desorption and ionization of the biopolymer. In addition, MALDI-MS has been performed on polypeptides using the water of hydration (i.e., ice) or glycerol as a matrix. When the water of hydration was used as a matrix, it was necessary to first lyophilize or air dry the protein prior to performing MALDI-MS (Berkenkamp et al. (1996) Proc. Natl. Acad. Sci. USA 93:7003-7007). The upper mass limit for this method was reported to be 30 kDa with limited sensitivity (i.e., at least 10 pmol of protein was required).

MALDI-TOF mass spectrometry has been employed in conjunction with conventional Sanger sequencing or similar primer-extension based methods to obtain sequence information, including the detection of SNPs (see, e.g., U.S. Pat. Nos. 5,547,835; 6,194,144; 6,225,450; 5,691,141 and 6,238,871; H. Koster et al., Nature BiotechnoL, 14:1123-1128, 1996; WO 96/29431; WO 98/20166; WO 98/12355; U.S. Pat. No. 5,869,242; WO 97/33000; WO 98/54571; A. Braun et al., Genomics, 46:18, 1997; D. P. Little et al., Nat. Med., 3:1413, 1997; L. Haff et al., Genome Res., 7:378, 1997; P. Ross et al., Nat. Biotechnol., 16:1347, 1998; K. Tang et al., Proc. Natl. Acad. Sci. USA, 96:10016, 1999). Since each of the four naturally occurring nucleotide bases dC, dT, dA and dG, also referred to herein as C, T, A and G, in DNA has a different molecular weight: $M.sub.C=289.2$; $M.sub.T=304.2$; $M.sub.A=313.2$; $M.sub.G=329.2$; where M.sub.C, M.sub.T, M.sub.A, M.sub.G are average molecular weights (under the natural isotopic distribution) in daltons of the nucleotide bases deoxycytidine, thymidine, deoxyadenosine, and deoxyguanosine, respectively, it is possible to read an entire sequence in a single mass spectrum. If a single spectrum is used to analyze the products of a conventional Sanger sequencing reaction, where chain termination is achieved at every base position by the incorporation of dideoxynucleotides, a base sequence can be determined by calculation of the mass differences between adjacent peaks. For the detection of SNPs, alleles or other sequence variations (e.g., insertions, deletions), variant-specific primer extension is carried out immediately adjacent to the polymorphic SNP or sequence variation site in the target nucleic acid molecule. The mass of the extension product and the difference in mass between the extended and unextended product is indicative of the type of allele, SNP or other sequence variation.

U.S. Pat. No. 5,622,824, describes methods for DNA sequencing based on mass spectrometric detection. To achieve this, the DNA is by means of protection, specificity of enzymatic activity, or immobilization, unilaterally degraded in a stepwise manner via exonuclease digestion and the nucleotides or derivatives detected by mass spectrometry. Prior to the enzymatic degradation, sets of ordered deletions that span a cloned DNA sequence can be created. In this manner, mass-modified nucleotides can be incorporated using a combination of exonuclease and DNA/RNA polymerase. This permits either multiplex mass spectrometric detection, or modulation of the activity of the exonuclease so as to synchronize the degradative process.

U.S. Pat. Nos. 5,605,798 and 5,547,835 provide methods for detecting a particular nucleic acid sequence in a biological sample. Depending on the sequence to be detected, the processes can be used, for example, in methods of diagnosis.

Technologies have been developed to apply MALDI-TOF mass spectrometry to the analysis of genetic variations such as microsatellites, insertion and/or deletion mutations and single nucleotide polymorphisms (SNPs) on an industrial scale. These technologies can be applied to large numbers of either individual samples, or pooled samples to study allelic frequencies or the frequency of SNPs in populations of individuals, or in heterogeneous tumor samples. The analyses can be performed on chip-based formats in which the target nucleic acids or primers are linked to a solid support, such as a silicon or silicon-coated substrate, preferably in the form of an array (see, e.g., K. Tang et al., Proc. Natl. Acad. Sci. USA, 96:10016, 1999). Generally, when analyses are performed using mass spectrometry, particularly MALDI, small nanoliter volumes of sample are loaded onto a substrate such that the resulting spot is about, or smaller than, the size of the laser spot. It has been found that when this is achieved, the results from the mass spectrometric analysis are quantitative. The area under the signals in the resulting mass spectra are proportional to concentration (when normalized and corrected for background). Methods for preparing and using such chips are described in U.S. Pat. No. 6,024,925, co-pending U.S. application Ser. Nos. 08/786,988, 09/364,774, 09/371,150 and 09/297,575; see, also, U.S. application Ser. No. PCT/US97/20195, which published as WO 98/20020. Chips and kits for performing these analyses are commercially available from SEQUENOM, INC. under the trademark MassARRAY.TM.. MassARRAY.TM. relies on mass spectral analysis combined with the miniaturized array and MALDI-TOF (Matrix-Assisted Laser Desorption Ionization-Time of Flight) mass spectrometry to deliver results rapidly. It accurately distinguishes single base changes in the size of DNA fragments associated with genetic variants without tags.

Although the use of MALDI for obtaining nucleic acid sequence information, especially from DNA fragments as described above, offers the advantages of high throughput due to high-speed signal acquisition and automated analysis off solid surfaces, there are limitations in its application. When the SNP or mutation or other sequence variation is unknown, the variant mass spectrum or other indicator of mass, such as mobility in the case of gel electrophoresis, must be simulated for every possible sequence change of a reference sequence that does not contain the sequence variation. Each simulated variant spectrum corresponding to a particular sequence variation or set of sequence variations must then be matched against the actual variant mass spectrum to determine the most likely sequence change or changes that resulted in the variant spectrum. Such a purely simulation-based approach is time consuming. For example, given a reference sequence of 1000 bases, there exist approximately 9000 potential single base sequence variations. For every such potential sequence variation, one would have to simulate the expected spectra and to match them against the experimentally measured spectra. The problem is further compounded when multiple base variations or multiple sequence variations rather than only single base or sequence variations are present.

Comparative Sequence Analysis Embodiments

Comparative sequence analysis matches peak patterns generated from a sample to peak patterns generated by *in silico* base-specific cleavages from at least one or a set of known reference nucleic acid sequences or reference peak patterns generated from known samples, referred as references. Scores are calculated for each sample against all the references in the set, and one or more references with the best scores are selected as the potential match for each sample. Subsequently variations and confidence values are established and evaluated for each sample against the best match reference.

The first step in the process is to create reference peak patterns. In the case that some reference nucleic acid sequences are known, peak patterns can be obtained by simulating, e.g. RNase-A cleavage reactions or any other chemical cleavage reaction including base-specific and partial cleavage reactions from the reference sequences or from the consensus sequences. Peak patterns can also be obtained by measuring the cleavage reaction products of reference samples (either pure sample or mixture sample). To simulate peak patterns for mixture, two or more patterns from pure samples or reference nucleic acid sequences can be combined. One or more peak lists, e.g., peak lists corresponding to T forward, C forward, T reverse and C reverse cleavage reactions, could be generated for each reference. For each reaction, all the peaks from references in the set are aligned by mass and each reference can then be represented by an n-dimension vector representing peak intensities (0 for not having that peak). The dimension n is the number of simulated masses in the specified mass range for the particular reaction from all the reference peaks in the set. Thus each reference can be represented by one or more vectors.

Distance matrix can be calculated based on these vectors:

$$D_{i,j} = \sum_r \sum_k [(|V_{i,r,k}-V_{j,r,k}|)^3/( V_{i,r,k}+V_{j,r,k})]$$

Where $V_{i,r,k}$ is the intensity for sequence i, reaction r and peak k, $V_{j,r,k}$ is the intensity for sequence j, reaction r and peak k, $\Sigma_k$ is summation over all peaks in reaction r, $\Sigma_r$ is summation over all simulated reactions, and $D_{i,j}$ is the distance between sequence i and j. The distance matrix can be used as input to other software, such as neighbor.exe in PHYLIP package or other packages, to cluster the references.

The reference peak lists and aligned peak patterns can be used to assess what cleavage reactions and how many reactions are required to discriminate all the references in a set. First, references are grouped into clusters based on discriminating features by finding peaks present in one set of references but absent in others. Clusters are then grouped into sub-clusters until each cluster has only one sequence or a set of indistinguishable sequences. Discriminating powers are calculated by summing up intensities of all the discriminating features, which are the unique peaks present only in the cluster as well as peaks with changed intensities from other clusters. The threshold of discriminating power, typically set to 2, is required to distinguish one reference from another with good confidence. By evaluating the discriminating power of all the references, minimum set of cleavage reactions can be determined. If references are substantially different from each other, one reaction could be enough to discriminate them all.

To ensure quality spectra are acquired, spectra are evaluated during acquisition by comparing the detected peak patterns with a set of anchor peaks selected from the reference peak patterns. Anchor peak sets are selected in such a way that all the references are represented by one or more peaks in each anchor peak set. Typically, 10-20 anchor peak sets are selected from the reference peak patterns. In the case where detected sample peak patterns deviate substantially from the reference or references in the set, e.g. only one or a few references are known while samples to be detected might be quite different from the known references, sets of anchors are combined so that all samples can have meaningful quality judgment.

Once spectra are acquired, the next step is to extract all the meaningful peaks. Spectra are first filtered by applying a moving width filter with Gaussian kernel. Peak initial positions are identified by finding local maximum in the filtered spectra. Depending on peak separation, one or a set of peaks are grouped together and a common baseline in the original spectrum is determined for the group. The baseline corrected data points from the original spectrum for the group of peaks are fitted to Gaussian curves:

$$\text{Intensity} = \sum A_i * \exp\{-[(\text{mass} - \text{mass}_i)/\text{width}]^2\}$$

Where $A_i$ and $\text{mass}_i$ are the heights and masses for each peak in the group, width is the common peak width for the group and summation is over all the peaks. Peak intensities and signal to noise ratios (SNR) are then calculated from the heights and widths. Peaks with low SNRs are evaluated to obtain the cutoff for chemical noise peaks and they are removed from the final peak list. Peak intensities are then normalized in such a way that the detected intensities in mass range of 2000-4000 Da agree with those of reference peaks. These intensities are called normalized raw peak intensities.

Before data acquisition, mass spectrometer is usually calibrated by external calibration with calibrants at mass 1479.0, 3004.0, 5044.4 and 8486.6 or as appropriate. All spectra acquired during the session have the same mass calibration. However, due to variations in sample positions, the actual masses in each spectrum could differ from the initial calibration, sometime large enough to affect the identification. Thus, the next step is to calibrate peak masses by internal calibration. First, all the detected peaks are matched to reference peaks within a certain mass window and outliners are removed by evaluating the overall deviation patterns of the detected masses versus the reference masses. Once all the matched peaks are identified, high intensity peaks evenly distributed across the whole mass range are selected as anchor peaks. Then the masses of anchor peaks are fitted to equation:

$$\text{MASS} = A * [\text{sqrt}(B * \text{INDEX} + C) - 1]^2$$

where MASS is the mass of an anchor peak, INDEX is the peak mass index, and A, B and C are the mass calibration coefficients. The fitting typically runs through several rounds. After each round, the worst fit anchor peak is removed, and the fitting is run again until the goodness of fit reaches certain criteria, e.g., mass deviation less than 0.3, or the number of anchor peaks reaches the minimum (such as 5). The final calibration coefficients are then validated by ensuring the masses in different mass region calculated with the two sets of coefficients are close, e.g. masses at lowest mass range is less than 0.5 dalton apart and masses at highest mass range are less than 5 dalton apart. Then the new calibration is applied to all the peaks.

Spectrum quality is evaluated by combining two parts, one from assay and reference independent parameters and another from assay and reference dependent parameters. Assay and reference independent quality $Q_{peak}$ is obtained by considering the average normalized peak intensities and peak SNRs:

$$Q_{snr} = 1.0 - \exp[(2 - ave_{snr})/10$$

$$Q_{intens} = 0.5 * \{1.0/[1.0+\exp((0.3-ave_{intens})*10.0)] + \exp[- 0.25 / (ratio_{aveltoCN}{}^{\wedge}2)]\}$$

$$Q_{peak} = (Q_{intens} + Q_{snr}) / 2$$

Where $ave_{snr}$ is the average SNR for top 10 to 15 peaks in the spectrum, $ave_{intens}$ is the average intensity for top 10 to 15 peaks in the spectrum, and $ratio_{aveltoCN}$ is the ratio of $ave_{intens}$ to average intensity of chemical noise peaks. Chemical noise peaks are peaks not explained by any compomer assignment, i.e., the nucleic acid composition resulting from the specific cleavage reaction. $Q_{peak}$ is a better measure of the quality of peaks in the spectrum regardless whether the correct reference is assigned to it or not. The assay and reference dependent quality is obtained by comparing the number of peaks matching a preselected set of peaks (anchor peak sets) from the reference peak patterns:

$$Q_{match} = Intens_{match} / (Intens_{match} + Intens_{missing})$$

where $Intens_{match}$ is the sum of matched reference anchor peak intensity and $Intens_{missing}$ is sum of missing reference anchor peak intensity. $Q_{match}$ is a better measure whether the reaction works or not. It will also be able to tell if the user assigns wrong reaction or wrong references to the reaction. However, if the sample is not represented by the references in the set, or only one reference is available for a set of different samples, $Q_{match}$ could vary substantially from sample to sample. The overall spectrum quality is weighted combination of the two:

$$Q_{spec} = Q_{peak} * (1- weight) + Q_{match} * weight$$

where weight can be set to between 0 and 0.667 and can be 0.667 by default for samples matching references. Depending on particular experiment setting, weighting for the two qualities can be adjusted to obtain most meaningful spectrum quality.

The raw peak intensities vary over different mass range in the spectra acquired by the MALDI-TOF mass spectrometers. For the MassARRAY compact analyser (Sequenom, Inc.), tuned to a mass range between e.g. 1100 Da to 11000 Da, peaks have highest intensities between 2000 and 4000 Da. The mass dependent variations are corrected by a scaling curve, which is calculated for each spectrum. Depending on spectrometers, alternative fittings may be better. For the MassARRAY compact analyser (Sequenom) spectrometer from Sequenom, inc., the scaling curve is obtained by fitting peak intensities to standard profiles in a maximum of three different mass ranges, a possible center region of 2000 - 5000 Da, lower mass region of 1100 - 2500 Da  and higher mass region of above 4500 Da. The center mass region which can be between 2000 to 5000 Da is the most important region and generally has most of the peaks. Peaks in this region are fitted to Gaussian curve:

$$Intens = A * \exp\{- [(\log(m) - B)/C]^{\wedge}2\}$$

where m and Intens are peak masses and intensities respectively, and A, B and C are Gaussian coefficients; Peaks in lower mass range, e.g., 1100 - 2500 Da are fitted to an exponential increase curve:

$$Intensity = A * \exp(B * mass)$$

where coefficients A and B should always be positive values. Peaks in high mass range, e.g., above 4500 Da, are fitted to an exponential decay curve:

$$\text{Intensity} = A * \exp(- B * \text{mass})$$

where coefficients A and B should also be positive values. The three profiles are joined smoothly into one for the whole mass range to form the final mass scaling factor which represent the expected detected peak intensities at given masses if the reference intensity is 1. This profile is then used to calculate the revised intensities for all detected peaks:

$$I_{revised} = I_{raw} / F_{scaling}$$

Where $I_{revised}$ and $I_{raw}$ are the revised and raw intensity for the detected peak respectively and $F_{scaling}$ is the scaling factor at the peak mass.

The detected peak lists are then screened for side peaks (contaminants and side products) such as salt adduct peaks, matrix adduct peaks, doubly charged peaks and abortive cycling peaks. Peaks explained by only one type of side peak are pooled and the average ratios of these peaks to their parent peaks are calculated. The ratios are then used to adjust peak intensities for other peaks that match both side peaks and reference peaks or new peaks:

$$I_{adj} = I_{rev} - R_{side} * I_{sideparent}$$

Where $I_{adj}$ and $I_{rev}$ are the adjusted and revised intensity for a peak respectively, $R_{side}$ is the ratio to the parent peak and $I_{sideparent}$ is the revised intensity of the parent peak for the side peak. If the adjusted intensity is below the minimum peak intensity, that peak is assigned to side peak and excluded from score calculation. The adjusted intensities for detected peaks are used in all the scoring during identification and confidence evaluation described below.

It has been observed that peaks with different compositions, e.g., nucleic acid compositions, have different intensities in spectra obtained in MALDI-TOF MS or alternative spectrometers, particularly for T-rich fragments of C-cleavage reaction if the RNAse A cleavage is applied. It can be that the intensity of a T-rich main peak is lower than that of an adduct peak for a non-T-rich peak. To better identify and evaluate peaks, an empirical relationship between adjusted peak intensity and base composition for C-cleavage products has been built. Similar relationship can also be built for products from other cleavage reaction, e.g., T-cleavage using RNAse A.

For all the data in a training set, peak intensities were first scaled as described in previous section to remove mass dependency. Peaks with the same nucleic acid composition were averaged. Because the accuracy of mass dependent peak intensity scaling relies on the adjusted reference peak intensities and the adjusted peak intensity calculations depend on mass dependent peak intensity scaling, a few cycles of modeling have to be performed to reach convergence. For .shorter nucleic acid compositions up to 10 nucleotides, the average values from all the training sets were used for each nucleic acid composition. For example, the expected intensity is 1.29 for A2CG2, 0.69 for ACG2T, 0.36 for CG2T2, but only 0.09 for CT4.

For nucleic acid compositions above 10 nucleotides, empirical models of intensity as function of %T and %A were used:

If %T is above 0.75, adjustedIntensity = 0.17;
Else adjustedIntensity = %T * (-0.5545 * %T - 1.143) + 1.341

When %T is less than 0.37, adjusted intensity is modulated further by %A:

$$\text{adjustedIntensity} = 1.098 * \exp\{- [(\%A - 0.6786 )/ 1.139]^2\}$$

The adjusted peak intensities were then used in peak detection, peak scaling, score calculation and peak type evaluation.

Once detected peaks for a sample are extracted from the spectra, the next step is to identify the reference or references with the best matching peak patterns. This is done by assigning an overall score for each sequence. During identification process, the overall score is calculated by combining three different scores: the bitmap score, discriminating feature matching score and distance score.

The bitmap score ($score_{bitmap}$) is calculated by comparing all reference peaks generated in simulation with detected peaks. For each reference peak, if there is no matching detected peak, the score is zero. Otherwise, the score is calculated by evaluating the intensity ratio of detected versus reference. For the ratio in 0.7 - 1.5, a score of 1.0 is assigned; 0.5 - 0.7 or 1.5 - 2.0, a score of 0.75 is assigned; 0.3 - 0.5 or 2.0 - 3.0, a score of 0.5 is assigned; 0.2 - 0.3 or above 3.0, a score of 0.25 is assigned; 0.1 to 0.2, a score of 0.1 is assigned; the score is 0 if the ratio is less than 0.1. The bitmap score is then calculated by averaging scores for all the reference peaks weighted by reference intensities and mass scaling factors described earlier. Peaks having T-rich nucleic acid composition or peaks at low mass and high mass range which sometimes are not detected due to low intensities will have less impact on the score.

The discriminating feature matching score (score$_{disc}$) is calculated in a similar fashion except evaluating only a subset of peaks that can discriminate one reference from another or one set of references from another set. It is more sensitive in picking up minor differences between the peak intensities crucial for differentiation of different references. The summed intensity of all the discriminating peaks are called discriminating power. The higher the discriminating power, the higher the discriminating feature matching score will contribute to the overall score.

The distance score (score$_{dist}$) is calculated based on Euclidian distance of the sample vectors from the detected peaks to all reference vectors. It includes contributions from all detected peaks which are expected for the set of references regardless of whether they are present in a particular reference. Once the distances of a sample to all the references are calculated, a base score is calculated:

$$baseScore = \exp[\,-\,(minDist + offset)\,/\,200.0]$$

where minDist is the minimum distance and offset is the distance offset that takes account of number of top match sequences selected, number of good reactions, e.g., cleavage reactions, and additional peaks not in the bitmap vector. Then the distance score is calculated:

$$score_{dist} = baseScore * (1\,/\,\{1 + \exp[(dist - minDist)\,/\,(offset + aveDist - minDist) - 1] * 3\,\})$$

where dist is the Euclidian distance of the sample to the reference and aveDist is the average distance for the selected top match reference sequences.

The overall scores are the dynamic combination of all three scores:

$$overallScore = [Score_{bitmap} * (1 - w_{disc}) + score_{disc}] * (1 - w_{dist}) + score_{dist} * w_{dist}$$

where $w_{disc}$ is the weight for discriminating feature score ranging from 0 to 0.5 or alternative value depending on discriminating power and $w_{dist}$ is the weight for the distance score also ranging from 0 to 0.3 or alternative value depending on peak pattern matching.

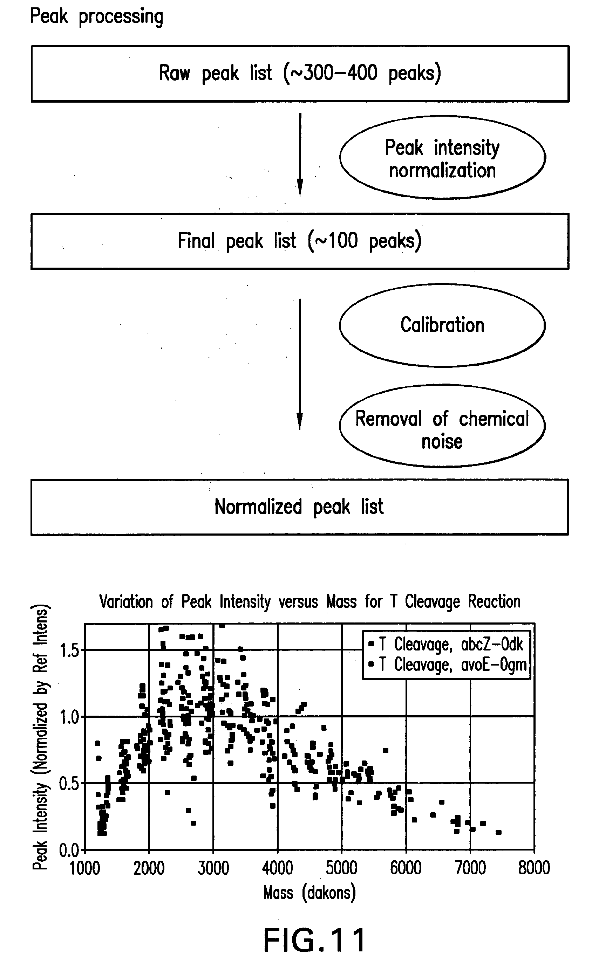

During identification, all the references are sorted by the overall scores and a portion of the top sequences are selected. The subset of sequences is then used to refine the intensities of the detected peak lists. The overall score is calculated again for this subset of sequences. This process continues until one sequence or several sequences with close scores that are considerably better than the rest are found for each sample, and they are selected as the top match or matches, as illustrated in Figure 11.

After the best matching reference or references are found, detected peak lists are reevaluated against the top matching reference for best explanation of each peak. Overall spectrum qualities are also calculated for each sample which will have major contribution from Q$_{spec}$, but also has contributions from other properties such as peak intensity matching, additional peaks, unknown peaks and amount of salt adduct peaks.

Peak pattern identity (PPIdentity) score is evaluated by calculating the ratio of summed intensity of matched peaks over the summed total intensity where the summed intensity of matched peaks is the summed intensity of all reference peaks for the particular reference sequence subtracted by those of the missing peaks and silent missing peaks (detected peaks much weaker than reference peaks), and the summed total intensity is the summed intensity of all reference peaks for the particular reference in addition to those of additional peaks and silent additional peaks (detected peaks expected but much stronger than reference peaks). This score ignores minor differences between peak intensities but includes contributions from new peaks that are not expected for the reference.

The final score is the average of the PPIdentity score and the bitmap score and is calculated for all the references in the set.

Another important parameter evaluated for each sample against all the references is the adjusted peak change, which is the summed intensity of missing peaks and additional peaks weighted by the overall spectrum qualities and adjusted by unknown peaks and adduct peaks. Large adjusted peak change is a good indicator that the sample has variation from the reference.

The next step in the process is to compare detected peaks and reference peaks for the top matching reference sequence to find whether there are any pattern or sequence variations using, e.g., SNP discovery algorithm (US 2005/0112590) which will be discussed in the next section. Once variations are detected, missing peaks and additional peaks are re-evaluated. The final score and adjusted peak change are recalculated for the top matching reference sequence.

The final step in the comparative sequence analysis process is to evaluate the confidence of the identification results, i.e. how well the selected reference matches the sample and whether there are additional variations. The common approach is to calculate the probability value (p-value) which estimates the probability of a random sequence having better score than the selected one. However, to get reasonably accurate p-value, the sampling space has to be so large that it would be computationally prohibitive to do. Thus the approach described here is based on empirical model with the assumption that at least one sample match the top match reference sequence (with or without resolved variations). The model was built based on training data sets. First, identify all the samples in the training sets. Then for each sample, simulate all mutations in the top match reference and calculate the final scores and adjusted peak changes for all the mutated sequences. For a single base change mutation, all the possible mutations from the top matching reference can be simulated. For two or more mutations, a random sampling of 5000 - 20000 can be performed. Finally the density distributions for scores and adjusted peak changes are plotted. For all the samples simulated, both density distributions for scores and adjusted peak changes can be described by Gaussian distribution. Alternatively other distributions such as Poisson distribution can also be used to describe the density distribution. For actual scores and adjusted peak changes, density contributions from two or more mutations are usually 10 to 100 folds lower than those from single mutations and they can be ignored. Thus the density distributions for scores and adjusted peak changes modeled from single mutations are used to estimate the probability of additional mutations. Both can be approximated by the function:

$$\phi(x) = \frac{1}{\sqrt{2\pi}} e^{-\frac{(x-x_0)^2}{2\sigma^2}}$$

Where $x_0$ is the center and $\sigma$ is the standard deviation of Gaussian distribution.

For each analysis, $x_0$ and $\sigma$ for either score or adjusted peak change are determined by empirical models. After selecting the best matching reference sequence and applying mutation detection, a preliminary confidence based on the score and adjusted peak change for each sample is evaluated. Samples showing low chance of mutations are collected and the modes for score ($mode_{score}$) and peak change ($mode_{peakChange}$) are calculated.

For the score, the initial sigma ($\sigma_{score}$) is set to standard value of 0.02, and an initial cutoff ($cutoff_{score}$) is set to 1 - 1.5 * $\sigma_{score}$ minus one half of the smaller of sigma and (1 - $mode_{score}$). Then the sigma and $cutoff_{score}$ is adjusted in accord to $mode_{score}$ as follows:

$$modeToCutoff = mode_{score} - cutoff_{score}$$

If modeToCutoff < 2 * $\sigma_{score}$ Then

$$cutoff_{score}\ -= modeToCutoff / 2$$

If modeToCutoff > $\sigma_{score}$ Then $\sigma_{score}\ += (modeToCutoff - \sigma_{score}) / 4$

Else

$$cutoff_{score}\ -= \sigma_{score}$$

$$\sigma_{score}\ += \sigma_{score}\ / 4 + (modeToCutoff - 2*\ \sigma_{score}) / 6$$

Endif

Finally the center of the density distribution is obtained by shifting the cutoff by 2 sigmas:

$$x_{0score} = cutoff_{score} - 2 * \sigma_{score}$$

For adjusted peak change, the initial sigma ($\sigma_{peakChange}$) is set to standard value of 0.4, and an initial cutoff (cutoff$_{peakchange}$) is set to $\sigma_{peakChange}$ plus one half of the smaller of sigma and the minimum peak change. Then the sigma and cutoff$_{peakchange}$ are adjusted by mode$_{peakChange}$ as follows:

$$\text{modeToCutoff} = \text{mode}_{peakChange} - \text{cutoff}_{peakchange}$$

If modeToCutoff < 2 * $\sigma_{peakChange}$ Then

  cutoff$_{peakchange}$ += modeToCutoff / 2

  If modeToCutoff > $\sigma_{peakChange}$ Then $\sigma_{peakChange}$ += (modeToCutoff - $\sigma_{peakChange}$) / 4

Else

  cutoff$_{peakchange}$ += $\sigma_{peakChange}$

  $\sigma_{peakChange}$ += $\sigma_{peakChange}$ / 4 + (modeToCutoff – 2* $\sigma_{peakChange}$) / 6

Endif

Finally the center of the density distribution for the adjusted peak change is obtained by shifting the cutoff by 2 sigmas:

$$x_{0peakChange} = \text{cutoff}_{peakchange} + 2 * \sigma_{peakChange}$$

The probability contributed from the score and peak change can be calculated by summing the appropriate density:

$$P_{score} = \int_{s0}^{1} \Phi_{score}(x)$$

$$P_{peakChange} = \int_{0}^{pc0} \Phi_{peakChange}(x)$$

Where s0 is the final score and pc0 is the adjusted peak change for a sample.
The final overall mutation probability is the combination of the two:

$$P_{mutation} = 1.0 - (1.0 - P_{score}) * (1.0 - P_{peakChange})$$

$P_{mutation}$ is an estimation of the probability for the sample having additional variations from the top matching reference.
Similar empirical model or models can also be built if alternative density distribution, e.g. Poisson distribution, is used.
Once all the samples are identified and finalized, they can be clustered based on the detected peak patterns. The distance matrix can be calculated based on the presence and absence of peaks similar to that used for restriction sites (Felsenstein, J. 1992. Phylogenies from restriction sites, a maximum likelihood approach. Evolution 46: 159-173). It can also be calculated using Euclidean distance, taking peak intensities into consideration. The algorithm used to calculate Euclidean distance is the same as the one used to calculate distance from reference peak patterns:

$$D_{i,j} = \sum_r \sum_k [(V_{i,r,k} - V_{i,r,k})^{\wedge} 3 / (V_{i,r,k} + V_{i,r,k})]$$

Where $V_{i,r,k}$ is the revised intensity for sample i, reaction r and peak k, $V_{j,r,k}$ is the revised intensity for sample j, reaction r and peak k, $\sum_k$ is summation over all peaks in reaction r, $\sum_r$ is summation over all reactions, and $D_{i,j}$ is the distance between sample i and j.

The sample distance matrix can be used to cluster samples even under experimental conditions where samples do not always match the known references. This detected peak based clustering provides a fast and efficient way to group samples. Mixture samples can also be clustered without having to resolve the individual sequences.

Detection of Biomolecule Sequence Variations

Comparative sequence analysis processes described herein may include determining whether there are sequence alterations in a given sequence (e.g., a reference sequence or sample sequence). Techniques that increase the speed with which mutations, polymorphisms or other sequence variations can be detected in a target sequence, relative to a reference sequence, are known to the person of ordinary skill in the art. Determining whether there are sequence alternations in a given sequence sometimes is performed after sequence determination methods described above have been performed. Sequence determination methods and sequence alternation determination methods may be provided together.

One approach is to reduce the number of possible sequence variations of a given target sequence whose cleavage patterns are simulated and compared against the actual cleavage pattern generated by cleavage of the target sequence. In the methods provided herein, an algorithm is used to output only those sequence variation candidates that are most likely to have generated the actual cleavage spectrum of the target sequence. A second algorithm then simulates only this subset of sequence variation candidates for comparison against the actual target sequence cleavage spectrum. Thus, the number of sequence variations for simulation analyses is drastically reduced.

In a first step, the cleavage products corresponding to difference in signals between a target sequence and a reference sequence that are absolute (presence or absence of a signal in the target spectrum relative to a reference spectrum) or quantitative (differences in signal intensities or signal to noise ratios) differences obtained by actual cleavage of the target sequence relative to actual or simulated cleavage of the reference sequence under the same conditions are identified, and the masses of these "different" target nucleic acid cleavage products are determined. Once the masses of the different cleavage products are determined, one or more nucleic acid base compositions (compomers) are identified whose masses differ from the actual measured mass of each different cleavage product by a value that is less than or equal to a sufficiently small mass difference. These compomers are called witness compomers. The value of the sufficiently small mass difference is determined by parameters such as the peak separation between cleavage products whose masses differ by a single nucleotide equivalent in type or length, and the absolute resolution of the mass spectrometer. Cleavage reactions specific for one or more of the four nucleic acid bases (A, G, C, T or U for RNA, or modifications thereof, or amino acids or modifications thereof for proteins) can be used to generate data sets comprising the possible witness compomers for each specifically cleaved product that nears or equals the measured mass of each different cleavage product by a value that is less than or equal to a sufficiently small mass difference.

Such techniques can reconstruct the target sequence variations from possible witness compomers corresponding to differences between the cleavage products of the target nucleic acid relative to the reference nucleic acid.

*Algorithm 1: Find Sequence Variation Candidates*

This is the basic technique used to analyze the results from one or more specific cleavage reactions of a target nucleic acid sequence. The first step identifies all possible compomers whose masses differ by a value that is less than or equal to a sufficiently small mass difference from the actual mass of each different fragment generated in the target nucleic acid cleavage reaction relative to the same reference nucleic acid cleavage reaction. These compomers are the 'compomer witnesses'. For example, suppose a different fragment peak is detected at 2501.3 Da. The only natural compomer having a mass within, e.g., a .+-.2 Da interval of the peak mass is A.sub.1C.sub.4G.sub.2T.sub.1 at 2502.6 Da. In the case of cleavage reactions that do not remove the recognized base (herein, T) at the cleavage site, (for example, UDG will remove the cleaved base, but RNAse A will not) the recognition base is subtracted, resulting in the compomer A.sub.1C.sub.4G.sub.2. Every compomer detected in this fashion is called a compomer witness.

The basic technique then determines all compomers that can be transformed into each compomer witness c' with at most k mutations, polymorphisms, or other sequence variations including, but not limited to, sequence variations between organisms. The value of k, the sequence variation order, is predefined by the user and is dependent on a number of parameters including, but not limited to, the expected type and number of sequence variations between a reference sequence and the target sequence, e.g., whether the sequence variation is a single base or multiple bases, whether sequence variations are present at one location or at more than one location on the target sequence relative to the reference sequence, or whether the sequence variations interact or do not interact with each in the target sequence. For example, for the detection of SNPs, the value of k is usually, although not necessarily, 1 or 2. As another example, for the detection of mutations and in resequencing, the value of k is usually, although not necessarily, 3 or higher.

A set of bounded compomers are constructed, which refers to the set of all compomers c that correspond to the set of subsequences of a reference sequence, with a boundary b that indicates whether or not cleavage sites are present at the two ends of each subsequence. The set of bounded compomers can be compared against possible compomer witnesses to construct all possible sequence variations of a target sequence relative to a reference sequence. Using the constructed pairs of compomer witnesses and bounded compomers, the algorithm then constructs all sequence variation candidates that would lead to the obtained differences in the cleavage pattern of a target sequence relative to a reference sequence under the same cleavage conditions.

The determination of sequence variation candidates significantly reduces the sample set of sequence variations that are analyzed to determine the actual sequence variations in the target sequence, relative to the previous approach of simulating the cleavage pattern of every possible sequence that is a variation of a reference sequence, and comparing the simulated patterns with the actual cleavage pattern of the target nucleic acid sequence.

Two functions d.sub.+, d.sub.- are defined as:

d.sub.+(c):=.SIGMA..sub.b in {A,C,G,T}c(b) for those b with c(b)>0
d.sub.-(c):=.SIGMA..sub.b in {A,C,G,T}c(b) for those b with c(b)<0

and a function d(c) is defined as d(c):=max {d.sub.+(c), d.sub.-(c)} and d(c,c'):=d(c-c'). This is a metric function that provides a lower bound for the number of insertions, deletions, substitutions and other sequence variations that are needed to mutate one fragment, e.g., a reference fragment into another, e.g., a target fragment. If f,f are fragments and c,c' are the corresponding compomers, then we need at least d(c,c') sequence variations to transform f into f.

A substring (fragment) of the string s (full length sequence) is denoted s[i,j], where i,j are the start and end positions of the substring satisfying 1.ltoreq.i.ltoreq.j.ltoreq.length of s.

A compomer boundary or boundary is a subset of the set {L,R}. Possible values for b are { } (the empty set), {L}, {R}, {L,R}. For a boundary b, #b denotes the number of elements in b, that is, 0, 1, or 2. A bounded compomer (c, b) contains a compomer c and a boundary b. Bounded compomers refers to the set of all compomers c that correspond to the set of subsequences of a reference sequence, with a boundary that indicates whether or not cleavage sites are present at the two ends of each subsequence. The set of bounded compomers can be compared against possible compomer witnesses to construct all possible sequence variations of a target sequence relative to a reference sequence.

The distance between a compomer c' and a bounded compomer (c,b) is defined as:

$$D(c',c,b):=d(c',c)+\#b$$

The function D(c',c,b) measures the minimum number of sequence variations relative to a reference sequence that is needed to generate the compomer witness c'.

Given a specific cleavage reaction of a base, amino acid, or other feature X recognized by the cleavage reagent in a string s, then the boundary b[i,j] of the substring s[i,j] or the corresponding compomer c[i,j] refers to a set of markers indicating whether cleavage of string s does not take place immediately outside the substring s[i,j]. Possible markers are L, indicating whether "s is not cleaved directly before i", and R, indicating whether "s is not cleaved directly after j". Thus, b[i,j] is a subset of the set {L,R} that contains L if and only if X is present at position i-1 of the string s, and contains R if and only if X is present at position j+1 of the string s. #b denotes the number of elements in the set b, which can be 0, 1, or 2, depending on whether the substring s[i,j] is specifically cleaved at both immediately flanking positions (i.e., at positions i-1 and j+1), at one immediately flanking position (i.e., at either position i-1 or j+1) or at no immediately flanking position (i.e., at neither position i-1 nor j+1). b[i,j] is a subset of the set {L,R} and denotes the boundary of s[i,j] as defined by the following:

b[i,j]:={L,R} if s is neither cleaved directly before i nor after j
b[i,j]:={R} if s is cleaved directly before i, but not after j
b[i,j]:={L} if s is cleaved directly after j, but not before i
b[i,j]:={ } if s is cleaved directly before i and after j
#b[i,j] denotes the number of elements of the set b[i,j].

The set of all bounded compomers of s is defined as:

C:={(c[i,j],b[i,j]):1.ltoreq.i.ltoreq.j.ltoreq.length of s}, where the compomer corresponding to the substring s[i,j] of s is denoted c[i,j].

If there is a sequence variation of a target sequence containing at most k mutations, polymorphisms, or other sequence variations, including, but not limited to, sequence variations between organisms, insertions, deletions and substitutions (usually, for a nucleic acid, k would represent the number of single base variations in a sequence variation), and if c' is a compomer witness of this sequence variation, then there exists a bounded compomer (c,b) in C such that D(c',c, b).Itoreq.k. In other words, of every sequence variation of a target sequence containing at most k mutations, polymorphisms, or other sequence variations, including, but not limited to, sequence variations between organisms, insertions, deletions and substitutions (usually, for a nucleic acid, k would represent the number of single base variations in a sequence variation) that leads to a different fragment corresponding to a signal that is different in the target sequence relative to the reference sequence and that corresponds to a compomer witness c', there is a bounded compomer (c, b) in C with the property D(c',c,b).Itoreq.k. Thus, the number of fragments under consideration can be reduced to just those which contain at most k cleavage points:

C.sub.k:={(c[i,j], b[i,j]):1.Itoreq.i.Itoreq.j.Itoreq.length of s, and ord[i,j]+#b[i,j].Itoreq.k}, where ord[i,j] is the number of times the fragment s[i,j] will be cleaved.

*Algorithm 1: Find Sequence Variation Candidates*

INPUT: Reference sequences (or more than one reference sequence), description of cleavage reaction, whether modified nucleotides or amino acids are incorporated into all or part of the sequence, list of peaks corresponding to different cleavage products (either missing signals or additional signals or qualitative differences in the target sequence relative to the reference sequence(s)), maximal sequence variation order k.

OUTPUT:List of sequence variations that contain at most k insertions, deletions, and substitutions, and that have a different peak as a witness.

Given the reference sequence s and the specific cleavage reaction, compute all bounded compomers (c[i,j],b [i,j]) in C.sub.k, and store them together with the indices i,j. This is usually independent of the samples containing target sequences being analyzed, and is usually done once.

For every different peak, find all compomers with mass close to the peak mass by a sufficiently small mass difference, and store them as compomer witnesses.

For every compomer witness c', find all bounded compomers (c,b) in C.sub.k such that D(c',c,b).Itoreq.k.

For every such bounded compomer (c,b) with indices i,j compute all sequence variations of s to a new reference sequence s' using at most k insertions, deletions, and substitutions such that:

if L in b, then we insert/substitute to a cleaved base or amino acid directly before position i;
if R in b, then we insert/substitute to a cleaved base or amino acid directly after position j;

Use at most k-#b insertions, deletions, and insertions that transform the fragment f=s[i,j] with corresponding compomer c into some fragment f of s' with corresponding compomer c'.

Output every such sequence variation.

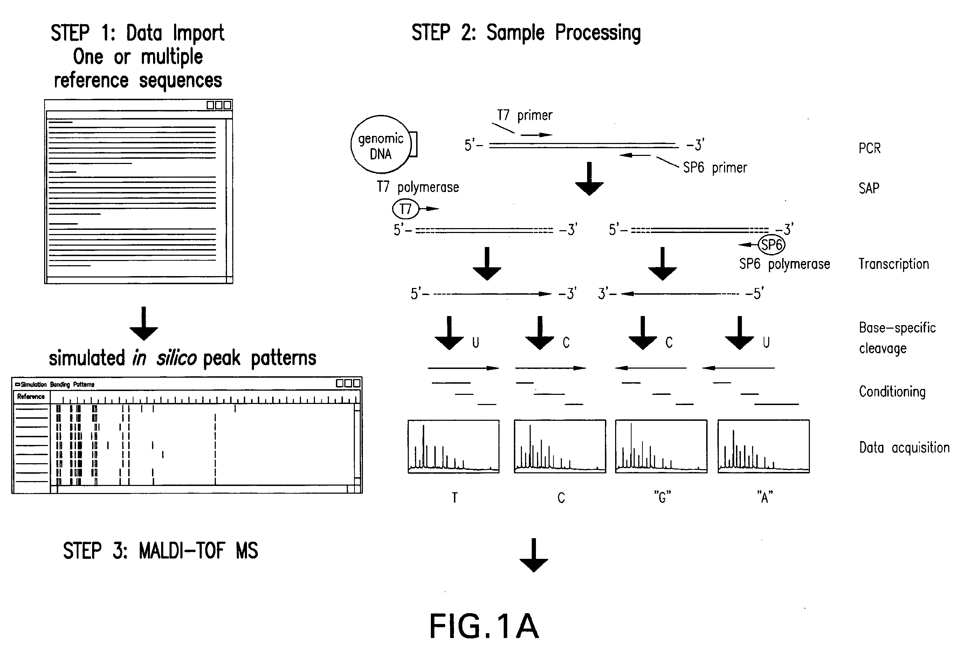

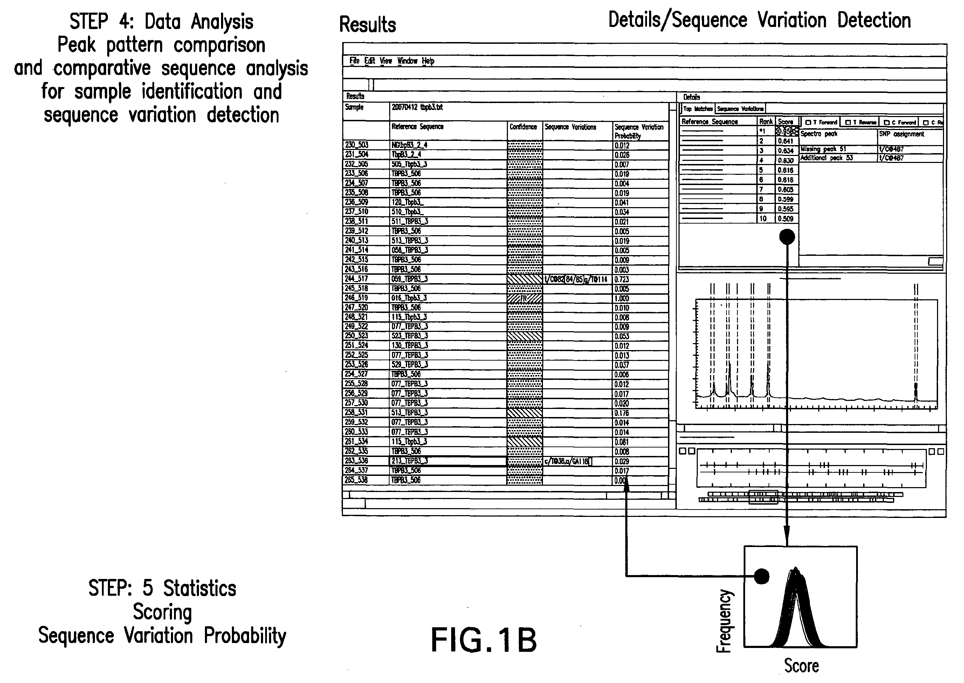

Figure 1 in US2005/0112590 is a flow diagram that illustrates operations performed with a computer system that is engaged in data analysis to determine those sequence variation candidates that satisfy the criteria described above. In the first operation, indicated by box 102, the target molecule is cleaved into fragments using one or more cleavage reagents, using techniques that are well-known to those of skill in the art and described herein. In the next operation, represented by box 104, the reference molecule is actually or virtually (by simulation) cleaved into cleavage products using the same one or more cleavage reagents. From the cleavage products produced by the cleavage reactions, data, such as mass spectra for the target and reference sequences, are produced. The produced data can be used to extract a list of peaks of the sequence data corresponding to fragments that represent differences between the target sequence and the reference sequence.

The next operation is to determine a reduced set of sequence variation candidates based on the identified different fragments. This operation is depicted by box 106. The sequence variation candidates are then scored (box 108), and the sequence variation candidates corresponding to the actual sequence variations in the target sequence are identified based on the value of the score. Usually, in a set of samples of target sequences, the highest score represents the most likely sequence variation in the target molecule, but other rules for selection can also be used, such as detecting a positive score, when a single target sequence is present.

Data produced from cleavage reactions comprises the output of conventional laboratory equipment for the analysis of molecular information. Such output is readily available in a variety of digital data formats, such as plain text or according to word processing formats or according to proprietary computer data representations.

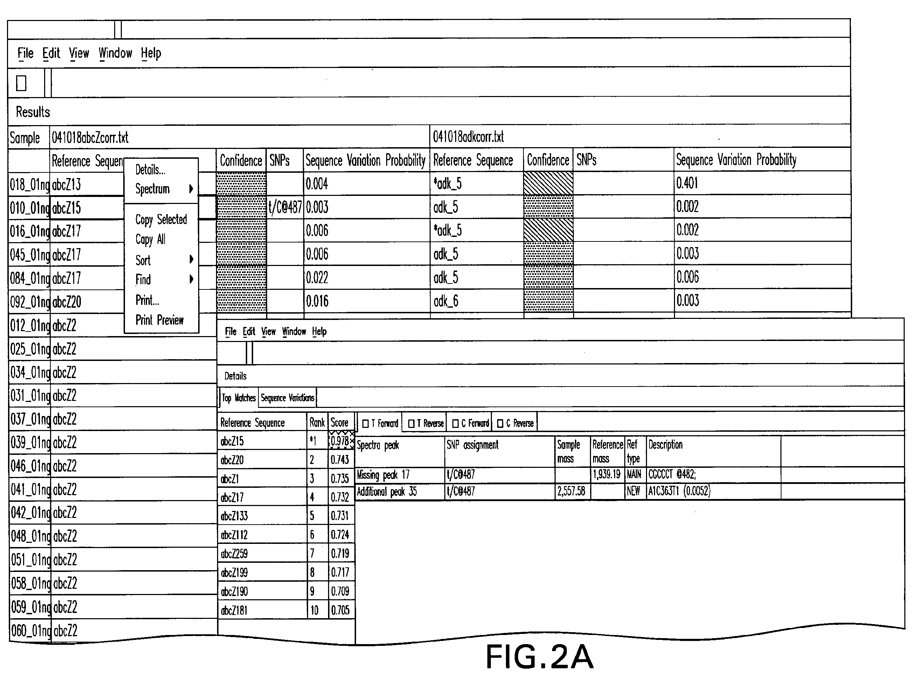

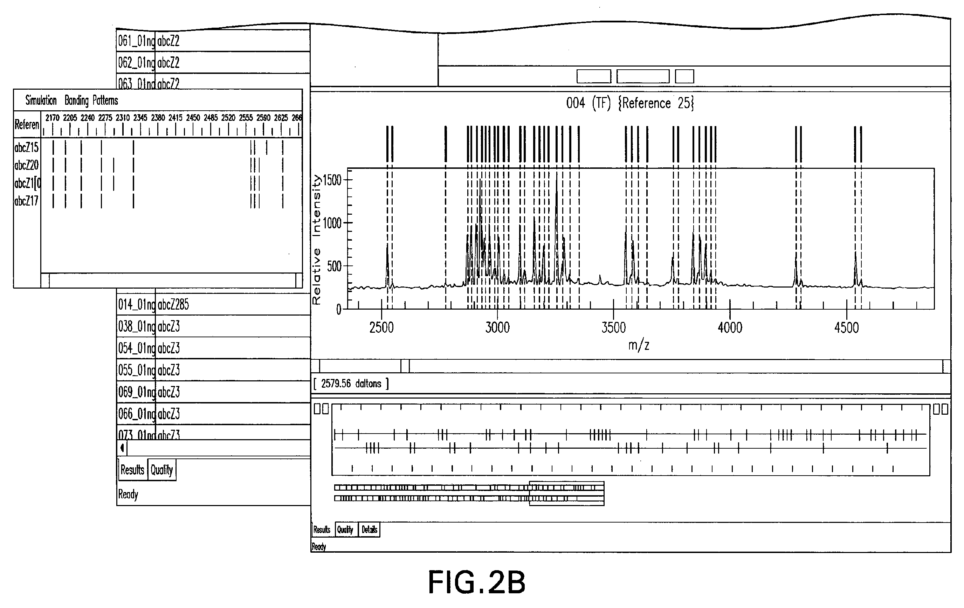

As described above, the process of determining a reduced set of sequence variation candidates based on the identified different fragments is preferably carried out with a programmed computer. Figure 2 in US2005/0112590 is a flow diagram that illustrates the operations executed by a computer system to determine the reduced set of sequence variation candidates.

In the first operation, represented by box 202, the reaction data described above is processed to compute all bounded compomers (c[i,j],b[i,j]) in C.sub.k, and stored together with the indices i,j, in accordance with the reference sequence s and the specific cleavage reaction data described above. The next operation, indicated by box 204, is to find, for every different peak, all compomers with mass that differs from the peak mass by a sufficiently small mass difference that is reasonably close to the peak mass. The value of the sufficiently small mass difference is determined by parameters that include, but are not limited to, the peak separation between cleavage products whose masses differ by a single nucleotide in type or length, and the absolute resolution of the mass spectrometer. These compomers are stored as compomer witnesses. After the compomer witnesses are identified, the next operation is to find, for every compomer witness c' identified from box 204, all bounded compomers (c,b) in C.sub.k such that D(c',c,b).Itoreq.k. The bounded compomer operation is represented by box 206. Box 208 represents the operation that involves the computation of all sequence variations of s to a new reference sequence s' using at most k insertions, deletions, and substitutions such that:

if L in b, then we insert/substitute to a cleaved base or amino acid directly before position i;
if R in b, then we insert/substitute to a cleaved base or amino acid directly after position j;
Use at most k-#b insertions, deletions, and insertions that transform the fragment f=s[i,j] with corresponding compomer c into some fragment f of s' with corresponding compomer c'.

The last operation, indicated by box 210, is to produce every such sequence variation computed from box 208 as the system output. Here, d(c,c') is the function as defined herein that determines the minimum number of sequence variations, polymorphisms or mutations (insertions, deletions, substitutions) that are needed to convert c to c', where c is a compomer of a fragment of the reference molecule and c' is the compomer of the target molecule resulting from mutation of the c fragment.

A substring (fragment) of the string s (full length sequence) is denoted s[i,j], where i,j are the start and end positions of the substring. Given a specific cleavage reaction of a base, amino acid, or other feature X recognized by the cleavage reagent in a string s, then the boundary b[i,j] of the substring s[i,j] or the corresponding compomer c[i,j] refers to a set of markers indicating whether cleavage of string s does not take place immediately outside the substring s[i,j]. Possible markers are L, indicating whether "s is not cleaved directly before i", and R, indicating whether "s is not cleaved directly after j". Thus, b[i,j] is a subset of the set {L,R} that contains L if and only if X is present at position i-1 of the string s, and contains R if and only if X is present at position j+1 of the string s. #b denotes the number of elements in the set b, which can be 0, 1, or 2, depending on whether the substring s[i,j] is specifically cleaved at both immediately flanking positions (i.e., at positions i-1 and j+1), at one immediately flanking position (i.e., at either position i-1 or j+1) or at no immediately flanking position (i.e., at neither position i-1 nor j+1). b[i,j] is a subset of the set {L,R} and denotes the boundary of s[i,j] as defined by the following:

b[i,j]:={L,R} if s is neither cleaved directly before i nor after j
b[i,j]:={R} if s is cleaved directly before i, but not after j
b[i,j]:={L} if s is cleaved directly after j, but not before i
b[i,j]:={} if s is cleaved directly before i and after j
#b[i,j] denotes the number of elements of the set b[i,j].
ord[i,j] refers to the number of times s[i,j] will be cleaved in a particular cleavage reaction; i.e., the number of cut strings present in s[i,j].
D(c',c,b):=d(c,c')+#b refers to the distance between compomer'c and bounded compomer (c,b)'; i.e., the total minimum number of changes needed to create the fragment with compomer c' from the fragment with compomer c, including sequence variations of the boundaries of substring s[i,j] into cut strings, if necessary.
C:={(c[i,j],b[i,j]):1.Itoreq.i.Itoreq.j.Itoreq.length of s} refers to the set of all bounded compomers within the string s; i.e., for all possible substrings s[i,j], find the bounded compomer (c[i,j],b[i,j]) and these will belong to the set C.
C.sub.k:={(c[i,j], b[i,j]):1.Itoreq.i.Itoreq.j.Itoreq.length of s, and ord[i,j]+#b[i,j].Itoreq.k} is the same as C above, except that compomers for substrings containing more than k number of sequence variations of the cut string will be excluded from the set, i.e., C.sub.k is a subset of C. It can be shown that if there is a sequence variation containing at most k insertions, deletions, and substitutions, and if c' is a compomer corresponding to a peak witness of this sequence variation, then there exists (c,b) in C.sub.k such that D(c',c,b).Itoreq.k. The algorithm is based on this reduced set of possible sequence variations corresponding to compomer witnesses.

Every sequence variation constructed in this fashion will lead to the creation of at least one different peak out of the list of input different peaks. Further, every sequence variation that contains at most k insertions, deletions, and insertions that was not constructed by the algorithm is either the superset of the union of one or more sequence variations that were constructed, or does not lead to the creation of any different peaks out of the list of different peaks that served as input for the algorithm.

Algorithm 1 can be repeated for more than one specific cleavage reagent generating more than one target cleavage pattern relative to a reference cleavage pattern, and more than one list of compomer witnesses. In one embodiment, the final output contains the set of sequence variation candidates that is the union of the sets of sequence variation candidates for each cleavage reaction.

*Algorithm 2*

A second algorithm can be used to generate a simulated spectrum for each computed output sequence variation candidate. The simulated spectrum for each sequence variation candidate is scored, using a third (scoring) algorithm, described below, against the actual target spectrum, applying the reference spectrum for the reference sequence. The value of the scores (the higher the score, the better the match, with the highest score usually being the sequence variation that is most likely to be present) can then be used to determine the sequence variation candidate that is actually present in the target nucleic acid sequence.

Provided below is an exemplary algorithm where the sequence variations to be detected are SNPs. Algorithms for detecting other types of sequence variations, including homozygous or heterozygous allelic variations, can be implemented in a similar fashion.

a) For each cleavage reaction, a simulated spectrum is generated for a given sequence variation candidate from Algorithm 1.

b) The simulated spectrum is scored against the actual target spectrum.

c) The scores from all cleavage reactions, preferably complementary cleavage reactions, for the given target sequence are added. The use of more than one specific cleavage reaction improves the accuracy with which a particular sequence variation can be identified.

d) After all scores have been calculated for all sequence variations, sequence variations are sorted according to their score.

*Algorithm* 2: *Find SNPs*

INPUT: Reference sequences, one or more cleavage reaction, for every cleavage reaction a simulated or actual reference cleavage spectrum, for every cleavage reaction a list of peaks found in the corresponding sample spectrum, maximal sequence variation order k.

OUTPUT: List of all SNP candidates corresponding to sequence variations containing at most k insertions, deletions, and substitutions, and that have a different peak as a witness; and for every such SNP candidate, a score.

For every cleavage reaction, extract the list of different peaks by comparing the sample spectrum with the simulated reference spectrum.

For every cleavage reaction, use FINDSEQUENCEVARIATIONCANDIDATES (Algorithm 1) with input s, the current cleavage reaction, the corresponding list of different peaks, and k.

Combine the lists of sequence variation candidates returned by FINDSEQUENCEVARIATIONCANDIDATES into a single list, removing duplicates.

For every sequence variation candidate:

Apply the sequence variation candidate, resulting in a sequence s'.

For every cleavage reaction, simulate the reference spectrum of s' under the given cleavage reaction.

Use SCORESNP (Algorithm 3) with the peak lists corresponding to the spectra of s,s' as well as the peak list for the measured sample spectrum as input, to calculate scores (heterozygous and homozygous) of this sequence variation (or SNP) candidate for the cleavage reaction.

Add up the scores of all cleavage reactions, keeping separate scores for heterozygous and homozygous variations.

Store a SNP candidate containing the sequence variation candidate plus its scores; the overall score of the SNP candidate is the maximum of its heterozygous and homozygous scores.

Sort the SNP candidates with respect to their scores.

Output the SNP candidates together with their scores.

An exemplary implementation of a scoring algorithm, SCORESNP, is as follows:

*Algorithm 3: Score SNP*

INPUT: Peak lists corresponding to reference sequence s (denoted L), modified reference sequence s' (denoted L'), and sample spectrum (denoted L.sub.s).

OUTPUT: Heterozygous score, homozygous score.

Set both scores to 0.

Compute a list of intensity changes (denoted L.sub..DELTA.) that includes those peaks in the lists corresponding to s,s' that show differences:

If a peak is present in L but not in L', add this peak to L.sub..DELTA. and mark it as wild-type.

If a peak is present in L' but not in L, add this peak to L.sub..DELTA. and mark it as mutant-type.

If a peak has different expected intensities in L and L', add this peak to L.sub..DELTA. together with the expected intensity change from L to L'.

For every peak in L.sub..DELTA. marked as mutant-type that is also found in L.sub.s, add +1 to both scores.

For every peak in L.sub..DELTA. marked as mutant-type that is not found in L.sub.s, add -1 to both scores.

For every peak in L.sub..DELTA. marked as wild-type that is not found in L.sub.s, add +1 to the homozygous score.

For every peak in L.sub..DELTA. marked as wild-type that is also found in L.sub.s, add -1 to the homozygous score.

Output both scores.

Other implementations of the scoring function will be obvious to those of skill in the art. For example, one implementation would make use of peaks that are not differentiated as either mutant or wild-type. Another implementation might, in addition or as a separate feature, take into account intensities in L, L.sub..DELTA., and L.sub.s. Other exemplary parameters include using peaks designated as "wild-type" to modify the heterozygous score, or incorporation of a weighing function that is based on the confidence level in the actual (measured) target sequence cleavage spectrum. A preferred implementation can use a logarithmic likelihood approach to calculate the scores.

In one embodiment, instead of using the scores of potential SNPs output by Algorithm 2 directly, scores from more than one target sequence expected to contain or actually containing the same SNP can be joined. When more than one target sequence is analyzed simultaneously against the same reference sequence, instead of reporting the SNP score for each target sequence independently, the scores of all identical scored sequence variations for the different target sequences may be joined to calculate a joined score for the SNP. The joined score can be calculated by applying a function to the set of scores, which function may include, but is not limited to, the maximum of scores, the sum of scores, or a combination thereof.

After all SNP or other sequence variation candidates with their scores have been calculated, a threshold score can be determined to report only those SNPs or sequence variations that have a score that is equal to or higher than the threshold score (and, therefore, a reasonable chance of being real, i.e., of corresponding to the actual sequence variation in the target sequence). Generally, the sequence variation with the highest score will correspond to an actual sequence variation in the target sequence. Sequence variations that are accepted as being real can then be used to modify the initial reference peak list L. The modified peak list can then be used to re-evaluate (score) all other potential sequence variations or SNPs using the SCORESNP algorithm, or even search for new witnesses in the case of homozygous SNPs. This leads to an iterative process of SNP or other sequence variation detection. For example, in the iterative process of detecting more than one sequence variation in a target sequence, the sequence variation with the highest score is accepted as an actual sequence variation, and the signal or peak corresponding to this sequence variation is added to the reference fragment spectrum to generate an updated reference cleavage spectrum. All remaining sequence variation candidates are then scored against this updated reference fragment spectrum to output the sequence variation candidate with the next highest score. This second sequence variation candidate can also represent a second actual sequence variation in the target sequence. Therefore, the peak corresponding to the second sequence variation can be added to the reference fragment spectrum to generate a second updated reference spectrum against which a third sequence variation can be detected according to its score. This process of iteration can be repeated until no more sequence variation candidates representing actual sequence variations in the target sequence are Identified.

The presented approach can be applied to any type and number of cleavage reactions that are complete, including 2-, 11/2-, or 11/4-base cutters. This approach can applied to partial cleavage experiments.

This approach is not limited to SNP and mutation detection but can be applied to detect any type of sequence variation, including polymorphisms, mutations and sequencing errors.

Since the presented algorithms are capable of dealing with homogeneous samples, it will be apparent to one of skill in the art that their use can be extended to the analysis of heterozygous samples or sample mixtures. Such "sample mixtures" usually contain the sequence variation or mutation or polymorphism containing target nucleic acid at very low frequency, with a high excess of wild type sequence. For example, in tumors, the tumor-causing mutation is usually present in less than 5-10% of the nucleic acid present in the tumor sample, which is a heterogeneous mixture of more than one tissue type or cell type. Similarly, in a population of individuals, most polymorphisms with functional consequences that are determinative of, e.g., a disease state or predisposition to disease, occur at low allele frequencies of less than 5%. The methods provided herein can detect high frequency sequence variations or can be adapted to detect low frequency mutations, sequence variations, alleles or polymorphisms that are present in the range of less than about 5-10%.

Applications

1. Microbial Identification

Provided herein is a process or method for identifying genera, species, strains, clones or subtypes of microorganisms and viruses. The microorganism(s) and viruses are selected from a variety of organisms including, but not limited to, bacteria, fungi, protozoa, ciliates, and viruses. The microorganisms are not limited to a particular genus, species, strain, subtype or serotype or any other classification. The microorganisms and viruses can be identified by determining sequence variations in a target microorganism sequence relative to one or more reference sequences or samples. The reference sequence(s) can be obtained from, for example, other microorganisms from the same or different genus, species strain or serotype or any other classification, or from a host prokaryotic or eukaryotic organism or any mixed population.

Identification and typing of pathogens (e.g., bacterial or viral) is critical in the clinical management of infectious diseases. Precise identity of a microbe is used not only to differentiate a disease state from a healthy state, but is also fundamental to determining the source of the infection and its spread and whether and which antibiotics or other antimicrobial therapies are most suitable for treatment. In addition treatment can be monitored. Traditional methods of pathogen typing have used a variety of phenotypic features, including growth characteristics, color, cell or colony morphology, antibiotic susceptibility, staining, smell, serotyping and reactivity with specific antibodies to identify microbes (e.g., bacteria). All of these methods require culture of the suspected pathogen, which suffers from a number of serious shortcomings, including high material and labor costs, danger of worker exposure, false positives due to mishandling and false negatives due to low numbers of viable cells or due to the fastidious culture requirements of many pathogens. In addition, culture methods require a relatively long time to achieve diagnosis, and because of the potentially life-threatening nature of such infections, antimicrobial therapy is often started before the results can be obtained. Some organisms cannot be maintained in culture or exhibit prohibitively slow growth rates (e.g., up to 6-8 weeks for *Mycobacterium tuberculosis).*

In many cases, the pathogens are present in minor amounts and/or are very similar to the organisms that make up the normal flora, and can be indistinguishable from the innocuous strains by the methods cited above. In these cases, determination of the presence of the pathogenic strain can require the higher resolution afforded by the molecular typing methods provided herein. For example, PCR amplification of a target nucleic acid sequence followed by base-specific cleavage by specific cleavage (e.g., base-specific), followed by matrix-assisted laser desorption/ionization time-of-flight mass spectrometry, followed by screening for sequence variations as provided herein, allows reliable discrimination of sequences differing by only one nucleotide and combines the discriminatory power of the sequence information generated with the speed of MALDI-TOF MS.

2. Detection of Sequence variations

Provided are improved methods for identifying the genomic basis of disease and markers thereof. The sequence variation candidates identified by the methods provided herein include sequences containing sequence variations that are polymorphisms. Polymorphisms include both naturally occurring, somatic sequence variations and those arising from mutation. Polymorphisms include but are not limited to: sequence microvariants where one or more nucleotides in a localized region vary from individual to individual, insertions and deletions which can vary in size from one nucleotides to millions of bases, and microsatellite or nucleotide repeats which vary by numbers of repeats. Nucleotide repeats include homogeneous repeats such as dinucleotide, trinucleotide, tetranucleotide or larger repeats, where the same sequence in repeated multiple times, and also heteronucleotide repeats where sequence motifs are found to repeat. For a given locus the number of nucleotide repeats can vary depending on the individual.

A polymorphic marker or site is the locus at which divergence occurs. Such a site can be as small as one base pair (an SNP). Polymorphic markers include, but are not limited to, restriction fragment length polymorphisms (RFLPs), variable number of tandem repeats (VNTR's), hypervariable regions, minisatellites, dinucleotide repeats, trinucleotide repeats, tetranucleotide repeats and other repeating patterns, simple sequence repeats and insertional elements, such as Alu. Polymorphic forms also are manifested as different Mendelian alleles for a gene. Polymorphisms can be observed by differences in proteins, protein modifications, RNA expression modification, DNA and RNA methylation, regulatory factors that alter gene expression and DNA replication, and any other manifestation of alterations in genomic nucleic acid or organelle nucleic acids.

Furthermore, numerous genes have polymorphic regions. Since individuals have any one of several allelic variants of a polymorphic region, individuals can be identified based on the type of allelic variants of polymorphic regions of genes. This can be used, for example, for forensic purposes. In other situations, it is crucial to know the identity of allelic variants that an individual has. For example, allelic differences in certain genes, for example, major histocompatibility complex (MHC) genes, are involved in graft rejection or graft versus host disease in bone marrow transportation. Accordingly, it is highly desirable to develop rapid, sensitive, and accurate methods for determining the identity of allelic variants of polymorphic regions of genes or genetic lesions. A method or a kit as provided herein can be used to genotype a subject by determining the identity of one or more allelic variants of one or more polymorphic regions in one or more genes or chromosomes of the subject. Genotyping a subject using a method as provided herein can be used for forensic or identity testing purposes and the polymorphic regions can be present in mitochondrial genes or can be short tandem repeats.

Single nucleotide polymorphisms (SNPs) are generally biallelic systems, that is, there are two alleles that an individual can have for any particular marker. This means that the information content per SNP marker is relatively low when compared to microsatellite markers, which can have upwards of 10 alleles. SNPs also tend to be very population-specific; a marker that is polymorphic in one population can not be very polymorphic in another. SNPs, found approximately every kilobase (see Wang et al. (1998) Science 280:1077-1082), offer the potential for generating very high density genetic maps, which will be extremely useful for developing haplotyping systems for genes or regions of interest, and because of the nature of SNPS, they can in fact be the polymorphisms associated with the disease phenotypes under study. The low mutation rate of SNPs also makes them excellent markers for studying complex genetic traits.

Much of the focus of genomics has been on the identification of SNPs, which are important for a variety of reasons. They allow indirect testing (association of haplotypes) and direct testing (functional variants). They are the most abundant and stable genetic markers. Common diseases are best explained by common genetic alterations, and the natural variation in the human population aids in understanding disease, therapy and environmental interactions.

3. Detecting the Presence of Viral or Bacterial Nucleic Acid Sequences Indicative of an Infection

The methods provided herein can be used to determine the presence of viral or bacterial nucleic acid sequences indicative of an infection by identifying sequence variations that are present in the viral or bacterial nucleic acid sequences relative to one or more reference sequences. The reference sequence(s) can include, but are not limited to, sequences obtained from related non-infectious organisms, or sequences from host organisms.

Viruses, bacteria, fungi and other infectious organisms contain distinct nucleic acid sequences, including sequence variants, which are different from the sequences contained in the host cell. A target DNA sequence can be part of a foreign genetic sequence such as the genome of an invading microorganism, including, for example, bacteria and their phages, viruses, fungi, protozoa, and the like. The processes provided herein are particularly applicable for distinguishing between different variants or strains of a microorganism (e.g., pathogenic, less pathogenic, resistant versus non-resistant and the like) in order, for example, to choose an appropriate therapeutic intervention. Examples of disease-causing viruses that infect humans and animals and that can be detected by a disclosed process include but are not limited to Retroviridae (e.g., human immunodeficiency viruses such as HIV-1 (also referred to as HTLV-III, LAV or HTLV-III/LAV; Ratner et al., Nature, 313:227-284 (1985); Wain Hobson et al., Cell, 40:9-17 (1985), HIV-2 (Guyader et al., Nature, 328:662-669 (1987); European Patent Publication No. 0 269 520; Chakrabarti et al., Nature, 328:543-547 (1987); European Patent Application No. 0 655 501), and other isolates such as HIV-LP (International Publication No. WO 94/00562); Picornaviridae (e.g., polioviruses, hepatitis A virus, (Gust et al., Intervirology, 20:1-7 (1983)); enteroviruses, human coxsackie viruses, rhinoviruses, echoviruses); Calcivirdae (e.g. strains that cause gastroenteritis); Togaviridae (e.g., equine encephalitis viruses, rubella viruses); Flaviridae (e.g., dengue viruses, encephalitis viruses, yellow fever viruses); Coronaviridae (e.g., coronaviruses); Rhabdoviridae (e.g., vesicular stomatitis viruses, rabies viruses); Filoviridae (e.g., ebola viruses); Paramyxoviridae (e.g., parainfluenza viruses, mumps virus, measles virus, respiratory syncytial virus); Orthomyxoviridae (e.g., influenza viruses); Bungaviridae (e.g., Hantaan viruses, bunga viruses, phleboviruses and Nairo viruses); Arenaviridae (hemorrhagic fever viruses); Reoviridae (e.g., reoviruses, orbiviruses and rotaviruses); Birnaviridae; Hepadnaviridae (Hepatitis B virus); Parvoviridae (parvoviruses); Papovaviridae; Hepadnaviridae (Hepatitis B virus); Parvoviridae (most adenoviruses); Papovaviridae (papilloma viruses, polyoma viruses); Adenoviridae (most adenoviruses); Herpesviridae (herpes simplex virus type 1 (HSV-1) and HSV-2, varicella zoster virus, cytomegalovirus, herpes viruses; Poxviridae (variola viruses, vaccinia viruses, pox viruses); Iridoviridae (e.g., African swine fever virus); and unclassified viruses (e.g., the etiological agents of Spongiform encephalopathies, the agent of delta hepatitis (thought to be a defective satellite of hepatitis B virus), the agents of non-A, non-B hepatitis (class 1=internally transmitted; class 2=parenterally transmitted, i.e., Hepatitis C); Norwalk and related viruses, and astroviruses.

Examples of infectious bacteria include but are not limited to Helicobacter pyloris, Borelia *burgdorferi,* Legionella *pneumophilia,* Mycobacteria sp. (e.g. *M. tuberculosis, M. avium, M. intracellulare, M. kansaii, M. gordonae),* Salmonella, *Staphylococcus aureus,* Neisseria *gonorrheae,* Neisseria *meningitidis,* Listeria *monocytogenes,* Streptococcus *pyogenes* (Group A Streptococcus), *Streptococcus agalactiae* (Group B Streptococcus), Streptococcus sp. (viridans group), Streptococcus faecalis, Streptococcus bovis, Streptococcus sp. (anaerobic species), Streptococcus *pneumoniae,* pathogenic Campylobacter sp., Enterococcus sp., Haemophilus *influenzae,* Bacillus *antracis,* Corynebacterium *diphtheriae,* Corynebacterium sp., Erysipelothrix *rhusiopathiae,* Clostridium *perfringens,* Clostridium *tetani,* Escherichia coli, Enterobacter *aerogenes,* Klebsiella *pneumoniae,* Pasturella *multocida,* Bacteroides sp., Fusobacterium *nucleatum,* Streptobacillus *moniliformis,* Treponema *pallidum,* Treponema *pertenue,* Leptospira, and Actinomyces *israelli* and any variants including antibiotic resistance variants

Examples of infectious fungi include but are not limited to Cryptococcus *neoformans,* Histoplasma *capsulatum,* Coccidioides *immitis,* Blastomyces *dermatitidis,* Chlamydia *trachomatis,* Candida *albicans.* Other infectious organisms include protists such as Plasmodium *falciparum* and Toxoplasma *gondii.*

### 4. Antibiotic Profiling

The analysis of specific cleavage patterns as provided herein improves the speed and accuracy of detection of nucleotide changes involved in drug resistance, including antibiotic resistance. Genetic loci involved in resistance to isoniazid, rifampin, streptomycin, fluoroquinolones, and ethionamide have been identified [Heym et al., Lancet 344:293 (1994) and Morris et al., J. Infect. Dis. 171:954 (1995)]. A combination of isoniazid (inh) and rifampin (rif) along with pyrazinamide and ethambutol or streptomycin, is routinely used as the first line of attack against confirmed cases of M. tuberculosis [Banerjee et al., Science 263:227 (1994)]. The increasing incidence of such resistant strains necessitates the development of rapid assays to detect them and thereby reduce the expense and community health hazards of pursuing ineffective, and possibly detrimental, treatments. The identification of some of the genetic loci involved in drug resistance has facilitated the adoption of mutation detection technologies for rapid screening of nucleotide changes that result in drug resistance. In addition, the technology facilitates treatment monitoring and tracking or microbial population structures as well as surveillance monitoring during treatment. In addition, correlations and surveillance monitoring of mixed populations can be performed.

### 5. Identifying Disease Markers

Provided herein are methods for the rapid and accurate identification of sequence variations that are genetic markers of disease, which can be used to diagnose or determine the prognosis of a disease. Diseases characterized by genetic markers can include, but are not limited to, atherosclerosis, obesity, diabetes, autoimmune disorders, and cancer. Diseases in all organisms have a genetic component, whether inherited or resulting from the body's response to environmental stresses, such as viruses and toxins. The ultimate goal of ongoing genomic research is to use this information to develop new ways to identify, treat and potentially cure these diseases. The first step has been to screen disease tissue and identify genomic changes at the level of individual samples. The identification of these "disease" markers is dependent on the ability to detect changes in genomic markers in order to identify errant genes or sequence variants. Genomic markers (all genetic loci including single nucleotide polymorphisms (SNPs), microsatellites and other noncoding genomic regions, tandem repeats, introns and exons) can be used for the identification of all organisms, including humans. These markers provide a way to not only identify populations but also allow stratification of populations according to their response to disease, drug treatment, resistance to environmental agents, and other factors.

### 6. Haplotyping

The methods provided herein can be used to detect haplotypes. In any diploid cell, there are two haplotypes at any gene or other chromosomal segment that contain at least one distinguishing variance. In many well-studied genetic systems, haplotypes are more powerfully correlated with phenotypes than single nucleotide variations. Thus, the determination of haplotypes is valuable for understanding the genetic basis of a variety of phenotypes including disease predisposition or susceptibility, response to therapeutic interventions, and other phenotypes of interest in medicine, animal husbandry, and agriculture.

Haplotyping procedures as provided herein permit the selection of a portion of sequence from one of an individual's two homologous chromosomes and to genotype linked SNPs on that portion of sequence. The direct resolution of haplotypes can yield increased information content, improving the diagnosis of any linked disease genes or identifying linkages associated with those diseases.

7. Microsatellites

The cleavage-based methods provided herein allow for rapid, unambiguous detection of sequence variations that are microsatellites. Microsatellites (sometimes referred to as variable number of tandem repeats or VNTRs) are short tandemly repeated nucleotide units of one to seven or more bases, the most prominent among them being di-, tri-, and tetranucleotide repeats. Microsatellites are present every 100,000 bp in genomic DNA (J. L. Weber and P. E. Can, Am. J. Hum. Genet. 44, 388 (1989); J. Weissenbach et al., Nature 359, 794 (1992)). CA dinucleotide repeats, for example, make up about 0.5% of the human extra-mitochondrial genome; CT and AG repeats together make up about 0.2%. CG repeats are rare, most probably due to the regulatory function of CpG islands. Microsatellites are highly polymorphic with respect to length and widely distributed over the whole genome with a main abundance in non-coding sequences, and their function within the genome is unknown.

Microsatellites are important in forensic applications, as a population will maintain a variety of microsatellites characteristic for that population and distinct from other populations which do not interbreed.

Many changes within microsatellites can be silent, but some can lead to significant alterations in gene products or expression levels. For example, trinucleotide repeats found in the coding regions of genes are affected in some tumors (C. T. Caskey et al., Science 256, 784 (1992) and alteration of the microsatellites can result in a genetic instability that results in a predisposition to cancer (P. J. McKinnen, Hum. Genet. 1 75, 197 (1987); J. German et al., Clin. Genet. 35, 57 (1989)).

8. Short Tandem Repeats

The methods provided herein can be used to identify short tandem repeat (STR) regions in some target sequences of the human genome relative to, for example, reference sequences in the human genome that do not contain STR regions. STR regions are polymorphic regions that are not related to any disease or condition. Many loci in the human genome contain a polymorphic short tandem repeat (STR) region. STR loci contain short, repetitive sequence elements of 3 to 7 base pairs in length. It is estimated that there are 200,000 expected trimeric and tetrameric STRs, which are present as frequently as once every 15 kb in the human genome (see, e.g., International PCT application No. WO 9213969 A1, Edwards et al., Nucl. Acids Res. 19:4791 (1991); Beckmann et al. (1992) Genomics 12:627-631). Nearly half of these STR loci are polymorphic, providing a rich source of genetic markers. Variation in the number of repeat units at a particular locus is responsible for the observed sequence variations reminiscent of variable nucleotide tandem repeat (VNTR) loci (Nakamura et al. (1987) Science 235:1616-1622); and minisatellite loci (Jeffreys et al. (1985) Nature 314:67-73), which contain longer repeat units, and microsatellite or dinucleotide repeat loci (Luty et al. (1991) Nucleic Acids Res. 19:4308; Litt et al. (1990) Nucleic Acids Res. 18:4301; Litt et al. (1990) Nucleic Acids Res. 18:5921; Luty et al. (1990) Am. J. Hum. Genet. 46:776-783; Tautz (1989) Nucl. Acids Res. 17:6463-6471; Weber et al. (1989) Am. J. Hum. Genet. 44:388-396; Beckmann et al. (1992) Genomics 12:627-631). VNTR typing is a very established tool in microbial typing e.g. M. *tuberculosis.*

Examples of STR loci include, but are not limited to, pentanucleotide repeats in the human CD4 locus (Edwards et al., Nucl. Acids Res. 19:4791 (1991)); tetranucleotide repeats in the human aromatase cytochrome P-450 gene (CYP19; Polymeropoulos et al., Nucl. Acids Res. 19:195 (1991)); tetranucleotide repeats in the human coagulation factor XIII A subunit gene (F13A1; Polymeropoulos et al., Nucl. Acids Res. 19:4306 (1991)); tetranucleotide repeats in the F13B locus (Nishimura et al., Nucl. Acids Res. 20:1167 (1992)); tetranucleotide repeats in the human c-les/fps, proto-oncogene (FES; Polymeropoulos et al., Nucl. Acids Res. 19:4018 (1991)); tetranucleotide repeats in the LFL gene (Zuliani et al., Nucl. Acids Res. 18:4958 (1990)); trinucleotide repeat sequence variations at the human pancreatic phospholipase A-2 gene (PLA2; Polymeropoulos et al., Nucl. Acids Res. 18:7468 (1990)); tetranucleotide repeat sequence variations in the VWF gene (Ploos et al., Nucl. Acids Res. 18:4957 (1990)); and tetranucleotide repeats in the human thyroid peroxidase (hTPO) locus (Anker et al., Hum. Mol. Genet. 1:137 (1992)).

9. Organism Identification

Polymorphic STR loci and other polymorphic regions of genes are sequence variations that are extremely useful markers for human identification, paternity and maternity testing, genetic mapping, immigration and inheritance disputes, zygosity testing in twins, tests for inbreeding in humans, quality control of human cultured cells, identification of human remains, and testing of semen samples, blood stains, microbes and other material in forensic medicine. Such loci also are useful markers in commercial animal breeding and pedigree analysis and in commercial plant breeding. Traits of economic importance in plant crops and animals can be identified through linkage analysis using polymorphic DNA markers. Efficient and accurate methods for determining the identity of such loci are provided herein.

10. Detecting Allelic Variation

The methods provided herein allow for high-throughput, fast and accurate detection of allelic variants. Studies of allelic variation involve not only detection of a specific sequence in a complex background, but also the discrimination between sequences with few, or single, nucleotide differences. One method for the detection of allele-specific variants by PCR is based upon the fact that it is difficult for Taq polymerase to synthesize a DNA strand when there is a mismatch between the template strand and the 3' end of the primer. An allele-specific variant can be detected by the use of a primer that is perfectly matched with only one of the possible alleles; the mismatch to the other allele acts to prevent the extension of the primer, thereby preventing the amplification of that sequence. This method has a substantial limitation in that the base composition of the mismatch influences the ability to prevent extension across the mismatch, and certain mismatches do not prevent extension or have only a minimal effect (Kwok et al., Nucl. Acids Res., 18:999 [1990]).) The cleavage-based methods provided herein overcome the limitations of the primer extension method.

11. Determining Allelic Frequency

The methods herein described are valuable for identifying one or more genetic markers whose frequency changes within the population as a function of age, ethnic group, sex or some  other criteria. For example, the age-dependent distribution of ApoE genotypes is known in the art (see, Schchter et al. (1994) Nature Genetics 6:29-32). The frequencies of sequence variations known to be associated at some level with disease can also be used to detect or monitor progression of a disease state. For example, the N291S polymorphism (N291S) of the Lipoprotein Lipase gene, which results in a substitution of a serine for an asparagine at amino acid codon 291, leads to reduced levels of high density lipoprotein cholesterol (HDL-C) that is associated with an increased risk of males for arteriosclerosis and in particular myocardial infarction (see, Reymer et al. (1995) Nature Genetics 10:28-34). In addition, determining changes in allelic frequency can allow the identification of previously unknown sequence variations and ultimately a gene or pathway involved in the onset and progression of disease.

12. Epigenetics

The methods provided herein can be used to study variations in a target nucleic acid or protein relative to a reference nucleic acid or protein that are not based on sequence, e.g., the identity of bases or amino acids that are the naturally occurring monomeric units of the nucleic acid or protein. For example, the specific cleavage reagents employed in the methods provided herein may recognize differences in sequence-independent features such as methylation patterns, the presence of modified bases or amino acids, or differences in higher order structure between the target molecule and the reference molecule, to generate fragments that are cleaved at sequence-independent sites. Epigenetics is the study of the inheritance of information based on differences in gene expression rather than differences in gene sequence. Epigenetic changes refer to mitotically and/or meiotically heritable changes in gene function or changes in higher order nucleic acid structure that cannot be explained by changes in nucleic acid sequence. Examples of features that are subject to epigenetic variation or change include, but are not limited to, DNA methylation patterns in animals, histone modification and the Polycomb-trithorax group (Pc-G/tx) protein complexes (see, e.g., Bird, A., Genes Dev., 16:6-21 (2002)).

Epigenetic changes usually, although not necessarily, lead to changes in gene expression that are usually, although not necessarily, inheritable. For example, as discussed further below, changes in methylation patterns is an early event in cancer and other disease development and progression. In many cancers, certain genes are inappropriately switched off or switched on due to aberrant methylation. The ability of methylation patterns to repress or activate transcription can be inherited. The Pc-G/trx protein complexes, like methylation, can repress transcription in a heritable fashion. The Pc-G/trx multiprotein assembly is targeted to specific regions of the genome where it effectively freezes the embryonic gene expression status of a gene, whether the gene is active or inactive, and propagates that state stably through development. The ability of the Pc-G/trx group of  proteins to target and bind to a genome affects only the level of expression of the genes contained in the genome, and not the properties of the gene products. The methods provided herein can be used with specific cleavage reagents that identify variations in a target sequence relative to a reference sequence that are based on sequence-independent changes, such as epigenetic changes.

13. Methylation Patterns

The methods provided herein can be used to detect sequence variations that are epigenetic changes in the target sequence, such as a change in methylation patterns in the target sequence. Analysis of cellular methylation is an emerging research discipline. The covalent addition of methyl groups to cytosine is primarily present at CpG dinucleotides (microsatellites). Although the function of CpG islands not located in promoter regions remains to be explored, CpG islands in promoter regions are of special interest because their methylation status regulates the transcription and expression of the associated gene. Methylation of promotor regions leads to silencing of gene expression. This silencing is permanent and continues through the process of mitosis. Due to its significant role in gene expression, DNA methylation has an impact on developmental processes, imprinting and X-chromosome inactivation as well as tumor genesis, aging, and also suppression of parasitic DNA. Methylation is thought to be involved in the cancerogenesis of many widespread tumors, such as lung, breast, and colon cancer, an in leukemia. There is also a relation between methylation and protein dysfunctions (long Q-T syndrome) or metabolic diseases (transient neonatal diabetes, type 2 diabetes).

Bisulfite treatment of genomic DNA can be utilized to analyze positions of methylated cytosine residues within the DNA. Treating nucleic acids with bisulfite deaminates cytosine residues to uracil residues, while methylated cytosine remains unmodified. Thus, by comparing the sequence of a target nucleic acid that is not treated with bisulfite with the sequence of the nucleic acid that is treated with bisulfite in the methods provided herein, the degree of methylation in a nucleic acid as well as the positions where cytosine is methylated can be deduced.

Methylation analysis via restriction endonuclease reaction is made possible by using restriction enzymes which have methylation-specific recognition sites, such as Hpall and MSPI. The basic principle is that certain enzymes are blocked by methylated cytosine in the recognition sequence. Once this differentiation is accomplished, subsequent analysis of the resulting fragments can be performed using the methods as provided herein.

These methods can be used together in combined bisulfite restriction analysis (COBRA). Treatment with bisulfite causes a loss in BstUI recognition site in amplified PCR product, which causes a new detectable fragment to appear on analysis compared to untreated sample. The cleavage-based methods provided herein can be used in conjunction with specific cleavage of methylation sites to provide rapid, reliable information on the methylation patterns in a target nucleic acid sequence.

14. Resequencing

The dramatically growing amount of available genomic sequence information from various organisms increases the need for technologies allowing large-scale comparative sequence analysis to correlate sequence information to function, phenotype, or identity. The application of such technologies for comparative sequence analysis can be widespread, including SNP discovery and sequence-specific identification of pathogens. Therefore, resequencing and high-throughput mutation screening technologies are critical to the identification of mutations underlying disease, as well as the genetic variability underlying differential drug response.

Several approaches have been developed in order to satisfy these needs. The current technology for high-throughput DNA sequencing includes DNA sequencers using electrophoresis and laser-induced fluorescence detection. Electrophoresis-based sequencing methods have inherent limitations for detecting heterozygotes and are compromised by GC compressions. Thus a DNA sequencing platform that produces digital data without using electrophoresis will overcome these problems. Matrix-assisted laser desorption/ionization time-of-flight mass spectrometry (MALDI-TOF MS) measures DNA fragments with digital data output. The methods of specific cleavage fragmentation analysis provided herein allow for high-throughput, high speed and high accuracy in the detection of sequence variations relative to a reference sequence. This approach makes it possible to routinely use MALDI-TOF MS sequencing for accurate mutation detection, such as screening for founder mutations in BRCA1 and BRCA2, which are linked to the development of breast cancer.

15. Multiplexing

The methods provided herein allow for the high-throughput detection or discovery of sequences in a plurality of target sequences relative to one or a plurality of reference sequences. Multiplexing refers to the simultaneous detection of more than one sequence, polymorphism or sequence variation. Methods for performing multiplexed reactions, particularly in conjunction with mass spectrometry, are known (see, e.g., U.S. Pat. Nos. 6,043,031, 5,547,835 and International PCT application No. WO 97/37041).

Multiplexing can be performed, for example, for the same target nucleic acid sequence using different complementary specific cleavage reactions as provided herein, or for different target nucleic acid sequences, and the cleavage patterns can in turn be analyzed against a plurality of reference nucleic acid sequences. Several mutations or sequence variations can also be simultaneously detected on one target sequence by employing the methods provided herein where each sequence variation corresponds to a different cleavage product relative to the cleavage pattern of the reference nucleic acid sequence. Multiplexing provides the advantage that a plurality of sequence variations can be identified in as few as a single mass spectrum, as compared to having to perform a separate mass spectrometry analysis for each individual sequence variation. The methods provided herein lend themselves to high-throughput, highly-automated processes for analyzing sequence variations with high speed and accuracy. Mixed population analysis of sequence variation detection in populations.

16. Disease outbreak monitoring

In times of global transportation and travel outbreaks of pathogenic endemics require close monitoring to prevent their worldwide spread and enable control. DNA based typing by high-throughput technologies enable a rapid sample throughput in a comparatively short time, as required in an outbreak situation (e.g. monitoring in the hospital environment, early warning systems). Monitoring is dependent of the microbial marker region used, but can facilitate monitoring to the genus, species, strain or subtype specific level. Add biodefense applications, application in metagenomics (e.g. analysis of the gut flora). Such monitoring of treatment progress or failure is described in U.S. Pat. No. 7,255,992, U.S. Pat. No. 7,217,510, U.S. Pat. No. 7,226,739 and U.S. Pat. No. 7,108,974.

17. Vaccine quality control and production clone quality control

The technology can be used to control the identity of recombinant production clones, which can be vaccines or e.g. insulin or any other production clone or biological or medical product.

18. Microbial monitoring in pharma for production control and QC

Systems and Software

Also provided are systems that automate sequence comparison processes using a computer programmed for performing comparison analyses described herein. The processes can be implemented, for example, by use of the following computer systems and using the following calculations, systems and methods.

An exemplary automated testing system contains a nucleic acid workstation that includes an analytical instrument, such as a gel electrophoresis apparatus or a mass spectrometer or other instrument for determining the mass of a nucleic acid molecule in a sample, and a computer for cleavage data analysis capable of communicating with the analytical instrument (see, e.g., U.S. Patent Application Nos. 09/285,481, 09/663,968 and 09/836,629; see, also International Application No. WO 00/60361 for examples of automated systems). In an embodiment, the computer is a desktop computer system, such as a computer that operates under control of the "Microsoft Windows" operation system of Microsoft Corporation or the "Macintosh" operating system of Apple Computer, Inc., that communicates with the instrument using a known communication standard such as a parallel or serial interface.

For example, systems for analysis of nucleic acid samples are provided. The systems include a processing station that performs a base-specific or other specific cleavage reaction as described herein; a robotic system that transports the resulting cleavage fragments from the processing station to a mass measuring station, where the masses of the products of the reaction are determined; and a data analysis system, such as a computer programmed to identify sequence variations in the target nucleic acid sequence using the cleavage data, that processes the data from the mass measuring station to identify a nucleotide or plurality thereof in a sample or plurality thereof. The system can also include a control system that determines when processing at each station is complete and, in response, moves the sample to the next test station, and continuously processes samples one after another until the control system receives a stop instruction.

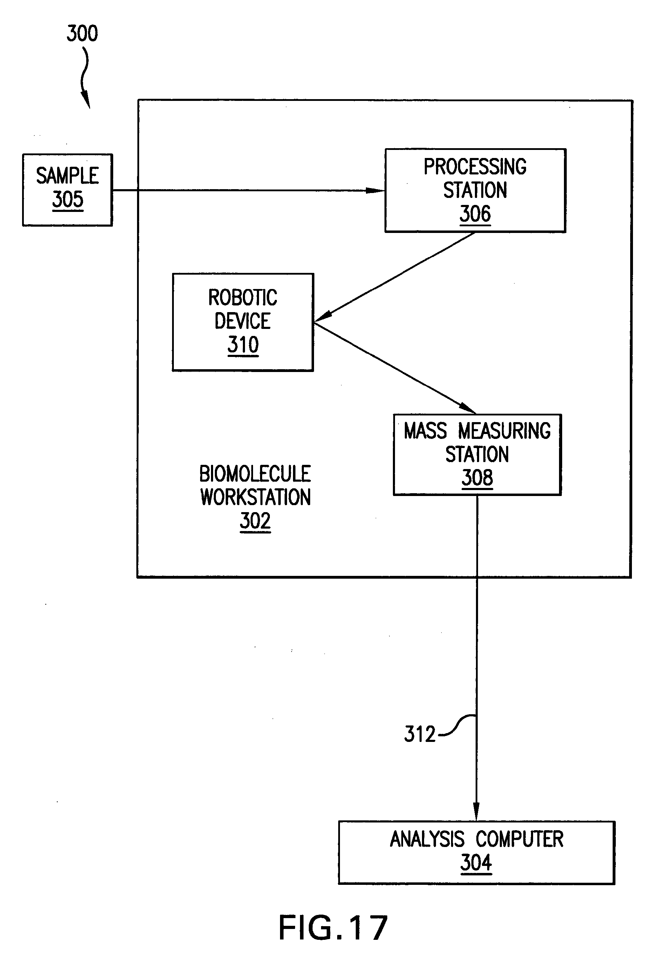

Figure 17 is a block diagram of a system that performs sample processing and performs the operations described herein. The system 300 includes a nucleic acid workstation 302 and an analysis computer 304. At the nucleic work station, one or more molecular samples 305 are received and prepared for analysis at a processing station 306, where the above-described cleavage reactions can take place. The samples are then moved to a mass measuring station 308, such as a mass spectrometer, where further sample processing takes place. The samples are preferably moved from the sample processing station 306 to the mass measuring station 308 by a computer-controlled robotic device 310.

The robotic device can include subsystems that ensure movement between the two processing stations 306, 308 that will preserve the integrity of the samples 305 and will ensure valid test results. The subsystems can include, for example, a mechanical lifting device or arm that can pick up a sample from the sample processing station 306, move to the mass measuring station 308, and then deposit the processed sample for a mass measurement operation. The robotic device 310 can then remove the measured sample and take appropriate action to move the next processed sample from the processing station 306. Sample preparation can be integrated in the sample carrier or in the measurement station, and in such embodiments, a lifting device or arm is optional. Samples may be processed on or in the robotic device, and in some embodiments, the complete system is a fully integrated platform.

The mass measurement station 308 produces data that identifies and quantifies the molecular components of the sample 305 being measured. Those skilled in the art will be familiar with molecular measurement systems, such as mass spectrometers, that can be used to produce the measurement data. The data is provided from the mass measuring station 308 to the analysis computer 304, either by manual entry of measurement results into the analysis computer or by communication between the mass measuring station and the analysis computer. For example, the mass measuring station 308 and the analysis computer 304 can be interconnected over a network 312 such that the data produced by the mass measuring station can be obtained by the analysis computer. The network 312 can comprise a local area network (LAN), or a wireless communication channel, or any other communications channel that is suitable for computer-to-computer data exchange.

The measurement processing function of the analysis computer 304 and the control function of the nucleic acid workstation 302 can be incorporated into a single computer device, if desired. In that configuration, for example, a single general purpose computer can be used to control the robotic device 310 and to perform the data processing of the data analysis computer 304. Similarly, the processing operations of the mass measuring station and the sample processing operations of the sample processing station 306 can be performed under the control of a single computer.

Thus, the processing and analysis functions of the stations and computers 302, 304, 306, 308, 310 can be performed by variety of computing devices, if the computing devices have a suitable interface to any appropriate subsystems (such as a mechanical arm of the robotic device 310) and have suitable processing power to control the systems and perform the data processing.

The data analysis computer 304 can be part of the analytical instrument or another system component or it can be at a remote location. The computer system can communicate with the instrument can communicate with the instrument, for example, through a wide area network or local area communication network or other suitable communication network. The system with the computer is programmed to automatically carry out steps of the methods herein and the requisite calculations. For embodiments that use predicted cleavage patterns (of a reference or target sequence) based on the cleavage reagent(s) and modified bases or amino acids employed, a user enters a sequence or measures reference samples to obtain the masses of the predicted cleavage products produced by the system. These data can be directly entered by the user from a keyboard or from other computers or computer systems linked by network connection, or on removable storage medium such as a data CD, minidisk (MD), DVD, floppy disk or other suitable storage medium. Next, the user initiates execution software that operates the system in which the cleavage product differences between the target nucleic acid sequence and the reference nucleic acid sequence, are identified.

Multiple of these systems can be networked and can feed into a global database.

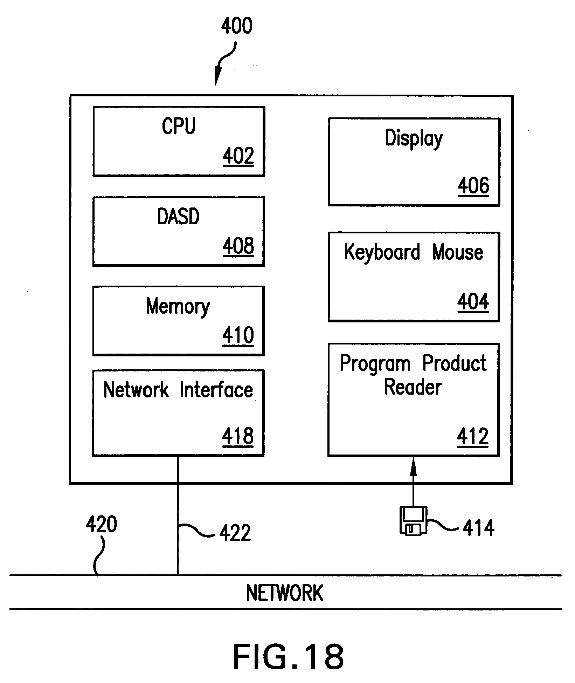

Figure 18 is a block diagram of a computer in the system 300 of Figure 17, illustrating the hardware components included in a computer that can provide the functionality of the stations and computers 302, 304, 306, 308. Those skilled in the art will appreciate that the stations and computers illustrated in Figure 17 can all have a similar computer construction, or can have alternative constructions consistent with the capabilities and respective functions described herein. The Figure 18 construction is especially suited for the data analysis computer 304 illustrated in Figure 17.

Figure 18 shows an exemplary computer 400 such as might comprise a computer that controls the operation of any of the stations and analysis computers 302, 304, 306, 308. Each computer 400 operates under control of a central processor unit (CPU) 402, such as a "Pentium" microprocessor and associated integrated circuit chips, available from Intel Corporation of Santa Clara, Calif., USA. A computer user can input commands and data from a keyboard and computer mouse 404, and can view inputs and computer output at a display 406. The display is typically a video monitor or flat panel display. The computer 400 also includes a direct access storage device (DASD) 408, such as a hard disk drive. The computer includes a memory 410 that typically comprises volatile semiconductor random access memory (RAM). Each computer preferably includes a program product reader 412 that accepts a program product storage device 414, from which the program product reader can read data (and to which it can optionally write data). The program product reader can comprise, for example, a disk drive, and the program product storage device can comprise removable storage media such as a magnetic floppy disk, a CD-R disc, a CD-RW disc, or DVD disc.

Each computer 400 can communicate with the other Figure 17 systems over a computer network 420 (such as, for example, the local network 312 or the Internet or an intranet) through a network interface 418 that enables communication over a connection 422 between the network 420 and the computer. The network interface 418 typically comprises, for example, a Network Interface Card (NIC) that permits communication over a variety of networks, along with associated network access subsystems, such as a modem.

The CPU 402 operates under control of programming instructions that are temporarily stored in the memory 410 of the computer 400. When the programming instructions are executed, the computer performs its functions. Thus, the programming instructions implement the functionality of the respective workstation or processor. The programming instructions can be received from the DASD 408, through the program product storage device 414, or through the network connection 422. The program product storage drive 412 can receive a program product 414, read programming instructions recorded thereon, and transfer the programming instructions into the memory 410 for execution by the CPU 402. As noted above, the program product storage device can comprise any one of multiple removable media having recorded computer-readable instructions, including magnetic floppy disks and CD-ROM storage discs. Other suitable program product storage devices can include magnetic tape and semiconductor memory chips. In this way, the processing instructions necessary for operation in accordance with them methods and disclosure herein can be embodied on a program product.

Alternatively, the program instructions can be received into the operating memory 410 over the network 420.

In the network method, the computer 400 receives data including program instructions into the memory 410 through the network interface 418 after network communication has been established over the network connection 422 by well-known methods that will be understood by those skilled in the art without further explanation. The program instructions are then executed by the CPU 402 thereby comprising a computer process.

It should be understood that all of the stations and computers of the system 300 illustrated in Figure 17 can have a construction similar to that shown in figure 18, so that details described with respect to the Figure 18 computer 400 will be understood to apply to all computers of the system 300. It should be appreciated that any of the communicating stations and computers can have an alternative construction, so long as they can communicate with the other communicating stations and computers illustrated in Figure 17 and can support the functionality described herein. For example, if a workstation will not receive program instructions from a program product device, then it is not necessary for that workstation to include that capability, and that workstation will not have the elements depicted in Figure 18 that are associated with that capability.

Examples

The following examples illustrate but do not limit the invention.

Accurate characterization of infectious disease agents is essential to epidemiological surveillance and public health decisions, such as outbreak recognition, detection of pathogen cross-transmission, determination of the source of infection, recognition of particularly virulent strains and monitoring vaccination programs, for example. While phenotypic characteristics such as morphology and physiological properties have traditionally been utilized to characterize microbes, nucleic acid analysis technologies paved the way for modern typing approaches. Phenotypic markers are subject to genetic regulation and respond to environmental stimuli such as culture, sub-culture and storage conditions, whereas suitable nucleic acid based characterization methods deliver a stable fingerprint of the sample important for global comparability and phylogenetic analysis.

Recently, the development and prevalence of microbial DNA-based identification and typing has significantly increased. Applications often are high-throughput in nature and appropriate typing methods require accuracy, reproducibility and laboratory automation (Clarke 2002).

Common nucleic acid analysis tools are based on gel electrophoresis or fingerprinting and rely on electrophoretic mobility. Pulse-field gel electrophoresis (PFGE) is still the most widely used method as a result of its discriminatory capacity between related and non related isolates. Standardized protocols and reference databases have been established worldwide, but as for classic fingerprinting, problems of this technology remain. These encompass manual scoring of ambiguous bands, variable signal intensities, background noise of the electrophoretic profile, different mobilities of high and low molecular bands, uncertainty of the genetic identity of two bands of equal size and distortion between gels. Digital formats of the results and data portability are challenging and not easily available on a global basis. Processing times of up to 3 days reduce the ability to analyze large number of samples (Olive and Bean 1999). New technologies for whole genome comparative sequencing, such as whole genome DNA microarrays, are prohibitively expensive and lack ease of use to allow for the comparison of large numbers of isolates in an automated high-throughput scenario

A multitude of additional DNA based techniques have been investigated for their applicability in epidemiology. These techniques include single nucleotide polymorphism (SNP) detection, ribotyping, insertion sequence (IS) profiling, variable number of tandem repeat (VNTR) analysis, or a combination of these. Nucleotide composition analysis of short amplification products, e.g., approximately 100 bp PCR products, by electrospray mass spectrometry has been described, where the detected mass of the product is used to determine a constrained list of nucleotide compositions for microbial identification. Sequence variations can be detected, but not localized or converted to a new sequence (Van Ert, M. N., Hofstadler, S.A., Jiang Y., Busch, J. D., Wagner, D. M., Drader J. J., Ecker, D. J., Hannis, J. C., Huynh, L. Y., Schupp, J. M. et al. (2004), Biotechniques 37, 642-644; Sampath, R., Hofstadler, S.A., Blyn, L. B., Eshoo, M. W., Hall, T. A., Massire, C., Levene, H. M., Hannis, J. C., Harrell, P. M., Neuman, B. et al. (2005) Emerg Infect Dis 11, 373-379; Ecker, J. A., Massire, C., Hall, T. A., Ranken, R., Pennella, T.T., Agasino Ivy, C., Blyn, L.B., Hofstadler, S. A., Endy, T. P., Scott, P. T. et al. (2006) J Clin Microbiol 44, 2921-2932).

Traditional microbial typing technologies for the characterization of pathogenic microorganisms and monitoring of their global spread are often difficult to standardize, poorly portable, and lack ease of use, throughput and automation.

To overcome these problems, introduced here is an approach for comparative sequence analysis by MALDI-TOF (matrix assisted laser desorption ionization time-of flight) mass spectrometry for automated high-throughput molecular-based microbial analysis. Multilocus sequence data derived from the public MLST database (World Wide Web URL "pubmlst.org/neisseria/") established a reference data set of simulated peak patterns. A model pathogen *Neisseria meningitidis* was used to validate the technology and explore its applicability as an alternative to dideoxy sequencing. One hundred *N. meningitidis* samples were typed by comparing MALDI-TOF MS fingerprints of the standard MLST loci to reference sequences available in the public MLST database. Identification results were in concordance with classical dideoxy sequencing. Sequence types (STs) of 89 samples were represented in the database, seven samples revealed new STs including three new alleles and four samples contained mixed populations of multiple STs. The approach shows interlaboratory reproducibility and allows for the exchange of mass spectrometric fingerprints to study the geographic spread of epidemic *N. meningitidis* strains or other microbes of clinical importance.

Reference sequence based MALDI-TOF MS typing is a generic approach, which facilitates comparative sequence analysis and the identification of any microbial taxa with a broad application across the fields of microbiology and epidemiology.

Reported here is the validation of base-specific cleavage and MALDI-TOF MS based MLST for the identification of lineages of the bacterial pathogen *Neisseria meningitidis.* The study was performed as a blind study with the goal of correct sequence type assignments for 100 isolates in reference to the database located at the World Wide Web (www) URL "pubmlst.org/neisseria/." MALDI-TOF MS signature sequence-based typing for high level discrimination of individual microbial taxa for signatures within variable regions in the 16S rDNA gene region has previously been applied to discriminate mycobacteria and Bordetella species (Lefmann et al. 2004; von Wintzingerode et al. 2002). In contrast, MLST is based on characterizing variations in the sequence of several loci, which are accumulating slowly within a microbial population. MLST thus requires differentiation of reference sequences based on single nucleotide deviations, a study to challenge the comparative sequencing approach by base-specific cleavage and MALDI-TOF MS.

Example 1: Materials and Methods

*Bacterial Strains*

A total of 100 *N. meningitidis* isolates from various serogroups were supplied by the National Meningitidis Reference Laboratory, Manchester, UK and by the National Collection of Type Cultures, London, UK. All strains were grown for 24 hours on Chocolate Agar (Media Dept., Cfl) in 10% $CO_2$ at 37 degrees C. Isolates were stored on MicrobankTM plastic storage beads (Pro-Lab Diagnostics) at 80 degrees C for long-term storage.

DNA extraction was performed using the Schleicher&Schuell DNA Iso-Code storage paper. In brief, two 1 microliter loops of growth were re-suspended in 100 microliters of $dH_2O$ and frozen overnight at -30 degrees C for cell lysis. Fifty (50) microliters of sample were spotted on each spot of the paper. Two 3 mm paper punches were used to subsequently elute the DNA in 1 ml $dH_2O$. 50 microliter aliquots of sample were heated for 20 mins at 95 degrees C to obtain DNA ready to use in PCR.

*MLST by Dideoxy sequencing*

The MLST scheme for *N. meningitidis* uses internal fragments of seven housekeeping genes abcZ (putative ABC transporter), adk (adenylate kinase), aroE (shikimate dehydrogenase), fumC (fumarate hydratase), gdh (glucose-6-phosphate dehydrogenase), pdhC (pyruvate dehydrogenase subunit) and pgm (phosphoglycomutase). These loci were amplified from chromosomal DNA of the 100 N. meningitidis strains and sequenced on both strands as described for the standard MLST PCR and sequencing protocol (World Wide Web URL address "pubmlst.org/neisseria/mlst-info/nmeningitidis/nmeningitidis-info.shtml"). For a head-to head comparison comparative sequence analysis by MALDI-TOF MS and dideoxy sequencing sequences of both strands were obtained by using a Beckman Coulter CEQ automated sequencer according to the manufacturers protocol (Beckman Coulter).

*MLST by MALDI-TOF MS*

*Reference sequence sets*

Reference sequence sets of the seven *N. meningitidis* specific.loci were used as published (World Wide Web URL address "pubmlst.org/neisseria/," updated 10/18/2004) to create import files for MALDI-TOF MS analysis. The sets were modified by the addition of the gene specific primer regions of the forward as well as the reverse primer and a stretch of consensus sequence to fill the gap between the primer sequence and the trimmed published reference.

For aroE the corresponding sequence stretch of *N. meningitidis* serogroup B strain MC58 (GenBank accession no. NC_003112) was utilized, while the corresponding sequence region of the *N. meningitidis* serogroup A strain Z2491 (GenBank accession no. NC_003116) was used for the rest of the loci.

*Amplicon design*

Standard MLST sequencing primers were utilized for PCR. All primers were tagged with a T7-RNA promoter sequence as well as a unique 10 bp sequence tag (Supplemental Table 2). Two sets of PCR primers allowed for transcription of either sense or anti-sense strand and thus base-specific analysis of both DNA strands.

*PCR, base-specific cleavage and MALDI-TOF MS*

Samples were processed in parallel in 384 microtiter plates utilizing a 96-channel automated pipetter (Sequenom). Loci of interest were amplified in 5-10microliters PCR reactions. Reactions contained 1x PCR buffer [Tris-HCl, KCl, (NH4)2SO4, MgCl2 at pH8.7; final concentration of 1.5 mM], 200 $\mu$M of each dNTP, 0.1 U of HotStar Taq polymerase (QIAGEN), 1 pmol of each primer and 1-5 ng of DNA. 45 PCR cycles with a 20 sec denaturation step at 95 degrees C, a 30 sec annealing step at 62 degrees C and a 1 min extension step at 72 degrees C followed the initial Taq polymerase activation at 95 degrees C for 10 min.

Negative controls without added DNA template are diagnostic for cross-contamination as well as primer-dimer formation and were incorporated per loci and plate. For optimizing PCR conditions a positive control reaction of template DNA with known MLST was included.

Post-PCR processing was performed according to the standard MassCLEAVETM protocol (Sequenom). Target regions were cleaved in four reactions at positions corresponding to each of the four bases. In brief, PCR reactions were treated with 0.3 U of Shrimp alkaline phosphatase at 37 degrees C for 20 min followed by enzyme deactivation at 85 degrees C for 5 min. Subsequent C- and T-specific cleavages were mediated by two *in vitro* transcription reactions per PCR reaction in a volume of 4 microliters. In each reaction, 2 microliters of the SAP treated PCR product were incubated with 0.22 microliters of C- or T-specific transcription mix, 5 mM DTT and 0.4 microliters of T7 RNA&DNA polymerase at 37 degrees C for 2 hours followed by the addition of 0.05 microliters of RNaseA and incubation at 37 degrees C for 1 hour. Samples were diluted with 21 microliters of H$_2$O and desalted by 6 mg of SpectroCLEAN resin (Sequenom) for 10 min at room temperature. After standardized transfer onto 384 SpectroCHIPs (Sequenom) analytes are subject to MS analysis on a MALDI linear time of flight mass spectrometer (Compact Analyser, Sequenom). The instrument is equipped with a 20 Hz nitrogen laser. Automated operations on the mass spectrometer were performed using the Sequenom RT-Workstation 3.4 software package. Spectral profiles were collected in a mass range of 1100-10,000 Da using delayed ion extraction.

Exclusively positive ions were analyzed with 10 shots per spectrum. Five spectra per sample were accumulated using real time spectra quality judgment and selection. Each chip run was calibrated by a five point oligonucleotide calibrant mix (Sequenom), while each spectrum was internally calibrated by unique sets of anchor signals.

Spectra of all four cleavage reactions for a total of 100 *N. meningidits* samples were acquired and stored in the database.

*Signature sequence Identification software*

Data analysis was performed using processes described herein in a proprietary software package (Signature Sequence Identification software, Prototype, Sequenom, now iSEQ™ Version 1.0). Reference sequence sets for *in silico* cleavage pattern simulations and primer sequences for PCR amplification are provided by the user in fasta or suitable text format and uploaded into the system database as described above, while analysis specific parameters are set through the interface. Sample spectra of up to four MassCLEAVE reactions are acquired and matched against the modified sequence at the World Wide Web URL address "pubmlst.org/neisseria/ database."

*Cluster analysis*

Cluster analysis by unweighted pair matching was performed using PHYLIP (Phylogeny Inference Package) version 3.6. Distributed by the author. Department of Genetics, University of Washington, Seattle 1993.

Example 2: Comparative Sequence Analysis with Pathogen Reference Sets

*N. meningitidis* causes often severe meningococcal meningiditis and septicemia, most frequently in young children, but may as well colonize the human nasophyarynx without the onset of disease. Epidemic outbreaks of varying scale up to global pandemics require intricate genetic typing to identify case clusters. MLST was found to be the most powerful and simultaneously portable approach to keep track of the epidemic spread and has identified particular clones with apparent increased virulence (Feavers et al. 1999; Jolley et al. 2000; Murphy et al. 2003; Sullivan et al. 2005) It can now be considered the gold standard marker set for genotyping N. meningitidis.

MLST of N. meningitidis summarizes the nature of sequence variations detected in 450- 500 bp sequences of internal fragments of seven housekeeping genes (abcZ, adk, aroE, fumC, gdh, pdhC and pgm). Different sequences present within the species are assigned as distinct alleles with given numbers. For each sample alleles at each of the seven loci are identified and define its allelic profile or sequence type (ST). Major clonal complexes, STs differing in only one or two alleles, are exclusively identified based on the series of these seven integers, a seven number code, while the number of nucleotide differences between alleles is ignored (Enright and Spratt 1999; Spratt 1999). Some clonal complexes have been shown to be related to disease, while others are related to carriage of the organism (Yazdankhah et al. 2004).

*MLST by base-specific cleavage and MALDI-TOF MS*

To evaluate automated microbial typing by MALDI-TOF MS, MLST was used to type 100 isolates of Neisseria meningitidis in reference to the N. meningitidis PubMLST allele sequence database (World Wide Web URL address "pubmlst.org/neisseria," updated 10/18/2004). The database contains data for a collection of isolates that represent the total known diversity of N. meningitidis species, about 5,300 different STs with ongoing compilation.

Between 209 and 344 published alleles per locus served as reference sequence sets for MALDI-TOF MS based typing. The concept of reference sequence based peak pattern analysis is, however, applicable to nucleic acid based typing and comparative sequence analysis of haploid organisms in general. This includes a broad range of microbial agents, pathogenic and nonpathogenic species and strain types as well as antibiotic susceptibility and virulence.

The four steps of automated MALDI-TOF MS based typing are shown in Figure 1. Reference sequence sets including the gene specific primer sequences are imported into the system database to generate *in silico* peak patterns (Figure 1, Step 1). DNA sample processing follows the standard MLST protocol (World Wide Web URL address "pubmlst.org/neisseria") utilizing the sequencing primer set to amplify the internal fragments of the seven house-keeping genes. Each sequencing primer set is tagged with a T7 promotor sequence and a 10 mer tag resulting in 2 sets of PCR primers. Alternatively, primers were tagged with T7 and SP6 promotor sequences and allowed for one PCR. PCR products of the T7 tagged forward primer and the T7 tagged reverse primer or T7 and SP6 tagged primers allow for *in vitro* transcription of the sense and anti-sense strands. Resulting RNAs are subject to base-specific cleavage at C and U generating representative compomer mixtures for cleavage reactions of virtually all four cleavage bases C, U, "G" and "A". Four resulting mass spectrometric fingerprints allow for a maximum redundancy of results (Figure 1, Step 2).

Since this process relies on PCR amplification, its sensitivity can be as high as one genome copy equivalent present in the reaction vial (Ding and Cantor 2003). The amplification gain by PCR and transcription is sufficient to produce a measurable product.

For MALDI-TOF MS measurement samples are desalted by anion exchange resin treatment and dispensed on a matrix coded chip (Figure 1, Step 3). Further purification of the PCR and subsequent products is not required as left over PCR primer lack a double stranded transcription promotor region and are thus not subject to transcription and base-specific cleavage.

Finally typing results and sequence deviations are automatically assigned by the Signature Sequence Identification software tool (Sequenom) (Figure 1, Step 4).

Of the 100 N. meningitidis isolates analyzed by base-specific cleavage and MALDI-TOF MS 89 samples were automatically assigned to alleles and resulted in STs existing in the database. Three samples resulted in STs with new sequences for one of the alleles; an additional two STs were defined by known alleles, but not listed in the database and four samples revealed untypeable mixed populations. Alleles, STs and clonal complexes of all samples are listed in Table 1. The 96 typeable samples represent 38 known STs of 11 clonal complexes and five new STs.

Table 1 shows base-specific cleavage and MALDI-TOF MS typing results for 100 N. meningitidis samples. STs with corresponding clonal-complexes and alleles are listed. Two samples were of undefined ST, three samples revealed new alleles not listed in the database and four samples were identified as unresolvable mixed populations.

Table 1

| Number of samples | abcZ | adk | aroE | fumC | gdh | pdhC | pgm | ST | Clonal_ Complex |
|---|---|---|---|---|---|---|---|---|---|
| 19 | 2 | 3 | 4 | 3 | 8 | 4 | 6 | 11 | ST-11 complex/ET-37 complex |
| 7 | 4 | 10 | 2 | 5 | 38 | 11 | 9 | 275 | ST-269 complex |
| 7 | 3 | 6 | 9 | 5 | 9 | 6 | 9 | 41 | ST-41/44 complex/ Lineage 3 |
| 5 | 4 | 10 | 15 | 9 | 8 | 11 | 9 | 269 | ST-269 complex |

(continued)

| Number of samples | abcZ | adk | aroE | fumC | gdh | pdhC | pgm | ST | Clonal_ Complex |
|---|---|---|---|---|---|---|---|---|---|
| 5 | 3 | 6 | 9 | 5 | 11 | 6 | 9 | 154 | ST-41/44 complex, Lineage3 |
| 4 | 4 | 10 | 5 | 4 | 5 | 3 | 2 | 74 | ST-32 complex/ET-5 complex |
| 3 | 17 | 5 | 19 | 17 | 3 | 26 | 2 | 60 | - |
| 3 | 2 | 3 | 4 | 3 | 8 | 4 | 6 | 4 | ST-11 complex/ET-37 complex |
| 2 | 11 | 5 | 18 | 8 | 11 | 24 | 21 | 22 | ST-22 complex |
| 3 | 8 | 10 | 5 | 4 | 5 | 3 | 8 | 34 | ST-32 complex/ET-5 complex |
| 2 | 2 | 3 | 4 | 3 | 8 | 26 | 6 | 1236 | ST-11 complex/ET-37 complex |
| 2 | 4 | 10 | 5 | 40 | 6 | 3 | 8 | 259 | ST-32 complex/ET-5 complex |
| 2 | 12 | 3 | 15 | 5 | 58 | 21 | 20 | - | - |
| 1 | 2 | 7 | 6 | 17 | 16 | 18 | 8 | 167 | - |
| 1 | 20 | 6 | 63 | 9 | 9 | 11 | 2 | 284 | - |
| 1 | 2 | 18 | 15 | 55 | 24 | 11 | 10 | 1220 | - |
| 1 | 13 | 5 | 6 | 5 | 24 | 8 | 8 | 2728 | - |
| 1 | 15 | 5 | 9 | 13 | 8 | 15 | 15 | 2875 | - |
| 1 | 1 | 3 | 1 | 1 | 1 | 1 | 3 | 1 | ST-1 complex/ subgroup I/II |
| 1 | 7 | 3 | 4 | 3 | 8 | 4 | 6 | 52 | ST-11 complex/ET-37 complex |
| 1 | 11 | 5 | 18 | 15 | 11 | 24 | 21 | 1158 | ST-22 complex |
| 1 | 2 | 5 | 18 | 8 | 11 | 24 | 21 | 3915 | ST-22 complex |
| 1 | 4 | 10 | 15 | 17 | 8 | 11 | 9 | 1049 | ST-269 complex |
| 1 | 4 | 10 | 15 | 9 | 8 | 11 | 6 | 1095 | ST-269 complex |
| 1 | 4 | 10 | 15 | 9 | 8 | 5 | 9 | 1195 | ST-269 complex |
| 1 | 4 | 10 | 5 | 4 | 6 | 3 | 8 | 32 | ST-32 complex/ET-5 complex |

(continued)

| Number of samples | abcZ | adk | aroE | fumC | gdh | pdhC | pgm | ST | Clonal_ Complex |
|---|---|---|---|---|---|---|---|---|---|
| 1 | 8 | 10 | 5 | 4 | 6 | 3 | 8 | 33 | ST-32 complex/ET-5 complex |
| 1 | 4 | 10 | 12 | 4 | 6 | 3 | 8 | 1100 | ST-32 complex/ET-5 complex |
| 1 | 4 | 10 | 5 | 4 | 3 | 3 | 8 | 1130 | ST-32 complex/ET-5 complex |
| 1 | 4 | 10 | 5 | 4 | 8 | 3 | 8 | 2489 | ST-32 complex/ET-5 complex |
| 1 | 4 | 10 | 5 | 4 | 11 | 3 | 8 | 2493 | ST-32 complex/ET-5 complex |
| 1 | 4 | 10 | 5 | 4 | 5 | 3 | 8 | 2506 | ST-32 complex/ET-5 complex |
| 1 | 12 | 6 | 9 | 17 | 9 | 6 | 9 | 206 | ST-41/44 complex/ Lineage3 |
| 1 | 9 | 6 | 9 | 9 | 9 | 6 | 9 | 44 | ST-41/44 complex/ Lineage3 |
| 1 | 12 | 2 | 9 | 9 | 9 | 6 | 10 | 1216 | ST-41/44 complex/ Lineage3 |
| 1 | 9 | 6 | 36 | 9 | 9 | 6 | 2 | 1282 | ST-41/44 complex/ Lineage3 |
| 1 | 1 | 1 | 2 | 1 | 3 | 2 | 19 | 5 | ST-5 complex/ subgroupIII |
| 1 | 8 | 7 | 6 | 124 | 26 | 78 | 2 | 6 | ST-549 complex |
| 1 | 8 | 5 | 6 | 17 | 26 | 68 | 2 | 432 | ST-549 complex |
| 1 | 2 | 3 | 7 | 90 | 8 | 5 | 2 | 1094 | ST-8 complex/ Cluster A4 |
| 1 | 4 | 10 | 5 | 60 | 9 | 3 | 8 | - | ST-32 complex/ET-5 complex |
| 1 | 4 | 10 | 11 | 9 | 8 | 10 | 2 | - | ST-35 complex |
| 1 | new allele | 29 | 2 | 26 | 26 | 21 | 20 | - | - |
| 1 | 7 | 18 | 9 | 9 | 3 | new allele | 13 | - | - |

(continued)

| Number of samples | abcZ | adk | aroE | fumC | gdh | pdhC | pgm | ST | Clonal_ Complex |
|---|---|---|---|---|---|---|---|---|---|
| 1 | 7 | 5 | new allele | 13 | 3 | 128 | 15 | - | - |
| 4 | - | - | - | - | - | - | - | - | mixed populations |

Concordance between MALDI-TOF MS and dideoxy sequencing based MLST of the 96 x 7 = 672 typeable alleles amounted to 98.9% representing 665 identically identified alleles. Detailed analysis of the differences revealed that the gdh alleles of four samples were misidentified by the spectra analysis software due to the failure of two transcription and cleavage reactions or undefined additional signals, but were flagged for manual analysis and recovered by user calls. Three new alleles including an abcZ, an aroE and a pdhC allele in three different samples were identified by MALDI-TOF MS and confirmed by dideoxy sequencing. The sequences showed 99.4, 99.8 and 99.6% identity with their corresponding best matching database references abcZ285, aroE9 and pdhC207 corresponding to deviations of three, two and one base pairs.

MLST MALDI-TOF MS data acquisition of the whole set of 100 samples was accomplished in a total of four hours, which shows that the approach enables the analysis of a large number of samples in a relatively short time. Operator variables are mostly removed by liquid handling and automated data acquisition. Samples and loci can be processed in sequences of 96 within seven hours or staggered to increase the throughput and provide sufficient speed to track an ongoing epidemic. The data acquisition and analysis of a complete set of seven loci per sample can be obtained on 28 matrix patches of a 384 chip in 2.5 min. One 384 chip allows for the analysis of the seven loci in 12 samples and a negative control. Considering the analysis of 4 cleavage reactions per locus and an average amplicon length of 500-800 bp, a single mass spectrometer with a data acquisition speed of 4.5 sec/reaction can scan about 2 million bp per day, which favorably compares with standard dideoxy sequencing equipment (Kling 2003).

*Signature Sequence Identification Software Tool (iSEQ$^{TM}$ Software Version 1.0)*

Data processing was performed with the Signature Sequence Identification software (Sequenom) specifically developed to analyze base-specific cleavage patterns in comparison to a given set of reference sequences, in our case the reference sequence sets of the seven MLST house-keeping genes of N. meningitidis.

The simulation module of the software performs *in silico* cleavage reactions for the imported set of reference sequences. The resulting simulated cleavage patterns are clustered based on their distinctive peak pattern in a way that resulting clusters can be uniquely identified and distinguished from one another. For N. meningitidis all sequences within the seven reference sequence sets were differentiable in this simulation. This demonstrates a comparable discriminatory power of MLST by MALDI-TOF MS with the dideoxy sequencing gold standard.

Spectra for four cleavage reactions per sample were acquired and recalibrated against a set of unique calibration peaks derived from the reference sequence set.

In theory, samples can be identified by simply finding the best matching of the detected peak pattern with the simulated pattern of a reference sequence set. However, due to various factors, such as intensity variations in the sample spectra, peak pattern matching requires additional scoring, particularly for large and often closely related reference sequence sets such as the one used in this study. Judgment of the peak pattern matching is therefore a dynamic combination of three scores, the basic pattern matching score, a discriminating peak matching score and the distance score. The discriminating peak matching score is calculated by evaluating only a subset of simulation-derived unique reference-specific identifier signals, whereas the distance score is determined based on Euclidian distances.

To further increase the robustness, identification is performed by iteration. Initially, scores are calculated for all reference sequences and a set of best matching reference sequences are selected. Detected peak patterns are re-evaluated against this subset and scores are recalculated to re-evaluate the subset and to find an even smaller set of best matching sequences. This process continues until one sequence or several sequences with close scores that are considerably better then the rest of the sequences are found for each of the samples. Finally, the top matching reference sequence is evaluated for potential mutations and a confidence is assigned based on spectra quality, missing and additional signals as well as unknown signals, which fail any compomer or adduct assignment.

The graphical user interface of the Signature Sequence Identification software (Sequenom) displays typing results, confidence levels and sequence deviations automatically in a tabulated report (Figure 2). An interactive details window is available for manual analysis of each of the samples. Several report functions like FASTA outputs of new reference sequences or distance matrices of simulated and acquired data allow for phylogenetic analysis and further evaluation of the data.

Data are stored in a database and may be analyzed either by local or remote access. Molecular typing by base-specific cleavage and MALDI-TOF MS is therefore amenable to standardization, global data comparability and electronic data portability of nucleotide data or corresponding mass peak patterns.

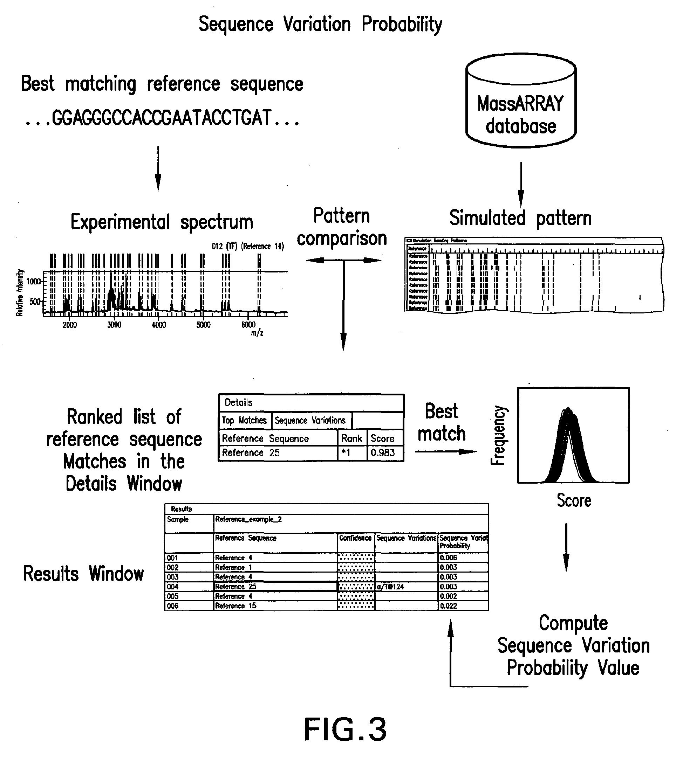

Figure 3 illustrates an example of a process used in identification and probability assignment. Acquired spectra (up to four per reaction) are correlated against theoretical peak pattern derived from an input reference sequence set as defined by the user. A scoring scheme is used to measure the degree of similarity. Matching reference sequences ranked according to the computed score. The reference sequence with the highest score is selected for further statistical analysis. The sequence variation probability accesses the quality of the match between the top matching reference pattern and the sample pattern and expresses the likelihood of any unexplained sequence variation in the selected best matching reference sequence.

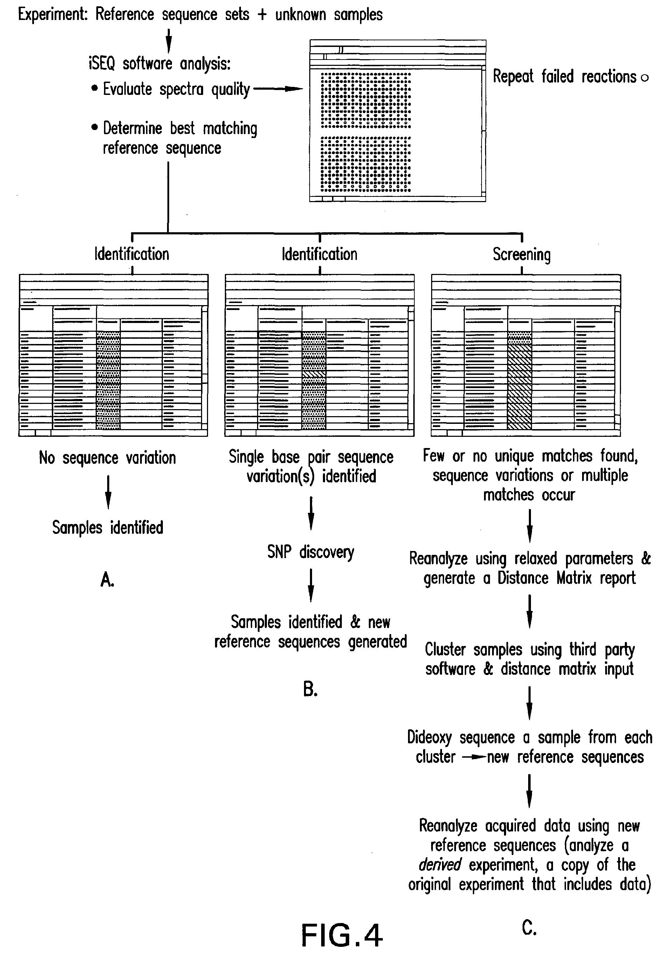

Figure 4 illustrates an example of different analysis options utilized with the different parameter sets. The first option identifies all samples as present in the reference set, the second analysis option includes a SNP analysis and the third option uses clustering for analysis and sample grouping (relaxed parameters).

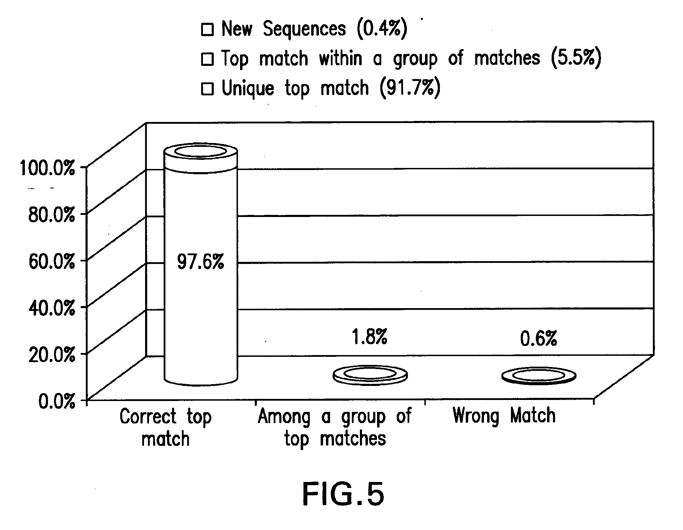

The typing statistics of the analysis software on the 96 typeable *N. meningitidis* samples is summarized in Figure 5. For 97.6% of a total of 672 alleles the software automatically identified the correct top matching reference sequence in agreement with dideoxy sequencing. Of these 91.7% were uniquely identified, 5.5% were listed as top matching reference among a group of homologous references and 0.4% were identified as new sequences extending the existing reference set. For 1.8% of the alleles the correct matching reference was listed among a group of top matching references and typing required manual selection of the best match. This was mainly due to the failure of one of the four cleavage reactions. Only 0.6% of the alleles, four gdh alleles out of a total of 672 alleles, were assigned to the wrong sequence, but correctly identified by user calls as stated above.

*Single base pair mutation detection*

New alleles were identified by a combination of the identification algorithm with a MALDI-TOF MS specific SNP Discovery algorithm (Bocker 2003, patent number). Single base pair differences between an assigned closest matching sequence and the correct sample sequence affect one or more cleavage products of the compomer mixtures in the cleavage reactions and show up as a deviation between the *in silico* derived and the detected sample spectrum. The SNP Discovery algorithm identifies these peak pattern changes and utilizes the observations to detect, identify and localize the single base pair changes.

Figure 6 exemplifies the detection of a novel aroE9 modification with a C to T single base deviation at position 443. Banding patterns derived from the reference sequence are used to illustrate the difference between the *in silico* pattern of aroE9 and the detected sample pattern. The T-specific reaction of the forward RNA transcript (Figure 6A) shows a missing signal at 8957.9 Da in comparison to the banding pattern. The signal represents a cleavage product that is localized at position 439 of the amplicon with a composition A8C10G9T1. A new signal appears at 7343.5 Da with a composition of A8C8G6T1. The deviation between the missing and the additional compomer can be explained by a substitution of a C with a T at position 443 and the introduction of a cleavage base at this position, which leads to the detected compomer at 7343.5 Da and a compomer C1 G3T1 at 1650.0 Da (data not shown). The latter is detected as a silent non informative signal being identical to two compomers of the same nucleotide composition derived from sequence stretches somewhere else in the reference. The T-cleavage reaction of the reverse RNA transcript confirms the observation (Figure 6B). The corresponding compomer A1 C5G3T1 at 3136.0 Da is missing, while an additional signal at 3120.0 Da with the composition A2C5G2T1 reflects the observed C to T change by the complementary event G to A. Additional confirmation is gained in the C-specific cleavage reaction of the forward RNA transcript from an additional signal at 2010.0 Da of composition C1G4T1. The signal is the result of the loss of the C-cleavage site in compomer C1 G3 at position 432 due to the C to T change. The corresponding missing signals of the two combined fragments are silent and below the mass range of detection. The C-specific cleavage reaction of the reverse RNA transcript does not add any additional information as the corresponding mass of the affected compomer GC is <1000 Da and thus out of the mass range of detection. Low mass range signals are the result of nucleic acid mono-, di- and trimers overlayed by matrix contamination and therefore discarded.

In conclusion, the C to T mismatch between the best matching reference sequence aroE9 and the sequence of the sample was detected by MALDI-TOF MS with a redundancy of two missing and three additional signals.

In addition, the SNP Discovery algorithm identified deviations in consensus sequence stretches, which were used for the missing sequence information between the MLST sequencing primer and the available reference sequences. Unlike standard dideoxy-sequencing based MLST, where the first 5-10 base pairs following the primer region are not resolved and the sequence reads require trimming prior to database query, base-specific cleavage and MALDI-TOF MS MLST analyzes the full length transcript starting at the ggg-transcription start of the T7-polymerase and at gga-transcription start of the SP6-polymerase. Thus, sequence information of gene specific primer regions of the forward as well as the reverse primer and a consensus sequence for the missing information of the trimmed sequence regions were irícluded in the analysis.

Allele sequence differences in the consensus regions were again identified by peak pattern deviations between the expected peak pattern from the *in silico* analysis and the detected sample spectrum. Results were confirmed by dideoxy sequencing and are available in Supplemental Table 1. Identified sequence deviations showed 100% homology within the alleles and maintained discrimination between alleles.

*Simulation*

A computational simulation tool systematically introduced all possible single nucleotide mutations in each sequence of the given MLST reference sequence sets and categorized resulting sequence variations according to the ability to detect them using four base-specific cleavage reactions and the SNP Discovery algorithm. Mass signals in a range of 1100 - 8000 Da were considered and a mass resolution (m/□m) of 600 was assumed, values routinely achieved with MALDI-TOF MS. The results summarized in Table 2 demonstrate that for the total of the seven reference sequence sets of this study 99.0% of all possible single nucleotide changes are detectable by base-specific cleavage and MALDI-TOF MS. Overall slightly higher detection rates are obtained for substitutions (99.4%), which are more likely to occur in typing approaches of house-keeping gene regions like MLST, when compared to detection rates for deletions (98.9%) and insertions (98.7%). This can be explained by the fact, that substitutions can lead to up to 10 observations (five missing and five additional signals), whereas insertions/deletions can lead to a maximum of nine observations in the sample spectra.

Table 2 shows simulated single base pair mutation detection rates by base-specific cleavage and MALDI-TOF MS for the MLST reference sequence sets of N. meningiditis.

Table 2

| Amplicon Set | Insertions | Deletions | Substitutions | Total # of SNPs |
|---|---|---|---|---|
| abcZ | 99.3 ±0.37 | 99.6±0.29 | 99.8±0.22 | 99.7±0.22 |
| adk | 98.7±0.57 | 98.8±0.58 | 99.6±0.18 | 99.1 ±0.40 |
| aroE | 98.3±0.74 | 98.9±0.45 | 99.3±0.28 | 99.0±0.32 |
| fumC | 98.8±0.63 | 98.4±0.53 | 98.9±0.48 | 98.6±0.48 |
| gdhC | 98.1 ±0.61 | 98.0±0.55 | 99.1 ±0.34 | 98.4±0.42 |
| pdhC | 97.9±0.84 | 98.4±0.64 | 99.1 ±0.32 | 98.6±0.48 |
| pgm | 99.8±0.50 | 99.8±0.39 | 99.9±0.20 | 99.8±0.32 |
| **Total** | **98.7±0.68** | **98.9±0.65** | **99.4±0.39** | **99.0 ±0.54** |

*Cluster analysis*

Detected mass signals of the four cleavage reactions can be used to characterize a defined fingerprint of a sample as an array of peak positions in combination with the intensities of the signals converted to integers. This allows for the display of a mass spectrometric fingerprint as a band-based pattern. A collection of the integers can be described as a matrix. The linkage of the corresponding samples can be analyzed by Euclidean distance (ED) and displayed as a dendrogram. A list of spectra that contain similar fingerprints and thus similar peak positions and intensities are described as a cluster, which displays similarities among the objects of the set without the need for the assignment of a known reference sequence. Cluster analysis of mass peak patterns allows for the rapid high-throughput analysis of large sample sets, when only limited numbers of reference sequences are available as needed for the identification of new informative marker sets.

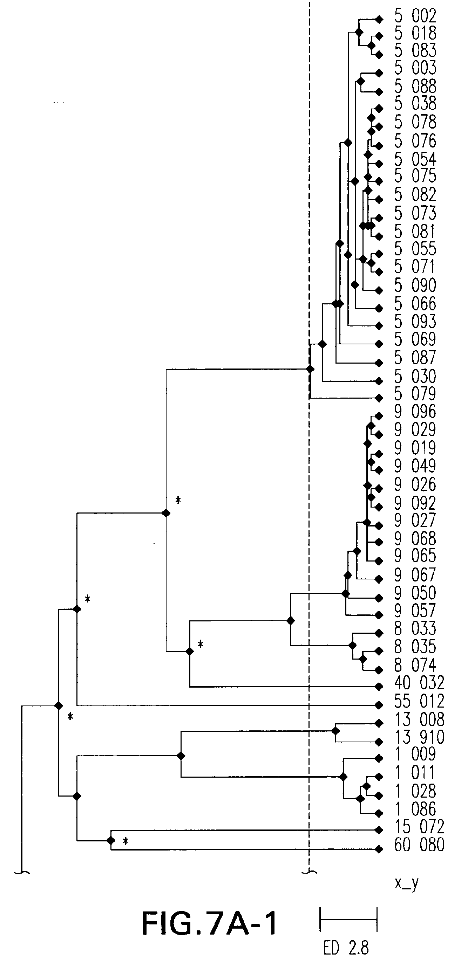

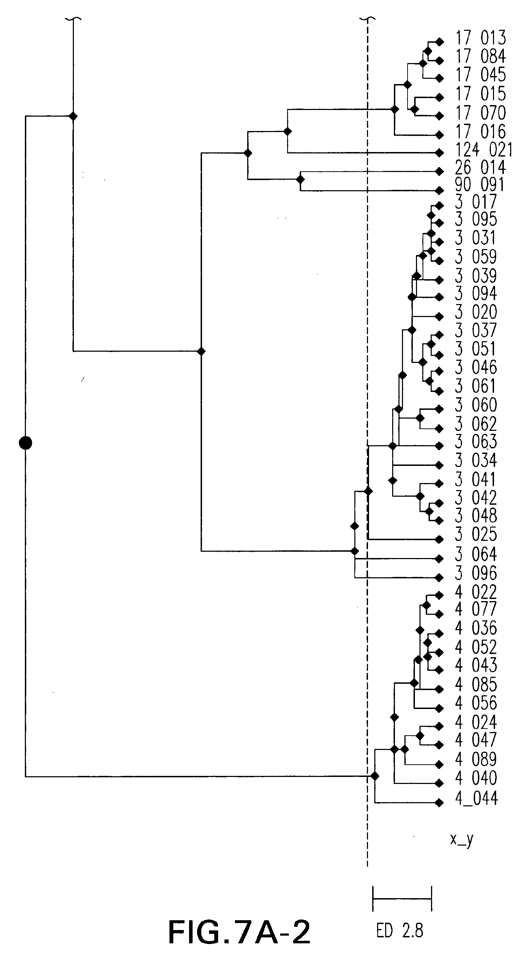

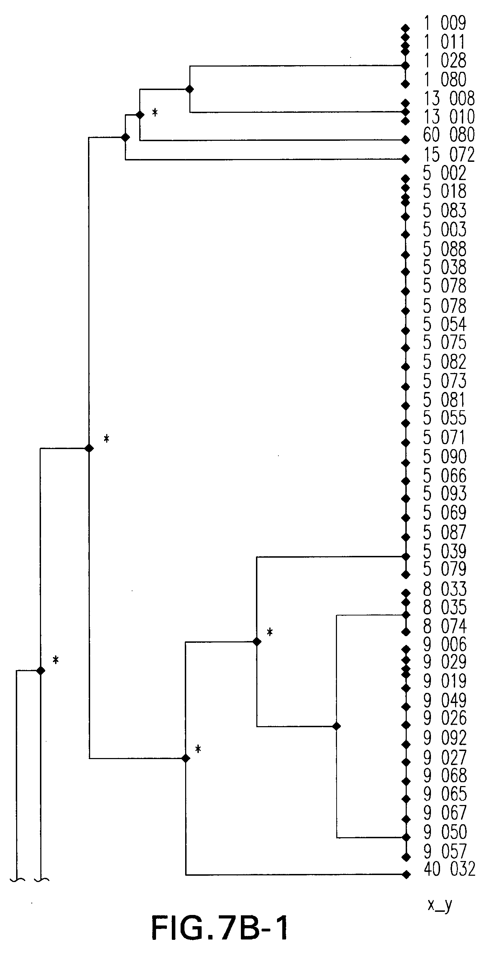

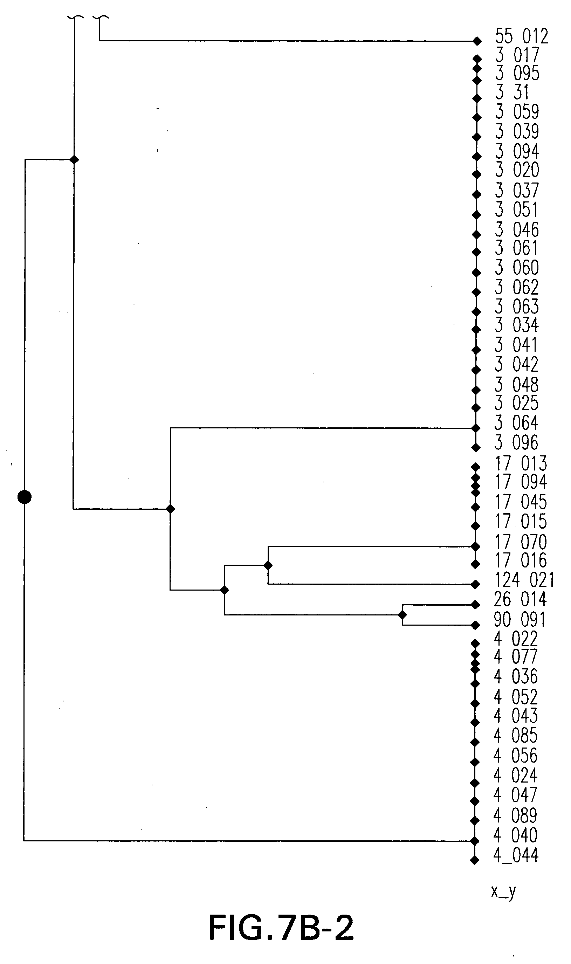

A cluster analysis using the Unweighted Pair Group Method (UPGMA) on MALDI-TOF MS fingerprints for the four cleavage reactions of 15 fumC alleles from 89 samples is demonstrated in Figure 7A. This dendrogram is consistent with the dendrogram produced by direct comparison of the primary sequences (Figure 7B). This demonstrates equal resolution of the sample set. An ED of 2.8 was found to be the similarity cut-off for samples with 100% sequence identity. All samples grouped within their corresponding alleles. Spectral patterns and primary sequences of the alleles fell into two major groups of identical clades with alleles 1, 5, 8, 9, 13, 15, 40, 55 and 60 in one clade and alleles 3, 4, 17, 26, 90 and 124 forming the other. A symmetry difference of 10 was obtained by the count of partitions present in one, but not in the other tree. Differences were found within the first group of clades, while there were no differences in the second.

Overall cluster analysis of base-specific cleavage mass signal patterns show clearly distinguishable clusters reflecting differences between alleles and their grouping by primary sequence analysis. (Figure 7)

*Reproducibility*

A random set of 23 samples representing 12 STs was chosen to assess the reproducibility of MALDI-TOF MS based typing on two mass spectrometers at the collaborating centers. Samples were processed in four runs on different days according to the standard protocol. Data for three of the four runs were acquired at Sequenom, Inc., San Diego, and for one of the four runs at the Health Protection Agency, London, UK. Results for the set of 644 expected data points are summarized in Table 3. 638 products were successfully amplified, transcribed and cleaved. Six reactions failed PCR or Post-PCR processing with four drop outs on the second day of processing and one drop out on day three and four, leaving 99.1 % of the data (638/644) for reproducibility analysis. Of these 99.1 % (632/638) were assigned to the correct allele. Six data points were ambiguously identified by multiple matching alleles including the correct allele with the option for a correct manual user call. Among these, one sample was identified as a mixture of two abcZ alleles resulting in the assignment of both alleles for the four repeated data points.

Overall 98.1 % (152/155) of the repeated typing events were reproducible. This reflects the stability of the molecular typing approach manifested in the specificity of the obtained MALDI-TOF MS patterns.

The presented system enables automated reference sequence based identification and characterization of DNA or RNA sequences and is suited to screen multiple loci in parallel as needed in polyphrasic approaches or MLST. Resulting digital data are both highly accurate and portable. Compared to traditional methods for analyzing PCR amplicons, including gel electrophoresis and dideoxy sequencing, mass spectrometry combines 384-well liquid handling robotics for PCR and post-PCR processing with mass accuracy and speed of a MALDI-TOF MS analyzer. Automated data analysis avoids time consuming trace analysis and sequence alignments. As opposed to dideoxy sequencing, band compression artifacts by repeats of single nucleotides in a sequence are not an issue and do not cause misreading of the sequence.

*Conclusions*

Reproducible large-scale monitoring of microbes, especially of human pathogens, including virulent, emerging and antibiotic resistant strains, is increasingly important in today's world of global transport and requires technologies that offer automated, less labor intensive and faster alternatives to replace traditional epidemiological typing methods. The genotypic MALDI-TOF MS based typing tool described here provides a standardized, accurate, automated, high-throughput alternative for microbial identification and characterization. Validation of the system by processing and analysis of a stable set of MLST markers in 100 isolates of *N. meningitidis* has shown typeability, reproducibility and concordance as well as a discriminatory power equal to standard dideoxy sequencing. The technology has the ability to type any pathogen or microbe with the same ease of use and data interpretation, provided that at least one stable 500-800 bp reference sequence is available. This technology is of importance as microbial genome sequencing projects constantly increase the availability of whole genome sequences for clinically relevant microorganisms and trigger the comparisons of selected signature sequences to develop improved diagnostic typing assays.

In addition, maintaining databases for the molecular characterization of microbes is an ongoing process. New isolates might develop over time or isolates might be absent or poorly represented in the database. The better the species is represented by the corresponding database, the less manual steps are involved in the analysis, which clearly emphasizes the value of the system for automated sample characterization in a diagnostic reference laboratory.

Stability of the reaction plates allows for their storage and shipment to a central MALDI-TOF MS facility. The approach enables the comparison of processed plates and the portability of data between different reference laboratories without exchanging strains. The technology ideally is suited for microbial testing on multiple regions supporting MLST typing schemes and polyphasic taxonomic approaches.

*Cited Documents*

Bocker, S. 2003. SNP and mutation discovery using base-specific cleavage and MALDI-TOF mass spectrometry. Bioinformatics 19 Suppl 1: i44-53.

Clarke, S.C. 2002. Nucleotide sequence-based typing of bacteria and the impact of automation. Bioessays 24: 858-862.

Ding, C. and C.R. Cantor. 2003. Direct molecular haplotyping of long-range genomic DNA with M1-PCR. Proc Natl Acad Sci U S A 100: 7449-7453.

Enright, M.C. and B.G. Spratt. 1999. Multilocus sequence typing. Trends Microbiol 7: 482-487.

Feavers, I.M., S.J. Gray, R. Urwin, J.E. Russell, J.A. Bygraves, E.B. Kaczmarski, and M.C. Maiden. 1999. Multilocus sequence typing and antigen gene sequencing in the investigation of a meningococcal disease outbreak. J Clin Microbiol 37: 3883-3887.

Garaizar, J., A. Rementeria, and S. Porwollik. 2006. DNA microarray technology: a new tool for the epidemiological typing of bacterial pathogens? FEMS Immunol Med Microbiol 47: 178-189.

Jolley, K.A., J. Kalmusova, E.J. Feil, S. Gupta, M. Musilek, P. Kriz, and M.C. Maiden. 2000. Carried meningococci in the Czech Republic: a diverse recombining population. J Clin Microbiol 38: 4492-4498.

Kling, J. 2003. Ultrafast DNA sequencing. Nat Biotechnol 21: 1425-1427.

Lefmann, M., C. Honisch, S. Bocker, N. Storm, F. von Wintzingerode, C. Schlotelburg, A. Moter, D. van den Boom, and U.B. Gobel. 2004. Novel mass spectrometry-based tool for genotypic identification of mycobacteria. J Clin Microbiol 42: 339-346.

Maiden, M.C. 2006. Multilocus Sequence Typing of Bacteria. Annu Rev Microbiol.

Maiden, M.C., J.A. Bygraves, E. Feil, G. Morelli, J.E. Russell, R. Urwin, Q. Zhang, J. Zhou, K. Zurth, D.A. Caugant, I.M. Feavers, M. Achtman, and B.G. Spratt. 1998. Multilocus sequence typing: a portable approach to the identification of clones within populations of pathogenic microorganisms. Proc Natl Acad Sci U S A 95: 3140-3145.

Murphy, K.M., K.A. O'Donnell, A.B. Higgins, C. O'Neill, and M.T. Cafferkey. 2003. Irish strains of Neisseria meningitidis: characterisation using multilocus sequence typing. Br J Biomed Sci 60: 204-209.

Olive, D.M. and P. Bean. 1999. Principles and applications of methods for DNA-based typing of microbial organisms. J Clin Microbiol 37: 1661-1669.

Pfaller, M.A. 1999. Molecular epidemiology in the care of patients. Arch Pathol Lab Med 123: 1007-1010.

Spratt, B.G. 1999. Multilocus sequence typing: molecular typing of bacterial pathogens in an era of rapid DNA sequencing and the internet. Curr Opin Microbiol 2: 312-316.

Stanssens, P., M. Zabeau, G. Meersseman, G. Remes, Y. Gansemans, N. Storm, R. Hartmer, C. Honisch, C.P. Rodi, S. Bocker, and D. van den Boom. 2004. High-throughput MALDI-TOF discovery of genomic sequence polymorphisms. Genome Res 14: 126-133.

Sullivan, C.B., M.A. Diggle, and S.C. Clarke. 2005. Multilocus sequence typing: Data analysis in clinical microbiology and public health. Mol Biotechnol 29: 245-254.

Urwin, R. and M.C. Maiden. 2003. Multi-locus sequence typing: a tool for global epidemiology. Trends Microbiol 11: 479-487.

van Belkum, A. 2003. High-throughput epidemiologic typing in clinical microbiology. Clin Microbiol Infect 9: 86-100.

von Wintzingerode, F., S. Bocker, C. Schlotelburg, N.H. Chiu, N. Storm, C. Jurinke, C.R. Cantor, U.B. Gobel, and D. van den Boom. 2002. Base-specific fragmentation of amplified 16S rRNA genes analyzed by mass spectrometry: a tool for rapid bacterial identification. Proc Natl Acad Sci U S A 99: 7039-7044.

Yazdankhah, S.P., P. Kriz, G. Tzanakaki, J. Kremastinou, J. Kalmusova, M. Musilek, T. Alvestad, K.A. Jolley, D.J. Wilson, N.D. McCarthy, D.A. Caugant, and M.C. Maiden. 2004. Distribution of serogroups and genotypes among disease-associated and carried isolates of Neisseria meningitidis from the Czech Republic, Greece, and Norway. J Clin Microbiol 42: 5146-5153.

## Claims

1. A process for identifying or determining the presence or absence of a target nucleotide sequence in a sample, which comprises:

   a. generating a sample set of mass signals for a sample set of nucleic acid fragments by

   (i) contacting a sample DNA with a primer;
   (ii) extending the primer to form a primer product;
   (iii) transcribing the primer product to form a primer product RNA;
   (iv) contacting the primer product RNA with a specific cleavage agent to form cleavage products; and
   (v) preparing a set of mass signals from the cleavage products by mass spectrometric analysis;

   b. generating a reference set of mass signals for a reference set of nucleic acid fragments by

   (b')

   (i) contacting a reference DNA with a primer;
   (ii) extending the primer to form a primer product;
   (iii) transcribing the primer product to form a primer product RNA;
   (iv) contacting the primer product RNA with a specific cleavage agent to form cleavage products; and

(v) preparing a set of mass signals from the cleavage products by mass spectrometric analysis, or

(b")

(i) virtually contacting the reference nucleotide sequence with a specific cleavage agent, and
(ii) preparing a set of mass signals from the cleavage products;

c. identifying reference or references with matching peak patterns between the sample set of mass signals and a reference set of mass signals and scoring matching peak patterns, wherein scoring is calculated from an overall score determined by

(i) a bitmap score calculated by comparing reference peaks with detected peaks,
(ii) a discriminating feature matching score calculated by evaluating a subset of peaks that can discriminate one reference from another or one set of references from another, and
(iii) a distance score calculated based on Euclidian distance of the identified feature vectors to all reference feature vectors;

d. selecting a top-ranked subset of matching peak patterns between the sample set of mass signals and the reference set of mass signals based on the overall scores determined in step (c);
e. iteratively re-scoring matching peak patterns in the subset and identifying one or more top-ranked matching peak patterns; and
f. determining the presence or absence of the target nucleotide sequence in the sample by the match between the one or more top-ranked matching peak patterns.

2. The process of claim 1, wherein the reference peak pattern is determined by:

aligning by mass all the reference peaks within a set;
representing each reference peak with a peak intensity;
calculating the distance between each peak intensity within the reference set; and
clustering reference peaks to generate a minimum set of cleavage reactions.

3. The process of claim 2, wherein the peak intensity is determined by:

acquiring and filtering a subset of mass spectra;
grouping one or more sets of peaks together;
calculating the group intensity using the heights and masses for each peak in the group; and normalizing the group intensities.

4. The process of claim 2, wherein the clustering is determined by:

identifying peaks present in one set of references but absent in other sets;
sub-clustering until each cluster has only one sequence or a set of indistinguishable sequences;
summing up the intensities of the peaks in the sub-clusters; and
evaluating the differences between sub-clusters.

5. The process of claim 1, wherein the sample matching peak patterns is further calibrated by:

matching the sample peaks to reference peaks within a certain mass window;
removing sample peak outliners by evaluating an overall deviation pattern;
selecting high intensity peaks which are evenly distributed across the whole mass range as anchor peaks; and
comparing the number of peaks matching a preselected set of peaks or anchor peak sets from the reference peak patterns

6. The process of claim 5, wherein the peak intensities are adjusted by:

fitting peak intensities to a standard profile of different mass ranges;
fitting the center mass regions of the profile to a Gaussian curve; and
revising the intensities for all detected peaks with the adjustment.

7. The process of claim 5, wherein the anchor peaks are calibrated by their mass and spectrum quality.

8. The process of claim 1, which comprises identifying potential sequence variations in the nucleotide sequence of the one or more top-ranked matching peak patterns of the reference set and/or the sample set.

9. The process of claim 1, which comprises assigning a confidence value to the match between the one or more top-ranked matching peak patterns.

10. The process of claim 1, wherein the reference set of mass signals is derived from cleavage products resulting from a reference nucleic acid virtually contacted with the specific cleavage agent.

11. The process of claim 10, wherein the reference set of mass signals is subject to clustering.

12. The process of claim 10, wherein each of the reference sets is compared to the sample set.

13. The process of claim 1, wherein the bitmap score is calculated by comparing intensities of detected and individual reference peak patterns weighted by reference peak intensity.

14. The process of claim 1, further comprising a peak pattern identity score from the sum of the matched peak intensities, missing and additional peak intensities, silent missing peak intensities and silent additional peak intensities for the reference peak patterns.

15. The process of claim 1, further comprising evaluating each sample against all the references for an adjusted peak change which is a summed intensity of missing peaks and additional peaks due to spectrum qualities and adjusted by unknown peaks and adduct peaks to determine variability of the sample from the reference.

16. The process of claim 15, further comprising evaluating the confidence of the subset of matching peaks patterns by determining a density distribution between scores and adjusted peak changes.

**Patentansprüche**

1. Verfahren zur Identifizierung oder Bestimmung des Vorhandenseins oder Fehlens einer Zielnukleotidsequenz in einer Probe, das umfasst:

   a. Generierung eines Probensets von Massesignalen für ein Probenset von Nukleinsäurefragmenten mittels

      (i) In-Kontakt-Bringen einer Proben-DNA mit einem Primer;
      (ii) Verlängerung des Primers zur Bildung eines Primerprodukts;
      (iii) Transkription des Primerprodukts zur Bildung einer Primer-Produkt-RNA;
      (iv) In-Kontakt-Bringen der Primer-Produkt-RNA mit einem spezifischen Spaltungsmittel, um Spaltprodukte zu bilden; und
      (v) Erstellen eines Sets von Massesignalen aus den Spaltprodukten mittels massenspektrometrischer Analyse;

   b. Generierung eines Referenzsets von Massesignalen für ein Referenzset von Nukleinsäurefragmenten mittels

     (b')

        (i) In-Kontakt-Bringen einer Referenz-DNA mit einem Primer;
        (ii) Verlängerung des Primers zur Bildung eines Primerprodukts;
        (iii) Transkription des Primerprodukts zur Bildung einer Primer-Produkt-RNA;
        (iv) In-Kontakt-Bringen der Primer-Produkt-RNA mit einem spezifischen Spaltungsmittel, um Spaltprodukte zu bilden; und
        (v) Bereitstellen eines Sets von Massesignalen aus den Spaltprodukten mittels massenspektrometrischer Analyse oder

     (b")

(i) virtuelles In-Kontakt-Bringen der Referenznukleotidsequenz mit einem spezifischen Spaltungsmittel, und

(ii) Erstellen eines Sets von Massesignalen aus den Spaltprodukten;

c. Identifizierung einer Referenz oder von Referenzen mit übereinstimmenden Peakmustern zwischen dem Probenset von Massesignalen und dem Referenzset von Massesignalen und Auswertung der übereinstimmenden Peakmuster, wobei die Auswertung ausgehend von einem Gesamtwert berechnet wird, der bestimmt wird mittels

(i) einem Bitmap-Wert, der über den Vergleich der Referenzpeaks mit den nachgewiesenen Peaks berechnet wird;

(ii) einem unterscheidenden Merkmals-Abgleichungs-Wert, der über die Evaluierung eines Subsets von Peaks berechnet wird, das eine Referenz von einer anderen oder ein Set von Referenzen von einem anderen unterscheiden kann, und

(iii) einem Distanz-Wert, der basierend auf dem euklidischen Abstand der identifizierten Merkmalsvektoren zu allen Referenzmerkmalsvektoren berechnet wird;

d. Auswahl eines an oberster Stelle stehenden Subsets von übereinstimmenden Peakmustern zwischen dem Probenset von Massesignalen und dem Referenzset von Massesignalen basierend auf den im Schritt (c) bestimmten Gesamtwerten,

e. iterative nochmalige Auswertung der übereinstimmenden Peakmuster in dem Subset und Identifizierung eines oder mehrerer an oberster Stelle stehender übereinstimmender Peakmuster, und

f. Bestimmung des Vorhandenseins oder Fehlens der Zielnukleotidsequenz in der Probe durch den Abgleich zwischen dem/den einen oder mehreren übereinstimmenden Peakmustern.

2. Verfahren nach Anspruch 1, wobei das Referenzpeakmuster bestimmt wird mittels:

Ausrichtung mittels Masse aller Referenzpeaks in einem Set;
Darstellung eines jeden Referenzpeaks mit einer Peakintensität;
Berechnung der Distanz zwischen jeder Peakintensität innerhalb des Referenzsets und
Clustern der Referenzpeaks, um ein minimales Set an Spaltreaktionen zu erzeugen.

3. Verfahren nach Anspruch 2, wobei die Peakintensität bestimmt wird mittels:

Erlangung und Filtrierung eines Subsets von Massenspektren;
Gruppierung einer oder mehrerer Sets von Peaks;
Berechnung der Gruppenintensität unter Verwendung der Höhen und
Massen für jeden Peak in der Gruppe und Normalisierung der Gruppenintensitäten.

4. Verfahren nach Anspruch 2, wobei das Clustern bestimmt wird mittels:

Identifizierung von Peaks, die in einem Set von Referenzen vorhanden sind, jedoch in anderen Sets fehlen;
Sub-Clustern, bis jedes Cluster nur eine Sequenz oder ein Subset von nichtunterscheidbaren Sequenzen hat;
Aufsummierung der Intensitäten der Peaks in den Sub-Clustern, und Auswertung der Unterschiede zwischen den Sub-Clustern.

5. Verfahren nach Anspruch 1, wobei die Proben-übereinstimmenden Peakmuster weiterhin kalibriert werden mittels

Abgleichung der Probenpeaks mit Referenzpeaks innerhalb eines bestimmten Massefensters;
Entfernung von außerhalb liegenden Probenpeaks mittels Auswertung eines allgemeinen Abweichungsmusters;
Auswahl von Hochintensitätspeaks, die gleichmäßig über den gesamten Massebereich verteilt sind, als Ankerpeaks; und
Vergleich der Anzahl von Peaks, die mit einem vorselektierten Set von Peaks oder Ankerpeaksets der Referenzpeakmuster übereinstimmen.

6. Verfahren nach Anspruch 5, wobei die Peakintensitäten angepasst werden mittels:

Einpassen von Peakintensitäten in ein Standardprofil von verschiedenen Massebereichen;

Einpassen der zentralen Massebereiche des Profils in eine Gauß-Kurve und Überarbeitung der Intensitäten für alle bestimmten Peaks mit der Anpassung.

**7.** Verfahren nach Anspruch 5, wobei die Ankerpeaks über ihre Masse und Spektrumqualität kalibriert werden.

**8.** Verfahren nach Anspruch 1, umfassend die Identifizierung von möglichen Sequenzvariationen in der Nukleotidsequenz des einen oder der mehreren an oberster Stelle stehenden übereinstimmenden Peakmusters/n des Referenzsets und/oder des Probensets.

**9.** Verfahren nach Anspruch 1, umfassend die Zuweisung eines Konfidenzwerts an die Übereinstimmung zwischen dem einen oder der mehreren an oberster Stelle stehenden übereinstimmenden Peakmuster(n).

**10.** Verfahren nach Anspruch 1, wobei das Referenzset an Massesignalen von Spaltprodukten abstammt, die aus den Referenznukleinsäuren stammen, die virtuell mit einem spezifischen Spaltmittel in Kontakt gebracht wurden.

**11.** Verfahren nach Anspruch 10, wobei das Referenzset an Massesignalen geclustert wird.

**12.** Verfahren nach Anspruch 10, wobei jedes Referenzset mit dem Probenset verglichen wird.

**13.** Verfahren nach Anspruch 1, wobei der Bitmap-Wert ausgerechnet wird, indem die Intensitäten der ermittelten und individuellen Referenzpeakmuster, gewichtet nach der Referenzpeakintensität, verglichen werden.

**14.** Verfahren nach Anspruch 1, weiterhin umfassend einen Peakmusterintensitätswert aus der Summe der übereinstimmenden Peakintensitäten, der fehlenden und zusätzlichen Peakintensitäten, der stummen fehlenden Peakintensitäten und der stummen zusätzlichen Peakintensitäten für die Referenzpeakmuster.

**15.** Verfahren nach Anspruch 1, weiterhin umfassend die Auswertung einer jeden Probe gegenüber all den Referenzen für eine angepasste Peakänderung, die eine summierte Intensität der aufgrund der Spektrumqualitäten fehlenden Peaks und zusätzlichen Peaks ist und mittels unbekannten Peaks und Addukt-Peaks angepasst ist, um die Variabilität der Probe im Vergleich zur Referenz zu bestimmen.

**16.** Verfahren nach Anspruch 15, weiterhin umfassend die Auswertung der Konfidenz des Subsets der übereinstimmenden Peakmuster durch die Bestimmung einer Dichteverteilung zwischen den Werten und den angepassten Peakveränderungen.

**Revendications**

**1.** Procédé d'identification ou de détermination de la présence ou de l'absence d'une séquence nucléotidique cible dans un échantillon, qui comprend :

    a. la génération d'un ensemble de signaux de masse des échantillons pour un ensemble de fragments d'acide nucléique des échantillons par

        (i) la mise en contact d'un ADN d'échantillon avec une amorce ;
        (ii) l'extension de l'amorce pour former un produit d'amorce ;
        (iii) la transcription du produit d'amorce pour former un ARN de produit d'amorce ;
        (iv) la mise en contact de l'ARN de produit d'amorce avec un agent de clivage spécifique pour former des produits de clivage ; et
        (v) la préparation d'un ensemble de signaux de masse à partir des produits de clivage par analyse de spectrométrie de masse ;

    b. la génération d'un ensemble de signaux de masse de références pour un ensemble de fragments d'acide nucléique de références par

        (b')

            (i) la mise en contact d'un ADN de référence avec une amorce ;

(ii) l'extension de l'amorce pour former un produit d'amorce ;

(iii) la transcription du produit d'amorce pour former un ARN de produit d'amorce ;

(iv) la mise en contact de l'ARN de produit d'amorce avec un agent de clivage spécifique pour former des produits de clivage ; et

(v) la préparation d'un ensemble de signaux de masse à partir des produits de clivage par analyse de spectrométrie de masse, ou

(b")

(i) la mise en contact virtuelle de la séquence nucléotidique de référence avec un agent de clivage spécifique, et

(ii) la préparation d'un ensemble de signaux de masse à partir des produits de clivage ;

c. l'identification de la référence ou des références avec des profils de pics correspondants entre l'ensemble des signaux de masse des échantillons et un ensemble de signaux de masse de références et la notation des profils de pics correspondants, où la notation est calculée à partir d'une note globale déterminée par

(i) une note de bitmap calculée en comparant les pics des références avec les pics détectés,

(ii) une note de correspondance des caractères discriminatoires calculée en évaluant un sous-ensemble de pics qui peut discriminer une référence d'une autre ou un ensemble de références d'un autre, et

(iii) une note de distance calculée en se basant sur la distance euclidienne des vecteurs des caractères identifiés par rapport à tous les vecteurs des caractères des références ;

d. la sélection d'un sous-ensemble le mieux noté des profils de pics correspondants entre l'ensemble des signaux de masse des échantillons et l'ensemble des signaux de masse des références en se basant sur les notes globales déterminées dans l'étape (c) ;

e. la renotation itérative des profils de pics correspondants dans le sous-ensemble et l'identification d'un ou de plusieurs profils de pics correspondants les mieux notés ; et

f. la détermination de la présence ou de l'absence de la séquence nucléotidique cible dans l'échantillon par la correspondance entre les un ou plusieurs profils de pics correspondants les mieux notés.

2. Procédé selon la revendication 1, dans lequel le profil de pic de référence est déterminé par :

l'alignement par masse de tous les pics des références au sein d'un ensemble ;

la représentation de chaque pic de référence avec une intensité de pic ;

le calcul de la distance entre chaque intensité de pic au sein de l'ensemble des références ; et

le regroupement des pics des références pour générer un ensemble minimal de réactions de clivage.

3. Procédé selon la revendication 2, dans lequel l'intensité de pic est déterminée par :

l'acquisition et la filtration d'un sous-ensemble de spectres de masse ;

le regroupement d'un ou de plusieurs ensembles de pics ensemble ;

le calcul de l'intensité de groupe en utilisant les hauteurs et les masses pour chaque pic dans le groupe ; et la normalisation des intensités des groupes.

4. Procédé selon la revendication 2, dans lequel le regroupement est déterminé par :

l'identification des pics présents dans un ensemble de références mais absents dans d'autres ensembles ;

la formation de sous-groupes jusqu'à ce que chaque groupe ne comporte qu'une séquence ou un ensemble de séquences impossibles à distinguer ;

le récapitulatif des intensités des pics dans les sous-groupes ; et

l'évaluation des différences entre les sous-groupes.

5. Procédé selon la revendication 1, dans lequel les profils de pics correspondants des échantillons sont en outre étalonnés par :

la correspondance des pics des échantillons avec les pics des références au sein d'une certaine fenêtre de masses ;

l'élimination des pics limites des échantillons en évaluant un profil de déviation globale ;
la sélection des pics de haute intensité qui sont distribués uniformément dans toute la plage des masses comme les pics d'ancrage ; et
la comparaison du nombre des pics correspondant à un ensemble présélectionné de pics ou à des ensembles de pics d'ancrage à partir des profils des pics des références.

6. Procédé selon la revendication 5, dans lequel les intensités des pics sont ajustées par :

l'ajustement des intensités des pics à un profil standard des différentes plages de masses ;
l'ajustement des régions des masses centrales du profil à une courbe gaussienne ; et
la révision des intensités pour tous les pics détectés avec l'ajustement.

7. Procédé selon la revendication 5, dans lequel les pics d'ancrage sont étalonnés par leur qualité de masse et de spectre.

8. Procédé selon la revendication 1, qui comprend l'identification de variations de séquence potentielles dans la séquence nucléotidique de l'un ou de plusieurs des profils de pics correspondants les mieux notés de l'ensemble des références et/ou de l'ensemble des échantillons.

9. Procédé selon la revendication 1, qui comprend l'attribution d'une valeur de confiance à la correspondance entre l'un ou plusieurs des profils de pics correspondants les mieux notés.

10. Procédé selon la revendication 1, dans lequel l'ensemble des signaux de masse des références est dérivé des produits de clivage résultant d'un acide nucléique de référence virtuellement mis en contact avec l'agent de clivage spécifique.

11. Procédé selon la revendication 10, dans lequel l'ensemble des signaux de masse des références est soumis à un regroupement.

12. Procédé selon la revendication 10, dans lequel chacun des ensembles des références est comparé à l'ensemble des échantillons.

13. Procédé selon la revendication 1, dans lequel la note de bitmap est calculée en comparant les intensités des profils des pics détectés et des références individuelles pondérées par une intensité de pic de référence.

14. Procédé selon la revendication 1, comprenant en outre une note d'identité de profil de pic provenant de la somme des intensités des pics correspondants, des intensités des pics manquants et supplémentaires, des intensités des pics manquants silencieux et des intensités des pics supplémentaires silencieux pour les profils des pics des références.

15. Procédé selon la revendication 1, comprenant en outre l'évaluation de chaque échantillon contre toutes les références pour une variation de pic ajustée qui est la somme de l'intensité des pics manquants et des pics supplémentaires dus aux qualités du spectre et ajustée par des pics inconnus et des pics de produits d'addition pour déterminer la variabilité de l'échantillon par rapport à la référence.

16. Procédé selon la revendication 15, comprenant en outre l'évaluation de la confiance du sous-ensemble des profils de pics correspondants par la détermination de la distribution de densité entre les notes et les variations de pic ajustées.

STEP 1: Data Import
One or multiple
reference sequences

simulated *in silico* peak patterns

STEP 3: MALDI-TOF MS

STEP 2: Sample Processing

FIG.1A

EP 2 145 180 B1

STEP 4: Data Analysis
Peak pattern comparison
and comparative sequence analysis
for sample identification and
sequence variation detection

Results

Details/Sequence Variation Detection

EP 2 145 180 B1

File Edit View Window Help

| Sample | 20070412 tbpb3.txt | | | |
|---|---|---|---|---|
| | Reference Sequence | Confidence | Sequence Variations | Sequence Variation Probability |
| 230_503 | NGtbpB3_2_4 | | | 0.012 |
| 231_504 | TbpB3_2_4 | | | 0.026 |
| 232_505 | 505_Tbpb3_3 | | | 0.007 |
| 233_506 | TBPB3_506 | | | 0.019 |
| 234_507 | TBPB3_506 | | | 0.004 |
| 235_508 | TBPB3_506 | | | 0.019 |
| 236_509 | 120_Tbpb3_ | | | 0.041 |
| 237_510 | 510_Tbpb3_ | | | 0.034 |
| 238_511 | 511_TBPB3_3 | | | 0.021 |
| 239_512 | TBPB3_506 | | | 0.005 |
| 240_513 | 513_TBPB3_3 | | | 0.019 |
| 241_514 | 058_TBPB3_3 | | | 0.005 |
| 242_515 | TBPB3_506 | | | 0.009 |
| 243_516 | TBPB3_506 | | | 0.003 |
| 244_517 | 059_TBPB3_3 | | t/C082[84/85]g/T0114 | 0.723 |
| 245_518 | TBPB3_506 | | | 0.005 |
| 246_519 | 016_Tbpb3_3 | | /R | 1.000 |
| 247_520 | TBPB3_506 | | | 0.010 |
| 248_521 | 115_Tbpb3_3 | | | 0.008 |
| 249_522 | 077_TBPB3_3 | | | 0.009 |
| 250_523 | 523_TBPB3_3 | | | 0.053 |
| 251_524 | 130_TBPB3_3 | | | 0.012 |
| 252_525 | 077_TBPB3_3 | | | 0.013 |
| 253_526 | 529_TBPB3_3 | | | 0.037 |
| 254_527 | TBPB3_506 | | | 0.006 |
| 255_528 | 077_TBPB3_3 | | | 0.012 |
| 256_529 | 077_TBPB3_3 | | | 0.017 |
| 257_530 | 077_TBPB3_3 | | | 0.020 |
| 258_531 | 513_TBPB3_3 | | | 0.176 |
| 259_532 | 077_TBPB3_3 | | | 0.014 |
| 260_533 | 077_TBPB3_3 | | | 0.014 |
| 261_534 | 115_Tbpb3_3 | | | 0.081 |
| 262_535 | TBPB3_506 | | | 0.008 |
| 263_536 | 213_TBPB3_3 | | c/T038a/GA118[] | 0.029 |
| 264_537 | TBPB3_506 | | | 0.017 |
| 265_538 | TBPB3_506 | | | 0.00 |

| Reference Sequence | Rank | Score |
|---|---|---|
| | *1 | 0.9998 |
| | 2 | 0.641 |
| | 3 | 0.634 |
| | 4 | 0.630 |
| | 5 | 0.616 |
| | 6 | 0.616 |
| | 7 | 0.605 |
| | 8 | 0.599 |
| | 9 | 0.595 |
| | 10 | 0.509 |

Top Matches | Sequence Variations

☐ T Forward | ☐ T Reverse | ☐ C Forward | ☐ C Re

| Spectra peak | SNP assignment |
|---|---|
| Missing peak 51 | t/C0487 |
| Additional peak 53 | t/C0487 |

STEP: 5 Statistics
Scoring
Sequence Variation Probability

FIG. 1B

Frequency

Score

EP 2 145 180 B1

File  Edit  View  Window  Help

Results

| Sample | 041018abcZcorr.txt | | | | 041018adkcorr.txt | | | |
|---|---|---|---|---|---|---|---|---|
| | Reference Sequence | | Confidence | SNPs | Sequence Variation Probability | Reference Sequence | Confidence | SNPs | Sequence Variation Probability |
| 018_01ng | abcZ13 | Details... | | | 0.004 | *adk_5 | | | 0.401 |
| 010_01ng | abcZ15 | Spectrum ▶ | | t/C@487 | 0.003 | adk_5 | | | 0.002 |
| 016_01ng | abcZ17 | Copy Selected | | | 0.006 | *adk_5 | | | 0.002 |
| 045_01ng | abcZ17 | Copy All | | | 0.006 | adk_5 | | | 0.003 |
| 084_01ng | abcZ17 | Sort ▶ | | | 0.022 | adk_5 | | | 0.006 |
| 092_01ng | abcZ20 | Find ▶ | | | 0.016 | adk_6 | | | 0.003 |
| 012_01ng | abcZ2 | Print... | | | | | | | |
| 025_01ng | abcZ2 | Print Preview | | | | | | | |
| 034_01ng | abcZ2 | | | | | | | | |
| 031_01ng | abcZ2 | | | | | | | | |
| 037_01ng | abcZ2 | | | | | | | | |
| 039_01ng | abcZ2 | | | | | | | | |
| 046_01ng | abcZ2 | | | | | | | | |
| 041_01ng | abcZ2 | | | | | | | | |
| 042_01ng | abcZ2 | | | | | | | | |
| 048_01ng | abcZ2 | | | | | | | | |
| 051_01ng | abcZ2 | | | | | | | | |
| 058_01ng | abcZ2 | | | | | | | | |
| 059_01ng | abcZ2 | | | | | | | | |
| 060_01ng | abcZ2 | | | | | | | | |

File  Edit  View  Window  Help

Details

Top Matches | Sequence Variations

| Reference Sequence | Rank | Score | ☐ T Forward  ☐ T Reverse  ☐ C Forward  ☐ C Reverse |
|---|---|---|---|
| abcZ15 | *1 | 0.978 | |
| abcZ20 | 2 | 0.743 | |
| abcZ1 | 3 | 0.735 | |
| abcZ17 | 4 | 0.732 | |
| abcZ133 | 5 | 0.731 | |
| abcZ112 | 6 | 0.724 | |
| abcZ259 | 7 | 0.719 | |
| abcZ199 | 8 | 0.717 | |
| abcZ190 | 9 | 0.709 | |
| abcZ181 | 10 | 0.705 | |

| Spectra peak | SNP assignment | Sample mass | Reference mass | Ref type | Description |
|---|---|---|---|---|---|
| Missing peak 17 | t/C@487 | | 1,939.19 | MAIN | CGCCCT @482; |
| Additional peak 35 | t/C@487 | 2,557.58 | | NEW | A1C363T1 (0.0052) |

FIG.2A

FIG.2B

Sequence Variation Probability

Best matching reference sequence

...GGAGGGCCACCGAATACCTGAT...

MassARRAY
database

Experimental spectrum

Pattern
comparison

Simulated pattern

012 (TF) (Reference 14)

Ranked list of
reference sequence
Matches in the
Details Window

| Details | | |
|---|---|---|
| Top Matches | Sequence Variations | |
| Reference Sequence | Rank | Score |
| Reference 25 | *1 | 0.983 |

Best
match

Score

Frequency

Results Window

| Results | | | | |
|---|---|---|---|---|
| Sample | Reference_example_2 | | | |
| | Reference Sequence | Confidence | Sequence Variations | Sequence Variat Probability |
| 001 | Reference 4 | | | 0.006 |
| 002 | Reference 1 | | | 0.003 |
| 003 | Reference 4 | | | 0.003 |
| 004 | Reference 25 | | a/T0124 | 0.003 |
| 005 | Reference 4 | | | 0.002 |
| 006 | Reference 15 | | | 0.022 |

Compute
Sequence Variation
Probability Value

FIG.3

Experiment: Reference sequence sets + unknown samples

iSEQ software analysis:
• Evaluate spectra quality ⟶
• Determine best matching reference sequence

Repeat failed reactions o

Identification

Identification

Screening

No sequence variation

Single base pair sequence variation(s) identified

Few or no unique matches found, sequence variations or multiple matches occur

Samples identified

**A.**

SNP discovery

Samples identified & new reference sequences generated

**B.**

Reanalyze using relaxed parameters & generate a Distance Matrix report

Cluster samples using third party software & distance matrix input

Dideoxy sequence a sample from each cluster ⟶ new reference sequences

Reanalyze acquired data using new reference sequences (analyze a *derived* experiment, a copy of the original experiment that includes data)

# FIG.4

**C.**

FIG.5

FIG.6A

FIG.6B

FIG.6C

FIG.7A-1

ED 2.8

FIG.7A-2

ED 2.8

FIG.7B-1

FIG.7B-2

Re-sequencing by MALDI-TOF MS

PCR

Post–PCR

*Microbial DNA*

$5'-$ ═══════ $-3'$
*Amplified target*

───────────
*Transcribed RNA*

$\underline{\quad}C \underline{\quad}^C \underline{\quad}C$

*Mixture of base-specific
cleavage products*

Database

Biochemistry

*Peak pattern matching
Mutation detection
Sample identification*

Analysis

Mass spectrometry

MALDI-TOF MS

*Cleavage product separation*

FIG.8

Simulation— *in silico* peak pattern

DNA reference
sequence

AACACGTGGGTGATCTGCCCTGCACTGGGAT

$\downarrow$  T–specific cleavage

RNA *in silico* cleavage

AACACGU  GAU  GCCCU  GGGAU
GGGU  CU  GCACU

*in silico* pattern

$C_1U_1$ = 614.4 Da

$G_1A_1U_1$ = 999.6 Da

1 $G_3U_1$ = 1390.8 Da

2 $G_1C_3U_1$ = 1537.9 Da

3 $G_1C_2A_1U_1$ = 1577.9 Da

4 $G_3A_1U_1$ = 1690.0 Da

5 $G_1C_2A_3U_1$ = 2236.4 Da

4 cleavage reactions per sample.

FIG.9

EP 2 145 180 B1

Biochemistry

T7 primer

genomic DNA

5'— ═══════════════════ —3'   DNA

SP6 primer

5'— ═══════════════════ —3'   PCR product

5'— — — — — — — → —3'   3'— ← — — — — — — —5'   RNA

U   C   C   U

base-specific compomers

T   C   "G"   "A"

# FIG.10

Peak processing

```
┌──────────────────────────────────────────────────────────┐
│          Raw peak list (~300-400 peaks)                    │
└──────────────────────────────────────────────────────────┘
                    │
                    │              ╱‾‾‾‾‾‾‾‾‾‾‾‾‾‾╲
                    │             │  Peak intensity │
                    ↓             │  normalization  │
                                   ╲_____╱
┌──────────────────────────────────────────────────────────┐
│          Final peak list (~100 peaks)                      │
└──────────────────────────────────────────────────────────┘
                    │
                    │                ╱‾‾‾‾‾‾‾‾‾‾‾╲
                    │               │  Calibration │
                    │                ╲_____╱
                    ↓              ╱‾‾‾‾‾‾‾‾‾‾‾‾‾‾╲
                                  │ Removal of chemical│
                                  │      noise         │
                                   ╲_____╱
┌──────────────────────────────────────────────────────────┐
│          Normalized peak list                              │
└──────────────────────────────────────────────────────────┘
```

Variation of Peak Intensity versus Mass for T Cleavage Reaction

- T Cleavage, abcZ-0dk
- T Cleavage, avoE-0gm

Peak Intensity (Normalized by Ref Intens)

Mass (dakons)

## FIG.11

Peak pattern matching

| RefMass | abcZ1 | abcZ2 | abcZ3 | abcZ4 | abcZ5 | abcZ6 | abcZ7 | abcZ8 | abcZ9 | abcZ10 |
|---|---|---|---|---|---|---|---|---|---|---|
| 2019.2 | 1.0 | | | | | | | | | |
| 2172.4 | 1.0 | 1.0 | 1.0 | 1.0 | 1.0 | 1.0 | 1.0 | 1.0 | 1.0 | 1.0 |
| 2196.4 | 1.0 | | | 1.0 | 1.0 | | 1.0 | 1.0 | 1.0 | |
| 2212.4 | | 1.0 | | | | | | | | |
| 2228.4 | 1.0 | 1.0 | 1.0 | 1.0 | 1.0 | 1.0 | 1.0 | 1.0 | 1.0 | 1.0 |
| 2268.4 | 1.0 | | | | | | | | | |
| 2276.4 | | | | 1.0 | | | | | | |
| 2292.4 | 1.0 | 1.0 | | 2.0 | 2.0 | 2.0 | 2.0 | | 1.0 | |
| 2308.4 | | 1.0 | 1.0 | | 1.0 | | | 1.0 | 1.0 | 1.0 |
| 2332.5 | 1.0 | 1.0 | | 2.0 | 2.0 | 2.0 | 2.0 | | | |

Reference Intensity

| Detected | Intens | Detected |
|---|---|---|
| missing | | 2019.2 |
| 2172.32 | 0.71 | 2172.4 |
| 2196.4 | 1.25 | 2196.4 |
| missing | | 2212.4 |
| 2218.45 | 0.13 | NAAD (2196.4) |
| 2228.43 | 0.85 | 2228.4 |
| 2234.08 | 0.16 | K-AD (2196.4) |
| 2250.64 | 0.13 | NAAD (2228.4) |
| missing | | 2268.4 |
| missing | | 2276.4 |
| 2292.3 | 1.07 | 2292.4 |
| 2308.63 | 0.84 | 2308.4 |

Both calibrated **peak mass** and normalized **peak intensity** are used for pattern matching.

# FIG.12

EP 2 145 180 B1

Iterative pattern matching

FIG.13

EP 2 145 180 B1

```
                          ( Start )
            ┌─────────────────┴─────────────────┐
            ▼                                    ▼
┌──────────────────────────┐      ┌──────────────────────────────┐
│ (1) Reference data       │      │ (2) Data acquisition         │
│ b.) by cleavage of a set │      │ cleavage of the target       │
│     of references or     │      │ molecule                     │
│ c.) by simulation of a   │      │ of a sample and detection of │
│     set of sequences     │      │ fragment data                │
└──────────────────────────┘      └──────────────────────────────┘
            │                                    ▼
            │                     ┌──────────────────────────────┐
            │                     │ (3) Peak Detection           │
            │                     └──────────────────────────────┘
            │                                    ▼
            │                     ┌──────────────────────────────┐
            │                     │ (4) Calibration              │
            │                     └──────────────────────────────┘
            │                                    ▼
            │                     ┌──────────────────────────────┐
            │                     │ (5) Normalization            │
            │                     └──────────────────────────────┘
            │                                    ▼
            │                     ┌──────────────────────────────┐
            │                     │ (6) Spectra Quality          │
            │                     └──────────────────────────────┘
            │                                    ▼
            │                     ┌──────────────────────────────┐
            │                     │ (7) Intensity Scaling        │
            │                     └──────────────────────────────┘
            │                                    ▼
            │                     ┌──────────────────────────────┐
            │                     │ (8) Compomer Adjustment      │
            │                     └──────────────────────────────┘
            └─────────────────┬───────────────────┘
                              ▼
            ┌──────────────────────────────────┐
            │ (9) Iterative Process            │
            │ identification of the best       │
            │ matching reference among         │
            │ the reference set                │
            └──────────────────────────────────┘
                              ▼
            ┌──────────────────────────────────┐
            │ (10) Scoring                     │
            │ of the best matching             │
            │ reference                        │
            └──────────────────────────────────┘
                              ▼
            ┌──────────────────────────────────┐
            │ (11) SNP Discovery               │
            │ (US 2005/0112590)                │
            └──────────────────────────────────┘
                              ▼
            ┌──────────────────────────────────┐
            │ (12) Confidence                  │
            └──────────────────────────────────┘
                              ▼
            ┌──────────────────────────────────┐
            │ (13) Probability Calculation     │
            └──────────────────────────────────┘
                              ▼
                          ( End )
```

FIG.14

Start

(13) Reference data
by cleavage of at least one
reference

(2) Data acquisition
cleavage of the target molecule
of a sample and detection of
fragment data

(3) Peak Detection

(4) Calibration

(5) Normalization

(6) Spectra Quality

(7) Intensity Scaling

(20) Distance Matrix
for
Clustering

FIG.15

FIG.16

300

SAMPLE
305

PROCESSING
STATION
306

ROBOTIC
DEVICE
310

MASS MEASURING
STATION
308

BIOMOLECULE
WORKSTATION
302

312

ANALYSIS COMPUTER
304

FIG.17

400

| CPU | | Display | |
|---|---|---|---|
| | 402 | | 406 |

| DASD | | Keyboard  Mouse | |
|---|---|---|---|
| | 408 | | 404 |

| Memory | | Program  Product | |
|---|---|---|---|
| | 410 | Reader | |

| Network  Interface | | | |
|---|---|---|---|
| | 418 | | 412 |

420

422

414

NETWORK

# FIG.18

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 9957318 W
- US 5118937 A
- WO 2004050839 A, D. van den Boom
- US 20050112590 A, Boom
- US 5173418 A
- US 5536649 A
- US 5888795 A
- US 5952176 A
- US 6099553 A
- US 6190865 B1
- WO 9703210 A
- WO 9954501 A
- WO 9421663 A
- US 6326174 B
- US 6194180 B
- US 6265167 B
- US 6096715 B
- US 5646020 B
- US 5843669 A
- US 5874283 A
- US 6090606 A
- US 5387518 A
- US 5391490 A
- US 5427927 A
- US 5252478 A
- US 6331427 B1
- US 6383775 B
- WO 9636732 A
- US 5792664 A
- WO 9957318 W
- US 5547835 A
- US 6194144 A
- US 6225450 A
- US 5691141 A
- US 6238871 A
- WO 9629431 A
- WO 9820166 A
- WO 9812355 A
- US 5869242 A
- WO 9733000 A
- WO 9854571 A
- US 5622824 A
- US 5605798 A
- US 6024925 A
- US 786988 A
- US 09364774 B
- US 09371150 B
- US 09297575 B
- US 9720195 W
- WO 9820020 A
- EP 0269520 A
- EP 0655501 A
- WO 9400562 A
- WO 9213969 A1
- US 6043031 A
- WO 9737041 A
- US 7255992 B
- US 7217510 B
- US 7226739 B
- US 7108974 B
- US 285481 A
- US 09663968 A
- US 09836629 A
- WO 0060361 A

### Non-patent literature cited in the description

- **T. STRACHAN.** The Human Genome. BIOS Scientific Publishers, 1992
- **HONISCH et al.** Improving the odds in comparative sequence analysis Mass spectrometry for sensitive detection of sequence variations. *program Nr: 1590 from 2003 ASHG Annual Meeting, www.ashg.org/genetics/abstracts/abs03/f1590.htm*
- **WOESE.** *Nucleic Acid Research,* 1997, vol. 25 (1), 109-11
- **SEELA, F. ; A. KEHNE.** *Biochemistry,* 1987, vol. 26, 2232-2238
- **J. SAMUELSSON.** Modular, scriptable and automated analysis tool for high-throughput peptide mass fingerprinting. *Bioinformatics,* 2004, vol. 20 (18
- **NORDHOFF et al.** Ion stability of nucleic acids in infrared matrix-assisted laser desorption/ionization mass spectrometry. *Nucl. Acids Res.,* 1993, vol. 21 (15), 3347-57
- **SARGENT et al.** *Methods Enzymol.,* 1988, vol. 152, 432
- **A. M. MAXAM ; W. GILBERT.** *Proc. Natl. Acad. Sci. USA,* 1977, vol. 74, 560-64

- **K. A. BROWNE.** Metal ion-catalyzed nucleic Acid alkylation and fragmentation. *J. Am. Chem. Soc.,* 2002, vol. 124 (27), 7950-62
- **I. G. GUT ; S. BECK.** A procedure for selective DNA alkylation and detection by mass spectrometry. *Nucleic Acids Res.,* 1995, vol. 23 (8), 1367-73
- **SHCHEPINOV et al.** Matrix-induced fragmentation of P3'-N5'-phosphoroamidate-containing DNA: high-throughput MALDI-TOF analysis of genomic sequence polymorphisms. *Nucleic Acids Res.,* 2001, vol. 25, 3864-3872
- **GOGOS et al.** *Nucl. Acids Res.,* 1990, vol. 18, 6807-6817
- **SAMBROOK, J. ; RUSSELL, D. W.** Molecular Cloning: A Laboratory Manual. Cold Spring Harbor Laboratory Press, 2001
- **HARRINGTON ; LIENER.** *Genes and Develop.,* 1994, vol. 8, 1344
- **MURANTE, R. S. et al.** *J. Biol. Chem.,* 1994, vol. 269, 1191
- Nucleic Acids and Molecular Biology. Springer-Verlag, 1988, vol. 2
- **BARLOW ; LEHRACH.** *Trends Genet.,* 1987, vol. 3, 167
- **PERLMAN ; BUTOW.** *Science,* 1989, vol. 246, 1106
- **HANNISH, J. ; M. MCCLELLAND.** Activity of DNA modification and restriction enzymes in KGB, a potassium glutamate buffer. *Gene Anal. Tech.,* 1988, vol. 5, 105
- **MCCLELLAND, M. et al.** A single buffer for all restriction endonucleases. *Nucleic Acid Res.,* 1988, vol. 16, 364
- **DONIS-KELLER et al.** *Nucleic Acids Res.,* 1977, vol. 4, 2527-2537
- **GUPTA ; RANDERATH.** *Nucleic Acids Res.,* 1977, vol. 4, 1957-1978
- **KUCHINO ; NISHIMURA.** *Methods Enzymol.,* 1989, vol. 180, 154-163
- **HAHNER et al.** *Nucl. Acids Res.,* 1997, vol. 25 (10), 1957-1964
- **BOGUSKI et al.** *J. Biol. Chem.,* 1980, vol. 255, 2160-2163
- **ROJO et al.** *Planta,* 1994, vol. 194, 328-338
- **DONIS-KELLER, H.** *Nucleic Acids Res.,* 1980, vol. 8, 3133-3142
- **SIMONCSITS et al.** *Nature,* 1977, vol. 269, 833-836
- **EFTEDAL et al.** *Nucleic Acids Res,* 1993, vol. 21, 2095-2101
- **BJELLAND ; SEEBERG.** *Nucleic Acids Res.,* 1987, vol. 15, 2787-2801
- **SAPARBAEV et al.** *Nucleic Acids Res.,* 1995, vol. 23, 3750-3755
- **BESSHO.** *Nucleic Acids Res.,* 1999, vol. 27, 979-983
- **CHUNG et al.** An endonuclease activity of Escherichia coli that specifically removes 8-hydroxyguanine residues from DNA. *Mutation Research,* 1991, vol. 254, 1-12
- **LU, A-L ; HSU, I-C.** *Genomics,* 1992, vol. 14, 249-255
- **HSU, I-C. et al.** *Carcinogenesis,* 1994, vol. 14, 1657-1662
- **CANNISTRARO et al.** *Eur. J. Biochem.,* 1989, vol. 181, 363-370
- **STEVENS et al.** *J. Bacteriol.,* 1985, vol. 164, 57-62
- **MAROTTA et al.** *Biochemistry,* 1973, vol. 12, 2901-2904
- **ANDERSON, S.** Shotgun DNA sequencing using cloned DNase I-generated fragments. *Nucleic Acids Res.,* 1981, vol. 9, 3015-3027
- **EHRLICH, S. D. et al.** Studies on acid deoxyribonuclease. IX. 5'-Hydroxy-terminal and penultimate nucleotides of oligonucleotides obtained from calf thymus deoxyribonucleic acid. *Biochemistry,* 1971, vol. 10 (11), 2000-9
- **SANTORO, S. W. ; JOYCE, G. F.** A general purpose RNA-cleaving DNA enzyme. *Proc. Natl. Acad. Sci. USA,* 1997, vol. 94, 4262-4266
- **YULE.** *Bio/Technology,* 1994, vol. 12, 1335
- **NIKODEM ; FRESCO.** *Anal. Biochem.,* 1979, vol. 97, 382-386
- **JAHNEN et al.** *Biochem. Biophys. Res. Commun.,* 1990, vol. 166, 139-145
- **VANFLETEREN et al.** *BioTechniques,* 1992, vol. 12, 550-557
- **SARIS et al.** *Anal. Biochem.,* 1983, vol. 132, 54-67
- **CLEVELAND et al.** *J. Biol. Chem.,* 1977, vol. 252, 1102-1106
- **HAFFEY, M. L. et al.** *DNA,* 1987, vol. 6, 565
- **NAGAI, K. ; THOGERSEN, H. C.** *Nature,* 1984, vol. 309, 810
- **SMITH, D. B. ; JOHNSON, K. S.** *Gene,* 1988, vol. 67, 31
- **GERMINO J. ; BASTIS, D.** *Proc. Natl. Acad. Sci. USA,* 1984, vol. 81, 4692
- **BIEMAN.** *Methods in Enzymology,* 1990, vol. 193, 455-479
- **ZAIA, J.** Protein and Peptide Analysis by Mass Spectrometry. Humana Press, 1996, 29-41
- **JOHNSON, R. S. et al.** *Mass Spectrom. Ion Processes,* 1988, vol. 86, 137-154
- **VATH, J. E. et al.** *Fresnius Z Anal. Chem.,* 1988, vol. 331, 248-252
- **STULTS, J. T et al.** *Anal. Chem.,* 1993, vol. 65, 1703-1708
- **ZAIA, J. et al.** *J Am. Soc. Mass Spectrom.,* 1995, vol. 6, 423-436
- **WAGNER, D. S. et al.** *Biol. Mass Spectrom.,* 1991, vol. 20, 419-425

- **HUANG, Z.-H. et al.** *Anal. Biochem.,* 1999, vol. 268, 305-317
- **MEANS et al.** *Chemical Modification of Proteins, Holden-Day, San Francisco,* 1971
- **FEENEY et al.** Modification of Proteins: Food, Nutritional and Pharmacological Aspects, Advances in Chemistry Series. American Chemical Society, 1982, vol. 198
- Genetics in Medicine. W.B. Saunders Co, 1991
- Meth. Enzymol., Vol. 193, Mass Spectrometry. Academic Press, 1990, vol. 193
- **MCLAFFERY et al.** *Acc. Chem. Res.,* 1994, vol. 27, 297-386
- **CHAIT ; KENT.** *Science,* 1992, vol. 257, 1885-1894
- **SIUZDAK.** *Proc. Natl. Acad. Sci., USA,* 1994, vol. 91, 11290-11297
- **HILLENKAMP et al.** *Anal. Chem.,* 1991, vol. 63, 1193A-1202A
- **BERKENKAMP et al.** *Proc. Natl. Acad. Sci. USA,* 1996, vol. 93, 7003-7007
- **H. KOSTER et al.** *Nature BiotechnoL,* 1996, vol. 14, 1123-1128
- **A. BRAUN et al.** *Genomics,* 1997, vol. 46, 18
- **D. P. LITTLE et al.** *Nat. Med.,* 1997, vol. 3, 1413
- **L. HAFF et al.** *Genome Res.,* 1997, vol. 7, 378
- **P. ROSS et al.** *Nat. Biotechnol.,* 1998, vol. 16, 1347
- **K. TANG et al.** *Proc. Natl. Acad. Sci. USA,* 1999, vol. 96, 10016
- **FELSENSTEIN, J.** Phylogenies from restriction sites, a maximum likelihood approach. *Evolution,* 1992, vol. 46, 159-173
- **WANG et al.** *Science,* 1998, vol. 280, 1077-1082
- **RATNER et al.** *Nature,* 1985, vol. 313, 227-284
- **WAIN HOBSON et al.** *Cell,* 1985, vol. 40, 9-17
- **GUYADER et al.** *Nature,* 1987, vol. 328, 662-669
- **CHAKRABARTI et al.** *Nature,* 1987, vol. 328, 543-547
- **GUST et al.** *Intervirology,* 1983, vol. 20, 1-7
- **HEYM et al.** *Lancet,* 1994, vol. 344, 293
- **MORRIS et al.** *J. Infect. Dis.,* 1995, vol. 171, 954
- **BANERJEE et al.** *Science,* 1994, vol. 263, 227
- **J. L. WEBER ; P. E. CAN.** *Am. J. Hum. Genet.,* 1989, vol. 44, 388
- **J. WEISSENBACH et al.** *Nature,* 1992, vol. 359, 794
- **C. T. CASKEY et al.** *Science,* 1992, vol. 256, 784
- **P. J. MCKINNEN.** *Hum. Genet. 1,* 1987, vol. 75, 197
- **J. GERMAN et al.** *Clin. Genet.,* 1989, vol. 35, 57
- **EDWARDS et al.** *Nucl. Acids Res.,* 1991, vol. 19, 4791
- **BECKMANN et al.** *Genomics,* 1992, vol. 12, 627-631
- **NAKAMURA et al.** *Science,* 1987, vol. 235, 1616-1622
- **JEFFREYS et al.** *Nature,* 1985, vol. 314, 67-73
- **LUTY et al.** *Nucleic Acids Res.,* 1991, vol. 19, 4308
- **LITT et al.** *Nucleic Acids Res.,* 1990, vol. 18, 4301
- **LITT et al.** *Nucleic Acids Res.,* 1990, vol. 18, 5921
- **LUTY et al.** *Am. J. Hum. Genet.,* 1990, vol. 46, 776-783
- **TAUTZ.** *Nucl. Acids Res.,* 1989, vol. 17, 6463-6471
- **WEBER et al.** *Am. J. Hum. Genet.,* 1989, vol. 44, 388-396
- **POLYMEROPOULOS et al.** *Nucl. Acids Res.,* 1991, vol. 19, 195
- **POLYMEROPOULOS et al.** *Nucl. Acids Res.,* 1991, vol. 19, 4306
- **NISHIMURA et al.** *Nucl. Acids Res.,* 1992, vol. 20, 1167
- **POLYMEROPOULOS et al.** *Nucl. Acids Res.,* 1991, vol. 19, 4018
- **ZULIANI et al.** *Nucl. Acids Res.,* 1990, vol. 18, 4958
- **POLYMEROPOULOS et al.** *Nucl. Acids Res.,* 1990, vol. 18, 7468
- **PLOOS et al.** *Nucl. Acids Res.,* 1990, vol. 18, 4957
- **ANKER et al.** *Hum. Mol. Genet.,* 1992, vol. 1, 137
- **KWOK et al.** *Nucl. Acids Res.,* 1990, vol. 18, 999
- **SCHCHTER et al.** *Nature Genetics,* 1994, vol. 6, 29-32
- **REYMER et al.** *Nature Genetics,* 1995, vol. 10, 28-34
- **BIRD, A.** *Genes Dev.,* 2002, vol. 16, 6-21
- **VAN ERT, M. N. ; HOFSTADLER, S.A. ; JIANG Y. ; BUSCH, J. D. ; WAGNER, D. M. ; DRADER J. J. ; ECKER, D. J. ; HANNIS, J. C. ; HUYNH, L. Y. ; SCHUPP, J. M. et al.** *Biotechniques,* 2004, vol. 37, 642-644
- **SAMPATH, R. ; HOFSTADLER, S.A. ; BLYN, L. B. ; ESHOO, M. W. ; HALL, T. A. ; MASSIRE, C. ; LEVENE, H. M. ; HANNIS, J. C. ; HARRELL, P. M. ; NEUMAN, B. et al.** *Emerg Infect Dis,* 2005, vol. 11, 373-379

- **ECKER, J. A. ; MASSIRE, C. ; HALL, T. A. ; RANKEN, R. ; PENNELLA, T.T. ; AGASINO IVY, C. ; BLYN, L.B. ; HOFSTADLER, S. A. ; ENDY, T. P. ; SCOTT, P. T. et al.** *J Clin Microbiol,* 2006, vol. 44, 2921-2932
- **BOCKER, S.** SNP and mutation discovery using base-specific cleavage and MALDI-TOF mass spectrometry. *Bioinformatics,* 2003, vol. 19 (1), i44-53
- **CLARKE, S.C.** Nucleotide sequence-based typing of bacteria and the impact of automation. *Bioessays,* 2002, vol. 24, 858-862
- **DING, C. ; C.R. CANTOR.** Direct molecular haplotyping of long-range genomic DNA with M1-PCR. *Proc Natl Acad Sci U S A,* 2003, vol. 100, 7449-7453
- **ENRIGHT, M.C. ; B.G. SPRATT.** Multilocus sequence typing. *Trends Microbiol,* 1999, vol. 7, 482-487
- **FEAVERS, I.M. ; S.J. GRAY ; R. URWIN ; J.E. RUSSELL ; J.A. BYGRAVES ; E.B. KACZMARSKI ; M.C. MAIDEN.** Multilocus sequence typing and antigen gene sequencing in the investigation of a meningococcal disease outbreak. *J Clin Microbiol,* 1999, vol. 37, 3883-3887
- **GARAIZAR, J. ; A. REMENTERIA ; S. PORWOLLIK.** DNA microarray technology: a new tool for the epidemiological typing of bacterial pathogens?. *FEMS Immunol Med Microbiol,* 2006, vol. 47, 178-189
- **JOLLEY, K.A. ; J. KALMUSOVA ; E.J. FEIL ; S. GUPTA ; M. MUSILEK ; P. KRIZ ; M.C. MAIDEN.** Carried meningococci in the Czech Republic: a diverse recombining population. *J Clin Microbiol,* 2000, vol. 38, 4492-4498
- **KLING, J.** Ultrafast DNA sequencing. *Nat Biotechnol,* 2003, vol. 21, 1425-1427
- **LEFMANN, M. ; C. HONISCH ; S. BOCKER ; N. STORM ; F. VON WINTZINGERODE ; C. SCHLOTELBURG ; A. MOTER ; D. VAN DEN BOOM ; U.B. GOBEL.** Novel mass spectrometry-based tool for genotypic identification of mycobacteria. *J Clin Microbiol,* 2004, vol. 42, 339-346
- **MAIDEN, M.C.** Multilocus Sequence Typing of Bacteria. *Annu Rev Microbiol,* 2006
- **MAIDEN, M.C. ; J.A. BYGRAVES ; E. FEIL ; G. MORELLI ; J.E. RUSSELL ; R. URWIN ; Q. ZHANG ; J. ZHOU ; K. ZURTH ; D.A. CAUGANT.** Multilocus sequence typing: a portable approach to the identification of clones within populations of pathogenic microorganisms. *Proc Natl Acad Sci U S A,* vol. 95, 3140-3145
- **MURPHY, K.M. ; K.A. O'DONNELL ; A.B. HIGGINS ; C. O'NEILL ; M.T. CAFFERKEY.** Irish strains of Neisseria meningitidis: characterisation using multilocus sequence typing. *Br J Biomed Sci,* 2003, vol. 60, 204-209
- **OLIVE, D.M. ; P. BEAN.** Principles and applications of methods for DNA-based typing of microbial organisms. *J Clin Microbiol,* 1999, vol. 37, 1661-1669
- **PFALLER, M.A.** Molecular epidemiology in the care of patients. *Arch Pathol Lab Med,* 1999, vol. 123, 1007-1010
- **SPRATT, B.G.** Multilocus sequence typing: molecular typing of bacterial pathogens in an era of rapid DNA sequencing and the internet. *Curr Opin Microbiol,* 1999, vol. 2, 312-316
- **STANSSENS, P. ; M. ZABEAU ; G. MEERSSEMAN ; G. REMES ; Y. GANSEMANS ; N. STORM ; R. HARTMER ; C. HONISCH ; C.P. RODI ; S. BOCKER.** High-throughput MALDI-TOF discovery of genomic sequence polymorphisms. *Genome Res,* 2004, vol. 14, 126-133
- **SULLIVAN, C.B. ; M.A. DIGGLE ; S.C. CLARKE.** Multilocus sequence typing: Data analysis in clinical microbiology and public health. *Mol Biotechnol,* 2005, vol. 29, 245-254
- **URWIN, R. ; M.C. MAIDEN.** Multi-locus sequence typing: a tool for global epidemiology. *Trends Microbiol,* 2003, vol. 11, 479-487
- **VAN BELKUM, A.** High-throughput epidemiologic typing in clinical microbiology. *Clin Microbiol Infect,* 2003, vol. 9, 86-100
- **VON WINTZINGERODE, F. ; S. BOCKER ; C. SCHLOTELBURG ; N.H. CHIU ; N. STORM ; C. JURINKE ; C.R. CANTOR ; U.B. GOBEL ; D. VAN DEN BOOM.** Base-specific fragmentation of amplified 16S rRNA genes analyzed by mass spectrometry: a tool for rapid bacterial identification. *Proc Natl Acad Sci U S A,* 2002, vol. 99, 7039-7044
- **YAZDANKHAH, S.P. ; P. KRIZ ; G. TZANAKAKI ; J. KREMASTINOU ; J. KALMUSOVA ; M. MUSILEK ; T. ALVESTAD ; K.A. JOLLEY ; D.J. WILSON ; N.D. MCCARTHY.** Distribution of serogroups and genotypes among disease-associated and carried isolates of Neisseria meningitidis from the Czech Republic, Greece, and Norway. *J Clin Microbiol,* 2004, vol. 42, 5146-5153